(19) Europäisches Patentamt
European Patent Office
Office européen des brevets

(11) Publication number: 0 286 746 B1

(12) # EUROPEAN PATENT SPECIFICATION

(45) Date of publication of patent specification: 27.02.91

(51) Int. Cl.5: C07C 243/38, C07D 333/38, C07C 255/56, C07F 7/10, A01N 33/26, A01N 37/44, A01N 55/00

(21) Application number: 87303296.5

(22) Date of filing: 15.04.87

(54) **Insecticidal N-(optionally substituted) - N'-substituted-N,N'-disubstituted- hydrazines.**

(43) Date of publication of application:
19.10.88 Bulletin 88/42

(45) Publication of the grant of the patent:
27.02.91 Bulletin 91/09

(84) Designated Contracting States:
AT BE CH DE ES FR GB GR IT LI LU NL SE

(56) References cited:
DD-A- 212 889

CHEMICAL ABSTRACTS, vol. 76, no. 21, 22nd May 1972, page 417, abstract no. 125990y, Columbus, Ohio, US; Q.N. PORTER et al.: "Mass spectrometric studies. XI. Skeletal rearrangements in acrylhydrazines".

(73) Proprietor: ROHM AND HAAS COMPANY
Independence Mall West
Philadelphia Pennsylvania 19105(US)

(72) Inventor: Hsu, Adam Chi-Tung
1686 Heebner Way
Lansdale Pennsylvania 19446(US)
Inventor: Hamp, Donald Wesley
17 Blacksmith Circle
Horsham Pennsylvania 19044(US)

(74) Representative: Angell, David Whilton et al
ROHM AND HAAS (UK) LTD. European Operations Patent Department Lennig House 2 Mason's Avenue
Croydon CR9 3NB(GB)

EP 0 286 746 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with novel N-(optionally substituted)-N'-substituted-N,N'-disubstituted-hydrazines, which are useful as insecticides, compositions containing those compounds and methods of their use. The disclosed hydrazines are new compounds.

The search for compounds which have a combination of excellent insecticidal activity and low undesirable toxicity is a continuing one because of factors such as the desire for compounds exhibiting greater activity, better selectivity, low undesirable environmental impact, low production cost and effectiveness against insects resistant to many known insecticides.

Compounds of the present invention are particularly suitable for controlling plant-destructive insects in crops of cultivated plants, ornamentals and forestry.

Certain hydrazine derivatives have been disclosed in the literature.

In 25 Aust. J. Chem. , 523-529 (1972), several N,N'-dibenzoylhydrazine derivatives are disclosed including N'-i -propyl-; N'-n -propyl-; N'-(2-methylpropyl)-; N'-(3-methylbutyl)-; N'-benzyl- and N'-phenyl-N,N'-dibenzoylhydrazine in which one or both nitrogen atoms are alkylated or phenylated.

In 61 Helv. Chim. Acta , 1477-1510 (1978), several N,N'-dibenzoylhydrazine and hydrazide derivatives including N'-t -butyl-N-benzoyl-N'-(4-nitrobenzoyl)-hydrazine are disclosed.

In 44 J.A.C.S. , 2556-2567 (1922), isopropylhydrazine $(CH_3)_2CH\text{-}NH\text{-}NH_2$, symmetrical diisopropyl-hydrazine, dibenzoylisopropylhydrazine and certain derivatives are disclosed.

In 44 J.A.C.S. , 1557-1564 (1972), isopropyl, menthyl and bornyl semicarbazides are disclosed.

In 48 J.A.C.S. , 1030-1035 (1926), symmetrical di-methylphenylmethylhydrazine and certain related compounds including 1,2-bis-methylphenylmethyl-4-phenyl-semicarbazide are disclosed.

In 27 Bull. Chem. Soc. Japan , 624-627 (1954), certain hydrazine derivatives including alpha-beta-dibenzoylphenylhydrazine are disclosed.

In J. Chem. Soc. (C) , 1531-1536 (1966), N,N'-dibenzoylphenylhydrazine and N-acetyl-N'-benzoyl-p-nitrophenylhydrazine are disclosed.

In 56B Chem. Berichte , 954-962 (1923), symmetrical di-isopropylhydrazines, symmetrical diisobutyl-and certain derivatives including N,N'-diisobutyldibenzoylhydrazine are disclosed.

In 590 Annalen der Chemie , 1-36 (1954), certain N,N'-dibenzoylhydrazine derivatives are disclosed including N'-methyl- and N'-(2-phenyl)-isopropyl-N,N'-dibenzoylhydrazine.

In J. Chem. Soc. , 4191-4198 (1952), N,N'-di-n -propylhydrazine, N,N'-dibenzoylhydrazine and bis-3,5-dinitrobenzoyl are disclosed.

In 32 Zhur. Obs. Khim. , 2806-2809 (1962), N'-2,4-methyl-2,4-pentadiene-N,N'-dibenzoylhydrazine is disclosed.

In 17 Acta. Chim. Scand. , 95-102 (1963), 2-benzoyl-thiobenzhydrazide ($C_6H_5\text{-}CS\text{-}NHNH\text{-}CO\text{-}C_6H_5$) and certain hydrazone and hydrazine derivatives are disclosed including 1,2-dibenzoyl-benzylhydrazine.

In 25 Zhur. Obs. Khim , 1719-1723 (1955), N,N'-bis-cyclohexylhydrazine and N,N'-dibenzoylcyclohexyl-hydrazine are disclosed.

In J. Chem. Soc. , 4793-4800 (1964), certain dibenzoylhydrazine derivatives are disclosed including tribenzoylhydrazine and N,N'-dibenzoylcyclohexylhydrazine.

In 36 J. Prakt. Chem. , 197-201 (1967), certain dibenzoylhydrazine derivatives including N'-ethyl-; N'-n -propyl-; N'-isobutyl-; N'-neopentyl-; N'-n -heptyl-; and N'-cyclohexylmethyl-N,N'-dibenzoylhydrazines are disclosed.

In 26 J.O.C. , 4336-4340 (1961) N'-t -butyl-N,N'-di-( t -butoxycarbonyl)hydrazide is disclosed.

In 41 J.O.C. , 3763-3765 (1976), N'-t -butyl-N-(phenylmethoxycarbonyl)-N'-(chlorocarbonyl)hydrazide is disclosed.

In 94 J.A.C.S. , 7406-7416 (1972) N'-T -butyl-N,N'-dimethoxycarbonylhydrazide is disclosed.

In 43 J.O.C. , 808-815 (1978), N'-t -butyl-N-ethoxycarbonyl-N'-phenylaminocarbonylhydrazide and N'-t -butyl-N-ethoxycarbonyl-N'-methylaminocarbonylhydrazide are disclosed.

None of the above references discloses any biological activity for the specified compounds.

In 39 J. Econ. Ent. , 416-417 (1946), certain N-phenyl-N'-acylhydrazines are disclosed and evaluated for their toxicity against codling moth larvae.

The N-(optionally substituted)-N'-substituted-N,N'-disubstitutedhydrazines of the present invention differ from known compounds primarily by their N and N' substituents.

Compounds of the present invention are also distinguished by their excellent insecticidal activity, particularly against insects of the orders Lepidoptera and Coleoptera, and most particularly against insects of the order Lepidoptera, without material adverse impact on beneficial insects.

The present invention provides insecticidal compounds which are substituted hydrazines of the formula:

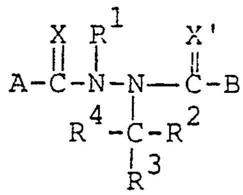

I

wherein

X and X′ are the same or different O, S or NR; $R^1$ is hydrogen; $(C_1\text{-}C_6)$alkyl; $(C_1\text{-}C_6)$alkoxy-$(C_1\text{-}C_6)$alkyl having independently the stated number of carbon atoms in each alkyl group; $(C_1\text{-}C_6)$alkyl-thio-$(C_1\text{-}C_6)$-alkyl having independently the stated number of carbon atoms in each alkyl group; $(C_2\text{-}C_6)$alkenyl; $(C_2\text{-}C_6)$alkynyl; or phen-$(C_1\text{-}C_4)$alkyl where the phenyl ring is unsubstituted or substituted with one to three of the same or different halo, cyano, nitro, hydroxy, $(C_1\text{-}C_4)$alkyl, halo-$(C_1\text{-}C_4)$alkyl $(C_1\text{-}C_4)$alkoxy, halo-$(C_1\text{-}C_4)$alkoxy, carboxy, $(C_1\text{-}C_4)$alkoxy-carbonyl, $(C_1\text{-}C_4)$alkanoyloxy or NZZ′;

$R^2$ and $R^3$ are the same or different hydrogen or $(C_1\text{-}C_4)$alkyl;

$R^4$ is $(C_1\text{-}C_4)$alkyl substituted with 1 to 4 fluoro; straight chain $(C_2\text{-}C_4)$alkenyl; carboxyl; $(C_1\text{-}C_3)$alkoxy-carbonyl; cyano; cyano substituted $(C_1\text{-}C_4)$alkyl; tri$(C_1\text{-}C_2)$alkylsilyl having independently the stated number of carbon atoms in each alkyl group; or tri$(C_1\text{-}C_2)$alkylsilylmethyl having independently the stated number of carbon atoms in each alkyl group; provided that $R^2$ and $R^3$ are both alkyl when $R^4$ is carboxyl or alkoxycarbonyl; and

A and B are the same or different unsubstituted or substituted naphthyl where the substituents can be from one to three of the same or different halo; nitro; $(C_1\text{-}C_4)$alkoxy; $(C_1\text{-}C_4)$alkyl or NZZ′;

unsubstituted or substituted phenyl where the substituents can be from one to five of the same or different halo; nitroso; nitro; cyano; hydroxy; $(C_1\text{-}C_6)$alkyl; halo-$(C_1\text{-}C_6)$alkyl; cyano-$(C_1\text{-}C_6)$alkyl; hydroxy-$(C_1\text{-}C_6)$alkyl; $(C_1\text{-}C_6)$alkoxy; halo-$(C_1\text{-}C_6)$alkoxy; $(C_1\text{-}C_6)$alkoxy-$(C_1\text{-}C_6)$alkyl having independently the stated number of carbon atoms in each alkyl group; $(C_1\text{-}C_6)$alkoxy-$(C_1\text{-}C_6)$alkoxy having independently the stated number of carbon atoms in each alkyl group; -ORSR′ group; $-OCO_2R$ group; $-OCO_2H$ group; $(C_1\text{-}C_6)$alkanoyloxy-$(C_1\text{-}C_6)$alkyl having independently the stated number of carbon atoms in each alkyl group; $(C_2\text{-}C_6)$alkenyl optionally substituted with halo, cyano, $(C_1\text{-}C_4)$alkyl, halo-$(C_1\text{-}C_4)$alkoxy, $(C_1\text{-}C_4)$-alkylthio or $(C_1\text{-}C_4)$alkoxy; $(C_1\text{-}C_4)$alkadienyl; $(C_2\text{-}C_6)$alkenyloxy; $(C_2\text{-}C_6)$alkenyl-carbonyl; $(C_2\text{-}C_6)$-alkenyloxy-carbonyloxy; $(C_2\text{-}C_6)$alkynyl optionally substituted with halo, cyano, nitro, hydroxy, $(C_1\text{-}C_4)$-alkoxy, halo-$(C_1\text{-}C_4)$-alkyl, $(C_1\text{-}C_4)$alkylthio or $(C_1\text{-}C_4)$alkyl; carboxy; $-RCO_2R'$ group; -COR group; -COH group; halo-$(C_1\text{-}C_6)$alkyl-carbonyl; $-CO_2R$ group; $(C_1\text{-}C_6)$haloalkoxy-carbonyl; -OCOR group; $-ORCO_2R'$ group; amino (-NZZ′); amino substituted with hydroxy, $(C_1\text{-}C_4)$alkoxy or $(C_1\text{-}C_4)$alkylthio; -CONZZ′ group; $(C_2\text{-}C_6)$alkenyl-carbonylamino; hydroxy-$(C_1\text{-}C_6)$alkylaminocarbonyl; -OCONZZ′ group; -NZCOZ′ group; $-NZCO_2Z'$ group; thiocyanato; isothiocyanato; thiocyanato-$(C_1\text{-}C_6)$alkyl; $(C_1\text{-}C_6)$alkylthio; halo-$(C_1\text{-}C_6)$-alkylthio; -S(O)Z group; $-SO_2Z$ group; $-OSO_2R$ group; $-OSO_2H$ group; $-SO_2NZZ'$ group; -CSR group; -CSH group; alkylcarbonylthio -SCOR group; -SCOH group; -NZCSZ′ group; unsubstituted or substituted phenyl having one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, amino, $(C_1\text{-}C_4)$alkylamino or di$(C_1\text{-}C_4)$alkylamino having independently the stated number of carbon atoms in each alkyl group; phenoxy where the phenyl ring is unsubstituted or substituted with one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkyl-carbonyl, $(C_1\text{-}C_4)$alkanoyloxy, amino, $(C_1\text{-}C_4)$alkylamino or di$(C_1\text{-}C_4)$alkylamino having independently the stated number of carbon atoms in each alkyl group; benzoyl where the phenyl ring is unsubstituted or substituted with one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, amino, $(C_1\text{-}C_4)$alkylamino or di$(C_1\text{-}C_4)$alkylamino having independently the stated number of carbon atoms in each alkyl group; benzoyloxy-$(C_1\text{-}C_6)$alkyl; phenylthio-$(C_1\text{-}C_6)$alkyl where the phenyl ring is unsubstituted or substituted with one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, amino, $(C_1\text{-}C_4)$alkylamino or di$(C_1\text{-}C_4)$-alkylamino having independently the stated number of carbon atoms in each alkyl group; -CR = N-R> group where R> is hydroxy, $(C_1\text{-}C_4)$alkyl, $(C_1\text{-}C_4)$alkoxy, amino (-NZZ′), phenylamino, -COR, -COH or benzoyl; $(C_2\text{-}C_6)$oxiranyl; acetylthiosemicarbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached,

a 5 or 6 membered dioxolano or dioxano heterocyclic ring;

unsubstituted or substituted ($C_1$-$C_{10}$)alkyl having one to four of the same or different halo, cyano, nitro, hydroxy, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy, phenyl or -NZZ';

unsubstituted or substituted ($C_3$-$C_8$)cycloalkyl or unsubstituted or substituted ($C_3$-$C_8$)cycloalkyl($C_1$-$C_4$)lkyl having one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkanoyl, ($C_1$-$C_4$)alkoxy-carbonyl, ($C_1$-$C_4$)alkanoyloxy or -NZZ';

unsubstituted or substituted ($C_2$-$C_8$)alkenyl or unsubstituted or substituted ($C_2$-$C_8$)alkadienyl having as substituent(s) a furyl, thienyl or pyridyl or one to four of the same or different halo, cyano, nitro, hydroxy, ($C_1$-$C_4$)alkyl, ($C_3$-$C_6$)cycloalkyl, halo-($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy or -NZZ';

unsubstituted or substituted ($C_3$-$C_8$)cycloalkenyl or unsubstituted or substituted ($C_3$-$C_8$)cycloalkadienyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy or -NZZ';

unsubstituted or substituted ($C_2$-$C_8$)alkynyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy, phenyl or -NZZ';

phenalkyl having one to four carbon atoms in the alkyl group and wherein the alkyl group is unsubstituted or substituted with one to three of the same or different halo, cyano, hydroxy, ($C_1$-$C_4$)-alkoxy, ($C_1$-$C_4$)alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, cyano- ($C_1$-$C_4$)-alkyl, ($C_1$-$C_4$)alkoxy, halo-($C_1$-$C_4$)- alkoxy, carboxy, ($C_1$-$C_4$)alkoxy-carbonyl, ($C_1$-$C_4$)alkanoyloxy, ($C_2$-$C_6$)-alkenyl, halo-($C_2$-$C_6$)alkenyl, ($C_2$-$C_6$)alkynyl or -NZZ';

phenalkenyl having two to six carbon atoms in the alkenyl group and the alkenyl group is unsubstituted or substituted with one to three of the same or different halo; cyano, hydroxy, ($C_1$-$C_4$)alkyl, halo-($C_1$-$C_4$)-alkyl, ($C_1$-$C_4$)alkoxy, halo-($C_1$-$C_4$)alkoxy, ($C_1$-$C_4$)alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy or -NZZ';

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; $-RCO_2H'$ group; $-RCO_2R'$ group; -CONZZ' group; amino (-NZZ'); -NZCOZ' group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl or amino (-NZZ'); or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; $-RCO_2H$ group; $-RCO_2R'$ group; -CONZZ' group; amino (-NZZ'); -NZCOZ' group; ($C_1$-$C_6$)alkylthio; or unsubstituted or substituted phenyl having one to three of the same or different halo, nitro, ($C_1$-$C_6$)alky, halo-($C_1$-$C_6$)-alkyl, ($C_1$-$C_6$)alkoxy, halo-($C_1$-$C_6$)alkoxy, carboxy, ($C_1$-$C_4$)alkoxy-carbonyl or amino (-NZZ');
where R, R' and R" are ($C_1$-$C_6$)alkyl; Z and Z' are hydrogen or ($C_1$-$C_4$)alkyl; "amino" means NZZ' or

agronomically acceptable salts thereof.

Insecticidal compositions of the invention comprise one or more of the above compounds together with agronomically acceptable diluent or carrier. The invention also extends to the use of said compounds and compositions as insecticides. The compositions usually contain from about 0.0001% to about 99% by

weight of the compound.

The term "halo" should be understood as including chloro, fluoro, bromo and iodo. The term "alkyl" by itself or as a part of another substituent, unless otherwise stated, includes straight or branched chain groups such as methyl, ethyl, n -propyl, isopropyl, n -butyl, t -butyl, isobutyl, neopentyl and the like and where indicated higher homologues and isomers such as n -octyl, isooctyl and the like. The term "haloalkyl" by itself or as part of another substituent is an alkyl group of the stated number of carbon atoms having one or more halo atoms bonded thereto such as chloromethyl, 1- or 2-bromoethyl, trifluoromethyl and the like. Analogously, "cyanoalkyl" by itself or as part of another group is an alkyl group of the stated number of carbon atoms having one or more cyano groups bonded thereto; "haloalkoxy" by itself or as part of another group is an alkoxy group of the stated number of carbon atoms having one or more halo atoms bonded thereto such as difluoromethoxy, trifluoromethoxy, 2-fluoroethoxy, 2,2,2-trifluoroethoxy and the like. "Alkenyl" and "alkynyl" by themselves or as part of another substituent comprise straight and branched chain groups of the stated number of carbon atoms. "Alkadienyl" is a straight or branched chain alkenyl group comprising two carbon-carbon double bonds that can be conjugated such as 1,3-butadienyl, cumulated such as 1,2-propadienyl or isolated such as 1,4-pentadienyl. Representative examples of five-membered heterocycles includes 2-furyl; 3-furyl; 2-thienyl; 3-thienyl; 4-(1,2,3-triazolyl); 3-(1,2,4-triazolyl); 5-(1,2,4-triazolyl); 2-pyrrolyl; 2-oxazolyl; and the like.

Because of their anticipated good insecticidal activity, compounds of the present invention for use in the insecticidal compositions and formulations include those wherein any one or combination of two or more of the substituents conform to the following definitions:

X and X′ are O or S;

$R^1$ is hydrogen; $(C_1$-$C_4)$alkyl; $(C_1$-$C_4)$alkoxy-$(C_1$-$C_4)$alkyl having independently the stated number of carbon atoms in each alkyl group; methylthiomethyl; $(C_2$-$C_5)$alkenyl; $(C_2$-$C_5)$-alkynyl; or phen-$(C_1$-$C_2)$-alkyl where the phenyl ring is unsubstituted or substituted with one or two halo, nitro, $(C_1$-$C_4)$alkyl or $(C_1$-$C_4)$alkoxy;

$R^2$ and $R^3$ the same or different hydrogen or $(C_1$-$C_3)$alkyl;

$R^4$ is $(C_1$-$C_3)$alkyl substituted with one to four fluoro; straight chain $(C_2$-$C_4)$alkenyl; carboxyl; $(C_1$-$C_2)$-alkoxy-carbonyl; cyano; cyano substituted $(C_1$-$C_3)$alkyl; tri$(C_1$-$C_2)$alkylsilyl having independently the stated number of carbon atoms in each alkyl group; or tri$(C_1$-$C_2)$alkylsilylmethyl having independently the stated number of carbon atoms in each alkyl group; provided that $R^2$ and $R^3$ are both alkyl when $R^4$ is carboxyl or alkoxycarbonyl; and

A and B are the same or different unsubstituted naphthyl;

unsubstituted or substituted phenyl where the substituents can be from one to three of the same or different halo; nitro; cyano; $(C_1$-$C_4)$alkyl; halo-$(C_1$-$C_4)$alkyl; cyano-$(C_1$-$C_4)$alkyl; $(C_1$-$C_4)$alkoxy; $(C_1$-$C_4)$-alkoxy-$(C_1$-$C_4)$alkyl having independently the stated number of carbon atoms in each alkyl group; -COZ; carboxy; $(C_1$-$C_4)$alkoxy-carbonyl; $(C_1$-$C_4)$alkanoyloxy; $(C_2$-$C_6)$alkenyl; $(C_2$-$C_6)$alkynyl; amino (-NZZ′); thiocyanato; $(C_1$-$C_4)$alkylthio; -CSR group; -CSH; unsubstituted or substituted phenyl having one to two of the same or different halo, nitro, $(C_1$-$C_4)$alkyl, $(C_1$-$C_4)$alkoxy, carboxy, -NZZ′; phenoxy where the phenyl ring is unsubstituted or substituted with one or two of the same or different halo, nitro, $(C_1$-$C_4)$-alkyl, $(C_1$-$C_4)$alkoxy, carboxy, -NZZ′; or when two adjacent positions on the phenyl ring are substituted with alkoxy groups, these groups may be joined to form together with the carbon atoms to which they are attached a 5- or 6-membered dioxolano or dioxano heterocyclic ring;

unsubstituted or substituted $(C_1$-$C_8)$alkyl having one to three of the same or different halo, cyano, $(C_1$-$C_4)$alkoxy, phenyl, $(C_1$-$C_4)$alkoxy-carbonyl or halo-$(C_1$-$C_4)$alkoxy;

unsubstituted or substituted $(C_3$-$C_6)$cycloalkyl or unsubstituted or substituted $(C_3$-$C_6)$cycloalkyl$(C_1$-$C_4)$-alkyl having one or two of the same or different halo, $(C_1$-$C_4)$alkyl, halo-$(C_1$-$C_4)$alkyl, $(C_1$-$C_4)$alkoxy, $(C_1$-$C_4)$alkanoyl or halo-$(C_1$-$C_4)$alkoxy;

unsubstituted or substituted $(C_2$-$C_6)$alkenyl having as substituent(s) a furyl or one to three of the same or different $(C_1$-$C_4)$alkyl, halo-$(C_1$-$C_4)$alkyl, $(C_1$-$C_4)$alkoxy or halo-$(C_1$-$C_4)$alkoxy;

unsubstituted or substituted $(C_3$-$C_6)$cycloalkenyl having as substituent(s) one or two of the same or different halo, $(C_1$-$C_4)$alkyl, halo-$(C_1$-$C_4)$alkyl, $(C_1$-$C_4)$alkoxy or halo-$(C_1$-$C_4)$alkoxy;

unsubstituted or phenyl substituted alkynyl; phenalkyl having one or two carbon atoms in the alkyl group and the alkyl group is unsubstituted or substituted with one or two of the same or different halo, $(C_1$-$C_4)$-

alkyl, halo-($C_1$-$C_4$)alkyl, ($C_1$-$C_4$)alkoxy or halo-($C_1$-$C_4$)alkoxy and the phenyl ring is unsubstituted or substituted with one or two of the same or different halo, cyano, ($C_1$-$C_4$)alkyl, halo-($C_1$-$C_4$)alkyl, ($C_1$-$C_4$)-alkoxy or halo-($C_1$-$C_4$)alkoxy;

phenalkenyl having two or three carbon atoms in the alkenyl group and the alkenyl group is unsubstituted or substituted with halo, ($C_1$-$C_4$)alkyl, halo-($C_1$-$C_4$)alkyl, ($C_1$-$C_4$)alkoxy or halo-($C_1$-$C_4$)alkoxy and the phenyl ring is unsubstituted or substituted with one or two of the same or different halo, cyano, ($C_1$-$C_4$)alkyl, halo-($C_1$-$C_4$)alkyl, ($C_1$-$C_4$)alkoxy or halo-($C_1$-$C_4$)alkoxy;

unsubstituted or substituted five-membered heterocycle selected from furyl, thienyl, triazolyl, pyrrolyl, and oxazolyl where the substituents can be one or two of the same or different halo; nitro; ($C_1$-$C_4$)alkyl; ($C_1$-$C_4$)alkoxy; -NZZ′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NZZ′; or

unsubstituted or substituted six-membered heterocycle having one or two nitrogen atoms and 4 to 5 nuclear carbon atoms where the substituents can be one or two of the same or different halo; nitro; ($C_1$-$C_4$)alkyl; ($C_1$-$C_4$)alkoxy; thio-($C_1$-$C_4$)alkoxy; -NZZ′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NZZ′;

where Z and Z′ are hydrogen or ($C_1$-$C_4$)alkyl; and
agronomically acceptable salts.

Insecticidal compounds of the present invention believed to have very good activity for use in the insecticidal compositions and formulations of the present invention include those where any one or combination of two or more of the substituents conform to the following definitions:

X and X′ are O or S;

$R^1$ is hydrogen; ($C_1$-$C_4$)alkyl; ($C_1$-$C_4$)alkoxy-($C_1$-$C_4$)alkyl having independently the stated number of carbon atoms in each alkyl group; ($C_2$-$C_5$)alkenyl; ($C_2$-$C_5$)alkynyl; or benzyl where the phenyl ring is unsubstituted or substituted with one halo, nitro, ($C_1$-$C_4$)alkyl or ($C_1$-$C_4$)alkoxy;

$R^2$ and $R^3$ are the same or different hydrogen or ($C_1$-$C_2$)alkyl;

$R^4$ is ($C_1$-$C_3$)alkyl substituted with 1 to 4 fluoro; straight chain ($C_2$-$C_4$)alkenyl; methoxycarbonyl; cyano; cyano substituted ($C_1$-$C_2$)alkyl; tri($C_1$-$C_2$)alkylsilyl having independently the stated number of carbon atoms in each alkyl group; or tri($C_1$-$C_2$)alkylsilylmethyl having independently the stated number of carbon atoms in each alkyl group; provided that $R^2$ and $R^3$ are both alkyl when $R^4$ is methoxycarbonyl; and

A and B are the same or different unsubstituted naphthyl;

unsubstituted or substituted phenyl having one to three of the same or different halo; nitro; cyano; ($C_1$-$C_4$)alkyl; halo-($C_1$-$C_4$)alkyl; cyano($C_1$-$C_4$)alkyl; ($C_1$-$C_4$)alkoxy; ($C_1$-$C_4$)alkoxy-($C_1$-$C_4$)alkyl having independently the stated number of carbon atoms in each alkyl group; -COZ; ($C_1$-$C_4$)alkoxy-carbonyl; ($C_1$-$C_4$)-alkanoyloxy;

thiocyanato; -NZZ′; ($C_1$-$C_4$)alkylthio; -CSZ; -$CS_2$Z; unsubstituted or substituted phenyl having one or two of the same or different halo, nitro, ($C_1$-$C_4$)alkyl, ($C_1$-$C_4$)alkoxy, carboxy, -NZZ′; or phenoxy where the phenyl ring is unsubstituted or substituted with one or two of the same or different halo, nitro, ($C_1$-$C_4$)-alkyl, ($C_1$-$C_4$)alkoxy, carboxy, -NZZ′; or when two adjacent positions on the phenyl ring are substituted with alkoxy groups, these groups may be joined to form together with the carbon atoms to which they are attached a 5- or 6-membered dioxolano or dioxano heterocyclic ring;

unsubstituted or substituted ($C_1$-$C_6$)alkyl having one to three of the same or different halo, cyano, nitro, carboxy, ($C_1$-$C_4$)alkoxy, phenyl, ($C_1$-$C_4$)alkoxy-carbonyl or halo-($C_1$-$C_4$)alkoxy;

unsubstituted or substituted ($C_3$-$C_6$)cycloalkyl or unsubstituted or substituted ($C_3$-$C_6$)cycloalkyl($C_1$-$C_4$)-alkyl, where the substituent is halo, ($C_1$-$C_4$)alkyl, ($C_1$-$C_4$)alkanoyl, ($C_1$-$C_4$)alkoxy or halo-($C_1$-$C_4$)alkoxy;

unsubstituted or substituted ($C_2$-$C_6$)alkenyl having one or two of the same or different halo, ($C_1$-$C_4$)alkyl, ($C_1$-$C_4$)alkoxy or halo-($C_1$-$C_4$)alkoxy;

unsubstituted or substituted ($C_4$-$C_6$)cycloalkenyl where the substituent is halo ($C_1$-$C_4$)alkyl, ($C_1$-$C_4$)alkoxy or halo-($C_1$-$C_4$)alkoxy;

unsubstituted or phenyl substituted alkynyl;

benzyl where the phenyl ring is unsubstituted or substituted with one or two of the same or different halo, methyl or ethyl;

unsubstituted or substituted five-membered heterocycle selected from furyl, thienyl, pyrrolyl and oxazolyl where the substituents can be one or two of the same or different halo, nitro, $(C_1-C_4)$alkyl or $(C_1-C_4)$-alkoxy; or

unsubstituted or substituted six-membered heterocycle having one or two nitrogen atoms and four to five nuclear carbon atoms where the substituents can be one or two of the same or different halo, nitro, $(C_1-C_4)$alkyl, $(C_1-C_4)$alkoxy or thio-$(C_1-C_4)$alkoxy;

where Z and Z′ are hydrogen or $(C_1-C_4)$alkyl; including
agronomically acceptable salts thereof.

Because of their anticipated excellent insecticidal activity, preferred compounds of the present invention for use in the insecticidal compositions and formulations of the present invention include those where any one or combination of two or more of the substituents conforms to the following definitions:

X and X′ are O;

$R^1$ is hydrogen; methyl; ethyl; $(C_1-C_2)$alkoxy-$(C_1-C_2)$alkyl having independently the stated number of carbon atoms in each alkyl group $(C_2-C_5)$alkenyl; $(C_2-C_5)$alkynyl; or benzyl where the phenyl ring is unsubstituted or substituted with halo;

$R^2$ and $R^3$ are the same or different hydrogen or $(C_1-C_2)$alkyl;

$R^4$ is $(C_1-C_2)$alkyl substituted with 1 to 4 fluoro; straight chain $(C_2-C_4)$alkenyl; cyano; cyano substituted $(C_1-C_2)$alkyl; tri$(C_1-C_2)$alkylsilyl having independently the stated number of carbon atoms in each alkyl group; or tri$(C_1-C_2)$alkylsilylmethyl having independently the stated number of carbon atoms in each alkyl group; and

A and B are the same or different phenyl or substituted phenyl where the substituents can be from one to three of the same or different halo, nitro, $(C_1-C_4)$alkyl, $(C_1-C_4)$alkoxy or halo-$(C_1-C_4)$alkyl;

unsubstituted or substituted $(C_1-C_6)$alkyl having one to two of the same or different halo, phenyl or cyano;

unsubstituted $(C_4-C_6)$cycloalkyl;

unsubstituted or substituted $(C_2-C_6)$alkenyl having one to three of the same or different halo or $(C_1-C_4)$-alkyl;

unsubstituted $(C_4-C_6)$cycloalkenyl;

phenalkyl having one or two carbon atoms in the alkyl group and the phenyl ring is unsubstituted or substituted with one or two of the same or different halo, $(C_1-C_4)$alkyl or $(C_1-C_4)$alkoxy;

unsubstituted or substituted pyridyl or 2,5-pyrazinyl where the substituent can be halo, nitro, $(C_1-C_4)$alkyl, $(C_1-C_4)$alkoxy or $(C_1-C_4)$thioalkoxy; or

unsubstituted furyl or thienyl or an unsubstituted or substituted pyrrolyl where the substituent can be $(C_1-C_4)$alkyl;

including
agronomically acceptable salts thereof.

Because of their anticipated outstanding insecticidal activity, more preferred compounds of the present invention for use in the insecticidal compositions and formulations of the present invention include those where any one or combination of two or more of the substituents conforms to the following definitions:

X and X′ are O;

$R^1$ is hydrogen; methyl; methoxymethyl; $(C_2-C_4)$alkenyl; $(C_2-C_5)$alkynyl; benzyl; or 4-halobenzyl;

$R^2$ and $R^3$ are the same or different hydrogen, methyl or ethyl;

$R^4$ is trifluoromethyl; 2,2,2-trifluoroethyl; straight chain $(C_2-C_4)$alkenyl; cyano; cyanomethyl; tri$(C_1-C_2)$-

alkylsilyl having independently the stated number of carbon atoms in each alkyl group; or trimethylsilylmethyl; and

A and B are the same or different phenyl or substituted phenyl where the substituents can be one, two or three of the same or different chloro, fluoro, bromo, iodo, nitro, methyl, ethyl, methoxy or trifluoromethyl; ($C_2$-$C_3$) substituted alkenyl having one to three of the same or different chloro, bromo, methyl or ethyl;

cyclohexenyl;

benzyl;

unsubstituted or substituted pyridyl or 2,5-pyrazinyl where the substituent can be halo, methyl, ethyl or methoxy; or

unsubstituted furyl or thienyl or an unsubstituted or substituted pyrrolyl where the substituent can be ethyl or methyl;

including

agronomically acceptable salts thereof.

Because of their relatively low insecticidal activity at low application rates, less preferred compounds are those wherein $R^4$ is $CF_3$ and A and B are unsubstituted phenyl or $R^4$ is trimethylsilyl and (a) A and B are 4-chlorophenyl or (b) A is 4-ethylphenyl and B is 3,5-dimethylphenyl.

Those N'-substituted,N,N'-diacylhydrazines of Formula I which possess acidic or basic functional groups may be further reacted to form novel salts with appropriate bases or acids. These salts also exhibit pesticidal activity. Typical salts are the agronomically acceptable metal salts, ammonium salts and acid addition salts. Among the metal salts are those in which the metal cation is an alkali metal cation such as sodium, potassium, lithium or the like; alkaline earth metal cation such as calcium, magnesium, barium, strontium or the like; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$ and $R^8$ are independently hydrogen, hydroxy, ($C_1$-$C_4$)-alkoxy, ($C_1$-$C_{20}$)alkyl, ($C_3$-$C_8$)alkenyl, ($C_3$-$C_8$)alkynyl, ($C_2$-$C_8$)hydroxyalkyl, ($C_2$-$C_8$)alkoxyalkyl, ($C_2$-$C_6$)-aminoalkyl, ($C_2$-$C_6$)haloalkyl, amino, ($C_1$-$C_4$)alkyl- or ($C_1$-$C_4$)dialkylamino, substituted or unsubstituted phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, ($C_1$-$C_8$)alkyl, ($C_1$-$C_4$)alkoxy, hydroxy, nitro, trifluoromethyl, cyano, amino, ($C_1$-$C_4$)alkylthio and the like. Such substituted phenyl groups preferably have up to two such substituents. Represenative ammonium cations include ammonium, dimethylammonium, 2-ethylhexylammonium, bis(2-hydroxyethyl)ammonium, tris(2-hydroxyethyl)ammonium, dicyclohexylammonium, t -octylammonium, 2-hydroxyethylammonium, morpholinium, piperidinium, 2-phenethylammonium, 2-methylbenzylammonium, n -hexylammonium, triethylammonium, trimethylammonium, tri( n -butyl)-ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium, 4-methylbenzyltrimethylammonium, and the like. Among the acid addition salts are those in which the anion is an agronomically acceptable anion such as hydrochloride, hydrobromide, sulfate, nitrate, perchlorate, acetate, oxalate and the like.

The above-identified preferred embodiments are based on the 34 examples which were made, tested and disclosed infra and the disclosure in the following six related European applications:

| U.S. Application No. | EPC Application No. |
|---|---|
| 911 177<br>789 797 | 86308068.5 |
| 858 482 | 87303092.8 |
| 821 187 | 87300534.2 |
| 835 073<br>005 824 | 87301730.5 |
| 012 380<br>885 508 | 87303090.2 |
| 911 928 | 87303091.0 |

These related applications disclose nearly 1,000 active compounds in which the presently disclosed $R^2$, $R^3$ and $R^4$ are methyl and A and B are various unsubstituted and substituted moieties including alkyl, phenyl and 5- and 6-membered heterocyclic rings.

The present inventors have determined that a critical feature of the structure which leads to activity is not the t -butyl substituent of the related applications ($R^2 = R^3 = R^4$ = methyl) but that one of the nitrogens be substituted with a bulky substituent. This bulky substituent is preferably a trisubstituted alpha carbon or a trisubstituted beta carbon. However, the bulky substituent may also be a trisubstituted gamma carbon or a disubstituted alpha carbon.

Compounds of the present invention which were made and used in insecticidal compositions and formulations include those where, independently,

X and X′ are O;

$R^1$ is hydrogen;

$R^2$ and $R^3$ are hydrogen, methyl or ethyl;

$R^4$ is trifluoromethyl, cyano, allyl, methoxycarbonyl, or trimethylsilyl; and

A and B are the same or different unsubstituted or substituted phenyl having one or two of the same or different chloro, fluoro, nitro, methyl or ethyl; or

unsubstituted thienyl.

Since one of ordinary skill in the art would anticipate homologues to be active, the present invention also includes compounds for use in insecticidal compositions and formulations where any one or combination of two or more of the substituents conforms to the following definitions:

X and X′ are O;

$R^1$ is hydrogen or methyl;

$R^2$ and $R^3$ are hydrogen or $(C_1-C_3)$alkyl;

$R^4$ is trifluoromethyl, ${}^2{}_1{}^2{}_1$2-trifluoroethyl, cyano, cyanomethyl, straight chain $(C_2-c_4)$-alkenyl, carboxyl, methoxycarbonyl, tri$(C_1-C_2)$alkylsilyl having independently the stated number of carbon atoms in each alkyl group or tri$(C_1-C_2)$alkylsilylmethyl having independently the stated number of carbon atoms in each alkyl group; and

A and B are the same or different thienyl or unsubstituted phenyl or substituted phenyl where the substituents can be one to three of the same or different chloro, fluoro, nitro, methyl, ethyl or propyl; including

agronomically acceptable salts thereof.

The compounds of this invention or their precursors can be prepared according to the following processes. Process A can be used when preparing compounds according to Formula I where X and X′ are both oxygen and A and B are the same (for example, both A and B are phenyl or 4-chlorophenyl) or different (for example, A is 4-methylphenyl and B is 4-chlorophenyl).

Process A:

Step 1

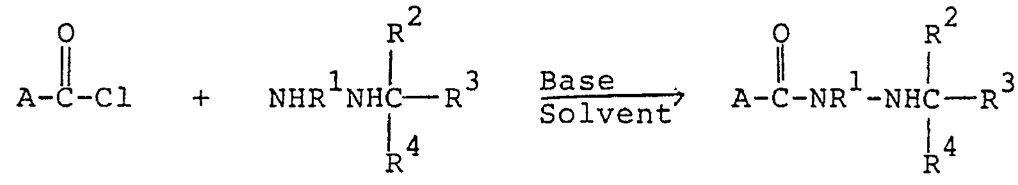


II III IV

Step 2

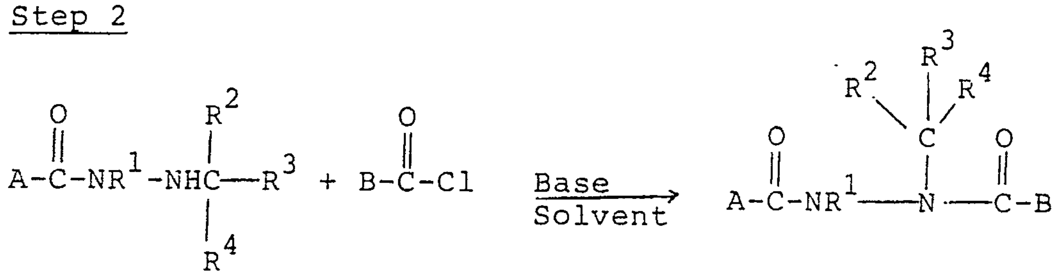


IV V I

where $R^1$, $R^2$, $R^3$, $R^4$, A and B are as defined above for Formula I and X and X′ are oxygen.

When $R^4$ is cyano the intermediate IV may be made as follows:

Step 1a

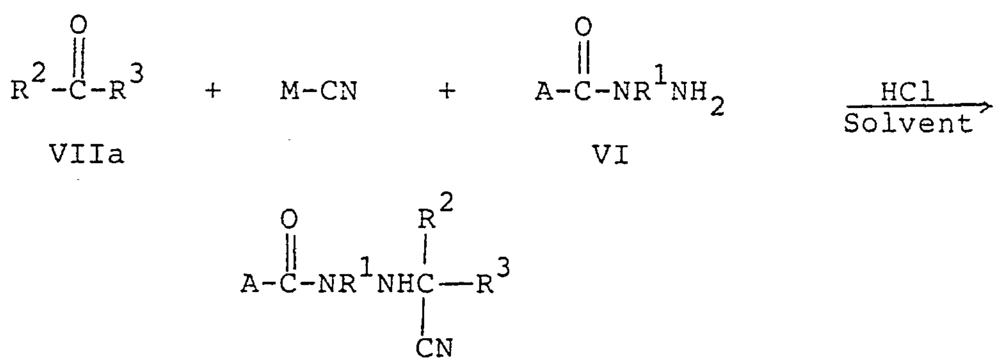


VIIa VI

IVa

where M = K, Na

Process B can be used when preparing compounds according to Formula I where X and X′ are oxygen, and $R^1$, $R^3$, $R^4$, A and B are as defined above for Formula I.

Process B:

Method 1

Step 1

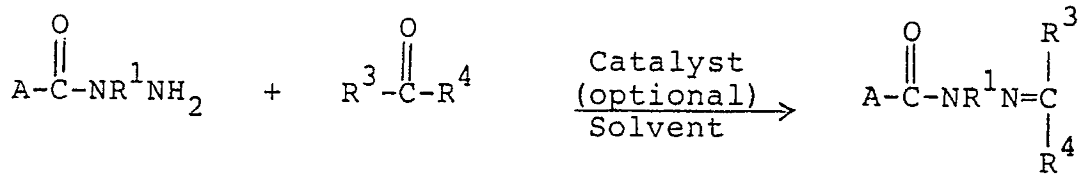


VI VII VIII

Step 2

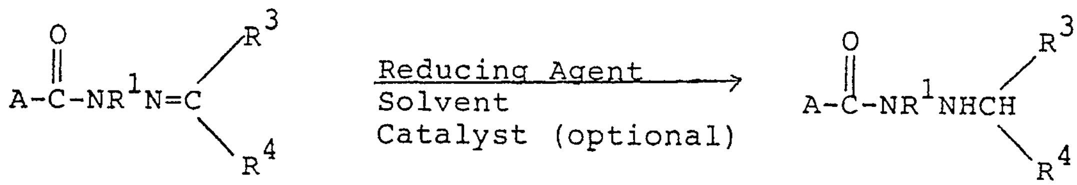


VIII IX (IV)

Step 3

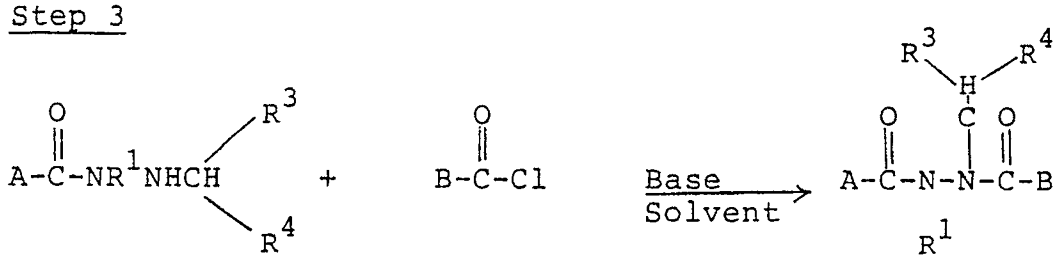


IX (IV) V X (I)

As can be seen above, the intermediate product of Step 2, the compounds of Formula IX, corresponds to the compounds of Formula IV. In addition, the compound of Formula X corresponds to the compounds of Formula I where X and X' are oxygen.

Method 2

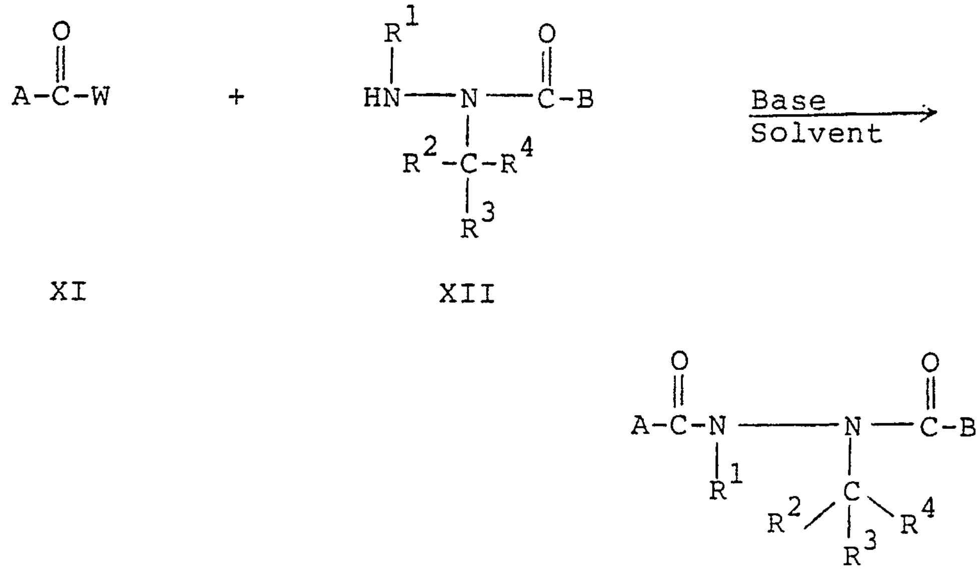


XI XII

I

where $R^1$, $R^2$, $R^3$, $R^4$, A and B are as defined above for Formula I and W is a good leaving group such as halo, for example, chloro; an alkoxy, for example, ethoxy; methyl sulfonate ($-OSO_2CH_3$) ; or an ester, for example, acetate ($-OC(O)CH_3$) .

Process C can be used when preparing compounds according to Formula I where A, B and $R^1$ are as defined for Formula I and one or both X and X' are sulfur.

Process C:

Step 1:

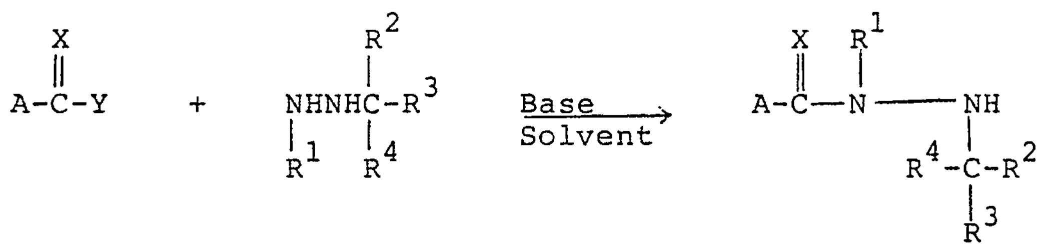


XIII III XIV

Step 2:

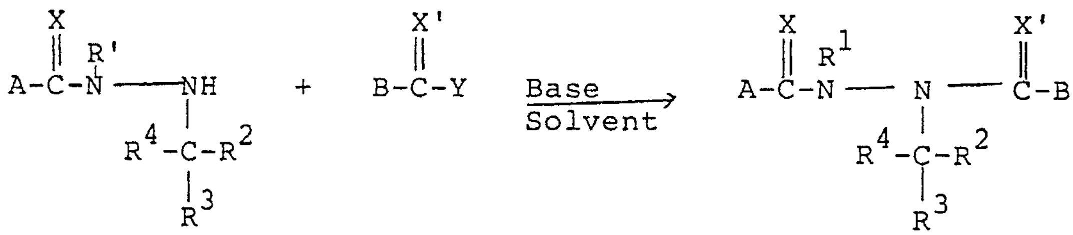


XIV XV I

where $R^1$, $R^2$, $R^3$, $R^4$, A and B are as defined above for Formula I and one or both X and X' are sulfur, and Y is a good leaving group such as carboxyalkylthio (for example, carboxymethylthio, - $SCH_2CO_2H$) ; alkylthio (for example, methylthio); or halo (for example, chloro).

Process D can be used when preparing compounds according to Formula I where X and X' are oxygen and $R^1$, A and B are as defined above for Formula I.

Process D:

Step 1:

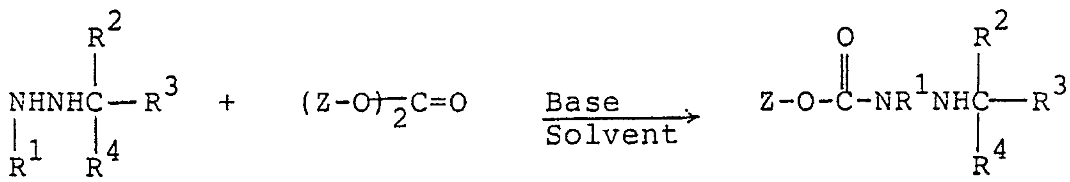


III XVI XVII

Step 2

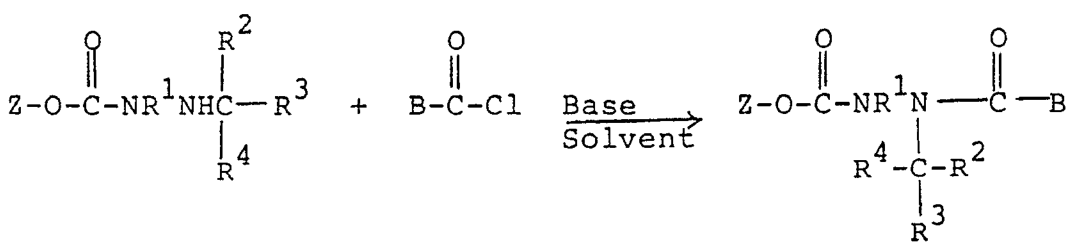


XVII V XVIII

Step 3

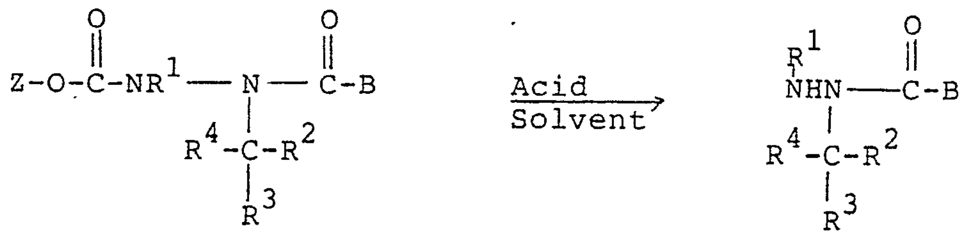


XVIII XIX

Step 4

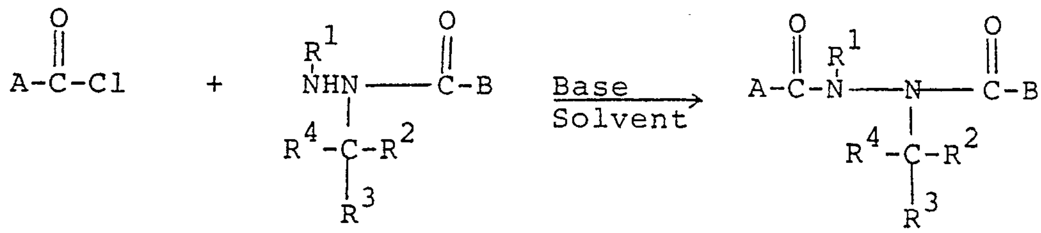


II XIX I

wherein $R^1$, $R^2$, $R^3$, $R^4$, A and B are as defined above for Formula I and Z is t -butyl; ethyl; phenyl; or benzyl.

The starting materials for each process are generally commercially available, or can be prepared by generally customary and known methods.

In Process A, a compound of Formula II is reacted with a monosubstituted hydrazine of Formula III or a corresponding acid addition salt such as the hydrochloride salt or the like in the presence of a base in an inert or substantially inert solvent or mixture of solvents to afford an intermediate product of Formula IV which can be isolated or further reacted with a compound of Formula V in the presence of a base in an inert or substantially inert solvent or mixture of solvents to afford the desired product of Formula I.

When A and B are the same, for example, both A and B are 4-chlorophenyl, two equivalents of a compound of Formula II or V are reacted with a monosubstituted hydrazine of Formula III in the presence of a base in an inert or substantially inert solvent or mixture of solvents to afford the desired product of Formula I.

Examples of the compounds of Formula II and/or Formula V which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride, 3-toluoyl chloride, 4-toluoyl chloride, 4-ethylbenzoyl chloride, 2-nitrobenzoyl chloride, 2,3-dimethylbenzoyl chloride, 2-nitro-5-toluoyl chloride and the like. The compounds of Formula II and/or Formula V are generally commercially available or can be prepared by known procedures.

Examples of the compounds of Formula III which can be used in the above processes include 1,1-dimethyl-3-butenylhydrazine, (trimethylsilylmethyl)hydrazine, (1,1,1-trifluoro-2-propyl)hydrazine, (2,2,2-trifluoroethyl)hydrazine, (1-cyano-1-methyl)ethylhydrazine and the like. The compounds of Formula III are generally commercially available or can be prepared by known procedures. For example, the Grignard reagent addition product of acetone azine in diethyl ether is hydrolyzed by the addition of an acid (such as oxalic acid), in a suitable solvent or mixture of solvents (such as ethanol and diethyl ether, 1:1) to afford the monosubstituted hydrazine of Formula III.

Suitable solvents for use in the above processes include water; alcohols such as methanol, ethanol, isopropanol and the like; hydrocarbons such as toluene, xylene, hexane, heptane and the like, glyme; tetrahydrofuran; acetonitrile; pyridine; or haloalkanes such as methylene chloride or mixtures of these solvents.

Preferred solvents are water, toluene, methylene chloride or a mixture of these solvents.

Examples of bases for use in the above processes include tertiary amines such as triethylamine;

pyridine; potassium carbonate; sodium carbonate; sodium bicarbonate; sodium hydroxide; or potassium hydroxide. Preferred bases are sodium hydroxide, potassium hydroxide or triethylamine.

In Process B, Method 1, a compound of Formula VI is reacted with a ketone or aldehyde of Formula VII in an inert or substantially inert solvent or mixture of solvents and optionally in the presence of a catalyst to afford an intermediate product of Formula VIII. The intermediate product of Formula VIII is then further reacted with a reducing agent in an inert or substantially inert solvent or mixture of solvents to afford a second intermediate product of Formula IX (IV) which can be isolated or further reacted with a compound of Formula V in the presence of a base in an inert or substantially inert solvent or mixture of solvents to afford the desired product of Formula X (I).

Examples of the compounds of Formula VI which can be used in the above Process B, Method 1, include benzoylhydrazine, 4-chlorobenzoylhydrazine, 2-methylbenzoylhydrazine, 4-methylbenzoylhydrazine, 3,5-dichlorobenzoylhydrazine and the like. The compounds of Formula VI are generally commercially available or can be prepared by known procedures.

Examples of the compounds of Formula VII which can be used in the above Process B, Method 1, include trifluoroacetone, methacrolein, ethyl pyruvate and the like. The compounds of Formula VII are generally commercially available or can be prepared by known procedures.

Optionally, a catalyst may be used in Step 1, Method 1 of Process B. Suitable catalysts generally include organic acids such as acetic acid, trifluoroacetic acid, oxalic acid and the like; mineral acids such as hydrochloric acid, sulfuric acid, nitric acid and the like; arylsulfonic acids such as toluenesulfonic acid; or pyridinium toluenesulfonate. Preferred catalysts are organic acids or arylsulfonic acids. Most preferred catalysts are acetic acid or trifluoroacetic acid.

Suitable solvents for use in the above Process B, Method 1, Step 1, include alcohols such as methanol, ethanol, isopropanol and the like; hydrocarbons such as toluene, benzene; ethers such as tetrahydrofuran (THF), glyme and the like; or dimethylformamide. Preferred solvents are alcohols and hydrocarbons. Most preferred solvents are alcohols such as methanol or ethanol.

Examples of suitable reducing agents for use in the above Process B, Method 1, Step 2, include hydrides such as sodium borohydride and derivatives thereof such as sodium cyanoborohydride, lithium aluminum hydride and derivatives thereof and the like; or diborane. Preferred reducing agents are sodium borohydride and derivatives thereof or lithium aluminum hydride and derivatives thereof. Most preferred as reducing agents are borane and diborane.

Optionally, in Process B, Method 1, Step 2, a catalyst may be included. Examples of suitable catalysts include organic acids such as acetic acid, trifluoroacetic acid; or mineral acids such as hydrochloric acid, sulfuric acid and the like. Preferred catalysts are organic acids or hydrochloric acid. Most preferred catalysts are acetic acid, trifluoroacetic acid or hydrochloric acid.

Suitable solvents for use in the above Process B, Method 1, Step 2, include alcohols such as methanol, ethanol, isopropanol and the like; ethers such as tetrahydrofuran (THF), diethylether, glyme and the like; or halohydrocarbons such as methylene chloride, chloroform and the like. Preferred solvents are alcohols and most preferred are methanol or ethanol.

Step 3 of Process B, Method 1 corresponds to Step 2 of Process A. Consequently, those bases and solvents suitable for use in Step 2 of Process A are suitable for use in Step 3, Method 1 of Process B including the preferred bases and solvents described above.

In Process B, Method 2, and N′-substituted-N′-benzoylhydrazine of Formula XII is reacted with a compound of Formula XI in the presence of a base in an inert or substantially inert solvent or mixture of solvents to afford the desired product of Formula I.

The compounds of Formula XI are generally commercially available or can be prepared from commercially available compounds by procedures well known to those skilled in the art as described below.

Examples of the compounds of Formula XII which can be used in the above Process B, Method 2, include N′-(1,1-dimethyl-3-butenyl)-N′-benzoylhydrazine, N′-(trimethylsilylmethyl)-N′-(4-methylbenzoyl)-hydrazine, N′-(1,1,1-trifluoro-2-propyl)-N′-(2,4-dichlorobenzoyl)hydrazine, N′-(1-cyano-1-methyl)ethyl-N′-(2-methylbenzoyl)hydrazine and the like.

Suitable solvents for use in the above Process B, Method 2, include water; hydrocarbons such as toluene, xylene, hexane, heptane and the like; alcohols such as methanol, ethanol, isopropanol, and the like; glyme; tetrahydrofuran; acetonitrile; pyridine; or haloalkanes such as methylene chloride; or mixtures of these solvents. Preferred solvents are water, toluene, methylene chloride or a mixture of these solvents.

Examples of bases suitable for use in the above Process C includes tertiary amines such as triethylamine; pyridine; potassium carbonate; sodium carbonate; sodium bicarbonate; sodium hydroxide; or potassium hydroxide. Preferred bases are sodium hydroxide, or triethylamine.

The compounds of Formula XI are commercially available, such as nicotinoyl chloride hydrochloride,

isonicotinoyl chloride hydrochloride and ethyl picolinate or can be prepared from commercially available materials by procedures known to those skilled in the art.

The compounds of Formula XII can be prepared by procedures known to those skilled in the art from commercially available reactants. By way of example, a suitably substituted hydrazine (such as (1,1,1-trifluoro-2-propyl)hydrazine) is reacted with an aldehyde or ketone (such as acetone) in the presence of a base (such as triethylamine) to afford a hydrazone which is then reacted with a benzoyl chloride in an inert or substantially inert solvent or mixture of solvents in the presence of a base (such as sodium hydroxide) to afford an N'-substituted-N'-benzoylhydrazone which is then reacted with an acid (such as hydrochloric acid) to afford the compound of Formula XII. Alternatively, a suitable substituted hydrazine (such as (trimethylsilylmethyl)hydrazine) is reacted with di-tert -butyldicarbonate in an inert or substantially inert solvent or mixture of solvents (such as toluene/water) to afford an N'-(trimethylsilylmethyl)-N-t -butoxycarbonylhydrazine which is then reacted with a benzoylchloride in an inert or substantially inert solvent or mixture of solvents to afford an N'-(trimethylsilylmethyl)-N'-benzoyl-N-t -butoxycarbonylhydrazine which is then reacted with an acid to afford the desired compound of Formula XII.

In Process C, a compound of Formula XIII is reacted with a monosubstituted hydrazine of Formula III or a corresponding acid addition salt such as the hydrochloride salt or the like in the presence of a base in an inert or substantially inert solvent or mixture of solvents to afford an intermediate compound of Formula XIV which can be isolated or further reacted with a compound of Formula XV in the presence of a base in an inert or substantially inert solvent or mixture of solvents to afford the desired product of Formula I.

In Process D, a monosubstituted hydrazine of Formula III or a corresponding acid addition salt, such as the hydrochloride salt or the like, is reacted with a compound of Formula XVI in the presence of a base in an inert or substantially inert solvent or mixture of solvents to afford an intermediate product of Formula XVII. The intermediate product of Formula XVII is then further reacted with a compound of Formula V in the presence of a base in an inert or substantially inert solvent or mixture of solvents to afford a second intermediate product of Formula XVIII. The second intermediate product of Formula XVIII is then further reacted with an acid in an inert or substantially inert solvent or mixture of solvents to afford a third intermediate product of Formula XIX. The third intermediate product of Formula XIX is then further reacted with a compound of Formula II in the presence of a base in an inert or substantially inert solvent or mixture of solvents to afford the desired product of Formula I.

Examples of the compounds of Formula XVI which can be used in the above Process D include di-t -butylcarbonate, diethylcarbonate, diphenylcarbonate, dibenzylcarbonate and the like. The compounds of Formula XVI are generally commercially available or can be prepared by known procedures.

Suitable solvents for use in the above Process D, Steps 1, 2 and 4 include water; tetrahydrofuran; dioxane; toluene; alcohols such as methanol, ethanol and isopropanol; hexane; acetonitrile; pyridine; and haloalkanes such as methylene chloride; or mixtures of these solvents.

Preferred solvents are dioxane; toluene; tetrahydrofuran; pyridine; methylene chloride or water.

Most preferred solvents are dioxane; water or toluene.

Examples of the bases for use in the above Process D, Steps 1, 2 and 4 include tertiary amines such as triethylamine; pyridine; potassium carbonate, sodium carbonate; sodium bicarbonate; sodium hydroxide; and potassium hydroxide.

Preferred bases are sodium hydroxide; potassium hydroxide; pyridine or triethylamine.

Suitable solvents for use in the above Process D, Step 3 include alcohols such as methanol, ethanol and isopropanol; water; tetrahydrofuran; dioxane; and acetonitrile.

Preferred solvents are methanol or ethanol.

Examples of acids for use in the above Process D, Step 3 include concentrated hydrochloric acid or concentrated sulfuric acid.

When A and B are the same, for example, both A and B are unsubstituted phenyl, two equivalents of a compound Formula XIII or XV are reacted with a mono-substituted hydrazine of Formula III in the presence of a base in an inert or substantially inert solvent or mixture of solvents to afford the desired product of Formula I.

Examples of the compounds of Formula XIII and/or Formula XV which can be used in the above Process C include 3-methyl-methylthio-thiobenzoate, 4-chloromethylthio-thiobenzoate, 4-methyl-methylthio-thiobenzoate, carboxymethylthio-thiobenzoate and the like. The compounds of Formula XIII and/or Formula XIV are generally commercially available or can be prepared by known procedures.

Suitable solvents for use in the above Process C are generally polar high-boiling solvents such as dimethylformamide (DMF); glyme; tetrahydrofuran (THF); and pyridine. The preferred solvent is pyridine.

Suitable bases for use in the above Process C include tertiary amines such as triethylamine; and pyridine. The preferred base is pyridine.

The above Processes A and B, Method 1, can be carried out at temperatures between about -20 °C and about 100 °C. Preferably, these reactions are carried out between about -5 °C and about 50 °C.

The above Process B, Method 2, can be carried out at temperatures between about -50 °C and about 150 °C. Preferably when W is a halo radical, the reaction is carried out between about 0 °C and about 30 °C. When W is alkoxy, the reaction is preferably carried out between about 100 °C and about 150 °C. When W is methyl sulfonate, the reaction is preferably carried out between about -20 °C to about 20 °C. When W is an ester, the reaction is preferably carried out between about 0 °C and about 50 °C.

Process C can be carried out at temperatures between about 10 °C and 200 °C. Preferably, this reaction is carried out between about 70 °C and about 100 °C.

Process D can be carried out at temperatures between about 0 °C and 100 °C. Preferably, these reactions are carried out between about 0 °C and about 50 °C.

Preparation of the compounds of the present invention by Processes A, B, C and D are preferably carried out at about atmospheric pressure, although higher or lower pressures can be used if desired.

Substantially equimolar amounts of reactants are preferably used in Processes A, B and C, although higher or lower amounts can be used if desired.

Generally, about one equivalent of base is used per equivalent of starting material of Formula II, V, XI and/or XIII. Where the acid addition salt of the monosubstituted hydrazine of Formula III is used, one additional equivalent of base is used. For example, in Process A, when substituents A and B are the same and a monosubstituted hydrazine is used, about two equivalents of base are used since about two equivalents of a suitably substituted benzoyl chloride of Formula II or V are employed. In Process A, when substituents A and B are different and an acid addition salt of the monosubstituted hydrazines of Formula III is used, about two equivalents of base are used in Step 1 and about one equivalent of base is used in Step 2.

Modifications to the above processes may be necessary to accommodate reactive functionalities of particular A and/or B substituents. Such modifications would be apparent and known to those skilled in the art.

It will be appreciated by those skilled in the art that electronic forces may give rise to more than one isomer of the compounds of Formula I such as enantiomers, conformers and the like. There may be a difference in properties such as physical characteristics and degree of biological activity between such isomers. Separation of a specific isomer can be accomplished by standard techniques well known to those skilled in the art such as silica gel chromatography.

The agronomically acceptable salts embraced by the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt of a compound of Formula I in a suitable solvent. When metal hydroxides are used as reagents, useful solvents include water; ethers such as glyme and the like; dioxane; tetrahydrofuran; alcohols such as methanol, ethanol, isopropanol and the like. When metal hydrides are used as reagents, useful solvents include nonhydroxylic solvents, for example, ethers such as dioxane, glyme, diethylether and the like; tetrahydrofuran; hydrocarbons such as toluene, xylene, hexane, pentane, heptane, octane and the like; dimethylformamide, and the like. When amines are used as reagents, useful solvents include alcohols, such as methanol or ethanol; hydrocarbons, such as toluene, xylene, hexane and the like; tetrahydrofuran; glyme; dioxane; or water. When ammonium salts are used as reagents, useful solvents include water; alcohols, such as methanol or ethanol; glyme; tetrahydrofuran; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0 °C to about 100 °C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones, haloalkanes and the like. The particular choice of solvent will depend on the relative solubilities of the starting materials and the resulting salts and slurries rather than solutions of certain reagents may be used to obtain the salts. Generally, equivalent molar amounts of starting materials are used and the salt-forming reaction is carried out at from about -10 °C to about 100 °C, preferably at about room temperature.

The following examples will further illustrate this invention but are not intended to limit it in any way. In Table I, some N'-substituted-N,N'-diacyl hydrazines of the present invention that have been made are listed.

The structure of these compounds was confirmed by NMR and in some cases by IR and/or elemental analysis. Specific illustrative preparation of the compounds of Examples 8, 15 and 28 are described after Table I.

TABLE I

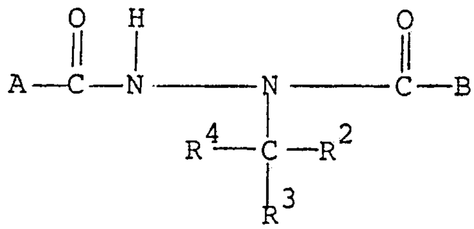

| Ex No. | $R^2$ | $R^3$ | $R^4$ | A | B |
|---|---|---|---|---|---|
| 1 | H | Me | $CF_3$ | $C_6H_3Me_2$-2,3 | $C_6H_3Cl_2$-2,4 |
| 2 | H | Me | $CF_3$ | $C_6H_3Me_2$-2,3 | $C_6H_3Me_2$-3,5 |
| 3 | H | Me | $CF_3$ | $C_6H_3Me_2$-2,3 | $C_6H_3NO_2Me$-2,5 |
| 4 | H | Me | $CF_3$ | $C_6H_5$ | $C_6H_5$ |
| 5 | H | H | $CF_3$ | $C_6H_3Cl_2$-3,4 | $C_6H_3Cl_2$-3,4 |
| 6 | H | H | $CF_3$ | $C_6H_4Cl$-4 | $C_6H_4Cl$-4 |
| 7 | H | H | $CF_3$ | $C_6H_5$ | $C_6H_5$ |
| 8 | Me | Me | CN | $C_6H_5$ | $C_6H_5$ |
| 9 | Me | Me | CN | $C_6H_5$ | $C_6H_4Cl$-4 |
| 10 | Me | Me | CN | $C_6H_4$4-Me | $C_6H_4Me$-3 |
| 11 | Me | Me | CN | $C_6H_5$ | $C_6H_4Me$-3 |
| 12 | Me | Me | CN | $C_6H_4Cl$-4 | $C_6H_4Cl$-4 |
| 13 | Me | Me | CN | $C_6H_4Me$-4 | $C_6H_3Me_2$3,5 |
| 14 | Me | Me | CN | $C_6H_4Me$-4 | $C_6H_4F$-4 |
| 15 | Me | Me | CN | $C_6H_4Me$-4 | $C_6H_4Me$-4 |
| 16 | Me | Et | CN | $C_6H_5$ | $C_6H_5$ |
| 17 | Me | Me | CN | 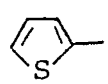 | $C_6H_4Me$-3 |
| 18 | Me | Me | CN | $C_6H_4Et$-4 | $C_6H_3Me_2$-3,5 |
| 19 | Me | Me | CN | $C_6H_4Me$-2 | $C_6H_3Me_2$-3,5 |
| 20 | Me | Me | CN | $C_6H_4Cl$-4 | $C_6H_4Me$-3 |
| 21 | Me | Me | CN | $C_6H_3F_2$-2,6 | $C_6H_4Cl$-4 |
| 22 | Me | Me | CN | $C_6H_4Me$-2 | $C_6H_4Me$-3 |
| 23 | Me | Me | CN | $C_6N_4NO_2$-2 | $C_6H_4Me$-3 |

| | | | | | |
|---|---|---|---|---|---|
| 24 | Me | Me | CN | $C_6H_4Cl$-2 | $C_6H_4Me$-3 |
| 25 | Me | Me | $CO_2Me$ | $C_6H_5$ | $C_6H_5$ |
| 26 | Me | Me | $CH_2CH{=}CH_2$ | $C_6H_4Me$-4 | $C_6H_4Me$-4 |
| 27 | Me | Me | $CH_2CH{=}CH_2$ | $C_6H_4Me$-3 | $C_6H_4Me$-3 |
| 28 | Me | Me | $CH_2CH{=}CH_2$ | $C_6H_5$ | $C_6H_5$ |
| 29 | H | H | $SiMe_3$ | $C_6H_4Et$-4 | $C_6H_3NO_2Me$-2,5 |
| 30 | H | H | $SiMe_3$ | $C_6H_4Cl$-4 | $C_6H_4Cl$-4 |
| 31 | H | H | $SiMe_3$ | $C_6H_4Me$-2 | $C_6H_4Me$-2 |
| 32 | H | H | $SiMe_3$ | $C_6H_4NO_2$-2 | $C_6H_4NO_2$-2 |
| 33 | H | H | $SiMe_3$ | $C_6H_5$ | $C_6H_5$ |
| 34 | H | H | $SiMe_3$ | $C_6H_4Et$-4 | $C_6H_3Me_2$-3,5 |

EXAMPLE No. 8 - Preparation of N'-(1-cyano-1-methyl) ethyl-N,N'-dibenzoylhydrazine

To a suspension of benzoylhydrazine (13.6 g, 0.1 mole) in deionized water (50 ml) at 5°C with stirring was dropwise added concentrated hydrogen chloride (9.8 g, 0.1 mole). To the resulting clear solution was added sodium cyanide (5.2 g, 0.1 mole) and acetone (6.5 g, 0.11 mole). A white and thick precipitate appeared. The cooling bath was removed and the reaction flask was stoppered tightly. The reaction mixture was stirred for 18 hours. The precipitate was collected by suction-filtration and was washed with a small amount of water to give 17.5 g (86.2% yielded) of desired intermediate, N'-(1-cyano-1-methyl)ethyl-N-benzoic acid hydrazide, as the starting material for the next step. (m.p. 82-92°C).

To the solution of N'-(1-cyano-1-methyl)ethyl-N-benzoic acid hydrazide (2 g, 0.01 mole) in dry methylene chloride (25 ml) under nitrogen at room temperature with stirring was added benzoyl chloride (2.02 g, 0.014 mole). To the above mixture was dropwise added triethylamine (1.31 g, 0.013 mole). The reaction mixture was stirred at room temperature for 5 hours. The reaction mixture was diluted with methylene chloride (50 ml), washed with water and brine. The organic layer was dried over $MgSO_4$ and the solvent was evaporated at reduced pressure to give a residue. The residue was treated with ethyl acetate/hexane mixture (1:1) affording a crude solid-formed product which was collected by suction-filtration (1 g, 33% yield). An analytical sample was obtained by crystallization from ethyyl acetate hexane (4:1), m.p. 202-204°C. NMR and IR spectra showed the desired product.

Elemental Analysis

| | C | H | N |
|---|---|---|---|
| Calc. (%) | 70.34 | 5.58 | 13.67 |
| Found (%) | 70.20 | 5.67 | 13.40 |

EXAMPLE No. 15 - Preparation of N'-(1-cyano-1-methyl) ethyl-N,N'-di-4-toluoylhydrazine

To the suspension of 4-toluic acid hydrazide (15.0 g, 0.1 mole) in water (150 ml) and ethanol (20 ml) at 5°C with stirring was dropwise added concentrated hydrogen chloride (10 g, 0.1 mole). To the above still suspension was carefully added sodium cyanide (5.3 g, 0.1 mole) and acetone (6.6 g, 0.11 mole). The reaction flask was stoppered tightly and the cooling bath was removed. The resulting viscous reaction

mixture was stirred at room temperature for more than 24 hours. The precipitate was collected by suction-filtration and was washed with a small amount of diluted hydrogen chloride and water affording 14.6 g. (64.8%) of N'-(1-cyano-1-methyl)ethyl 4-toluic acid hydrazide as the starting material for the next step. Analytical sample was obtained by crystallization from ethyl acetate/hexane (3:1), m.p. 146-148 °C.

To the solution of N'-(1-cyano-1-methyl)ethyl 4-toluic acid hydrazide (2.17 g, 0.01 mole) in dry methylene chloride (65 ml) under nitrogen with magnetic stirring was added 4-dimethylaminopyridine catalyst (1.34 g, 0.011 mole) followed by 4-methylbenzoyl chloride (2.52 g, 0.017 mole). To the above mixture was dropwise added triethylamine (1.1 g, 0.011 mole). The reaction mixture was slightly exothermic. After stirring at room temperature for 40 minutes the reaction mixture was diluted with methylene chloride (50 ml), washed with diluted HCl solution (2×50 ml), dil NaOH (50 ml), $H_2O$ (50 ml) and brine. The organic layer was dried over $MgSO_4$, and the solvent was evaporated under reduced pressure to give a residue. The residue was treated with ethyl acetate/hexane mixture (1:1) and the resulting solid was collected by suction-filtration and affording 2.75 g (82% yield) of almost pure product. (m.p. 192-198 °C) NMR and IR spectra showed the desired product.

By following substantially the procedures in Example 8 (without catalyst) or 15 (with catalyst) and using the reactants shown below in Table II, the products of Examples 8 to 24 were prepared.

TABLE II

| Ex. No. | Compound of Formula VI | Compound of Formula VIIa | Compound of Formula V |
|---|---|---|---|
| 8 | benzoylhydrazine | acetone | benzoyl chloride |
| 9 | benzoylhydrazine | acetone | 4-chlorobenzoyl chloride |
| 10 | 4-toluoylhydrazine | acetone | 3-methylbenzoyl chloride |
| 11 | benzoylhydrazine | acetone | 3-methylbenzoyl chloride |
| 12 | 4-chlorobenzoyl-hydrazine | acetone | 4-chlorobenzoyl chloride |
| 13 | 4-toluoylhydrazine | acetone | 3,5-dimethylbenzoyl chloride |
| 14 | 4-toluoylhydrazine | acetone | 4-fluorobenzoyl chloride |
| 15 | 4-toluoylhydrazine | acetone | 4-methylbenzoyl chloride |
| 16 | benzoylhydrazine | methylethylketone | benzoyl chloride |
| 17 | thienoylhydrazine | acetone | 3-methylbenzoyl chloride |
| 18 | 4-ethylbenzoyl-hydrazine | acetone | 3,5-dimethylbenzoyl chloride |
| 19 | 2-toluoylhydrazine | acetone | 3,5-dimethylbenzoyl chloride |
| 20 | 4-chlorobenzoyl-hydrazine | acetone | 3-methylbenzoyl chloride |

| Ex. No. | Compound of Formula VI | Compound of Formula VIIa | Compound of Formula V |
|---|---|---|---|
| 21 | 2,4-difluorobenzoyl-hydrazine | acetone | 4-chlorobenzoyl chloride |
| 22 | 2-toluoylhydrazine | acetone | 3-methylbenzoyl chloride |
| 23 | 2-nitrobenzoyl-hydrazine | acetone | 3-methylbenzoyl chloride |
| 24 | 2-chlorobenzoyl-hydrazine | acetone | 3-methylbenzoyl chloride |

EXAMPLE NO. 28 - Preparation of N'-(1,1-dimethyl-3-butenyl)-N,N'-dibenzoylhydrazine

To a gently refluxing solution of allyl magnesium bromide (380 ml of 1 M solution) was added acetone azine (20 g) dissolved in diethyl ether (200 ml). The solution was refluxed for three days. Upon cooling, a saturated solution of ammonium chloride (50 ml) was added. The aqueous layer was separated and washed twice with diethyl ether (200 ml). The combined ether extracts were dried over anhydrous magnesium sulfate, filtered and the ether removed at reduced pressure. The product was distilled through a vigreux column at 3.1 torr and collected in a dry ice/acetone cooled receiving flask. The boiling point was 60-65 °C. 15 g of product was collected.

Oxalic acid (16.7 g) was dissolved in a solution of ethanol:diethyl ether (1:1) (150 ml) and water (3.3 g) was added. To this acid solution was added the hydrazone (13 g) dissolved in diethyl ether (75 ml). The solution was stirred for 24 hours then filtered. The solid is washed once with diethyl ether. The filtrate was concentrated and combined with the solid to afford a 71% yield (17.2 g) of the hydrazine oxalate.

The 1,1-dimethyl-3-butenylhydrazine oxalate (2 g) was dissolved in toluene and neutralized with 50% aqueous sodium hydroxide. To this solution was added benzoyl chloride (2.8 g) and sodium hydroxide (50% Aq. solution) (3.2 g) at 25 °C. The reaction mixture was warmed to room temperature and stirred. The mixture was diluted with hexane and filtered to afford an oily product which solidified upon standing. (m.p. 105-112 °C).

Examples 26 and 27 were made generally in accordance with the procedures for Example 29 above.

The (trimethylsilylmethyl)hydrazine, trifluoroalkyl hydrazine and (2-carbmethoxy-2-propyl)hydrazine were made generally in accordance with the procedures from Noll, J.E.; Sprier, J.L.; Daubert, B.F.; JACS 73, 3867, 1951; Hung, S.C.; Le; Breton, G.C.; JOC 46, 5413, 1981; and Organic Synthesis Vol 5, pg. 43, respectively. From these starting materials Examples 1-7, 25 and 29-34 were made generally in accordance with the procedure for Example 28 above.

The reactants shown below in Table III were used to prepare Examples 1-7 and 25-34.

TABLE III

| Ex. No. | Compound of Formula II | Compound of Formula III | Compound of Formula V |
|---|---|---|---|
| 1 | 2,3-dimethyl benzoylchloride | (1-methyl-2,2,2-trifluoroethyl) hydrazine | 2,4-dichloro benzoylchloride |
| 2 | 2,3-dimethyl benzoylchloride | (1-methyl-2,2,2-trifluoroethyl) hydrazine | 3,5-dimethyl benzoylchloride |
| 3 | 2,3-dimethyl benzoylchloride | (1-methyl-2,2,2-trifluoroethyl) hydrazine | 2-nitro-5-toluoylchloride |

| Ex. No. | Compound of Formula II | Compound of Formula III | Compound of Formula V |
|---|---|---|---|
| 4 | benzoylchloride | (1-methyl-2,2,2-trifluoroethyl) hydrazine | benzoylchloride |
| 5 | 3,4-dichloro benzoylchloride | (2,2,2-trifluoroethyl) hydrazine | 3,4-dichloro benzoylchloride |
| 6 | 4-chlorobenzoyl chloride | (2,2,2-trifluoroethyl) hydrazine | 4-chlorobenzoyl chloride |
| 7 | benzoylchloride | (2,2,2-trifluoroethyl) hydrazine | benzoylchloride |
| 25 | benzoylchloride | (1-carbmethoxy-1-methylethyl) hydrazine | benzoylchloride |
| 26 | 4-toluoylchloride | (1,1-dimethyl-3-butenyl) hydrazine | 4-toluoylchloride |
| 27 | 3-toluoylchloride | (1,1-dimethyl-3-butenyl) hydrazine | 3-toluoylchloride |
| 28 | benzoylchloride | (1,1-dimethyl-3-butenyl) hydrazine | benzoylchloride |
| 29 | 4-ethylbenzoyl chloride | (trimethylsilylmethyl) hydrazine | 2-nitro-5-toluoyl chloride |
| 30 | 4-chlorobenzoyl chloride | (trimethylsilylmethyl) hydrazine | 4-chlorobenzoyl chloride |
| 31 | 2-toluoyl chloride | (trimethylsilylmethyl) hydrazine | 2-toluoyl chloride |
| 32 | 2-nitrobenzoyl chloride | (trimethylsilylmethyl) hydrazine | 2-nitrobenzoyl chloride |
| 33 | benzoyl chloride | (trimethylsilylmethyl) hydrazine | benzoyl chloride |
| 34 | 4-ethylbenzoyl chloride | (trimethylsilylmethyl) hydrazine | 3,5-dimethyl benzoylchloride |

It will be appreciated by those skilled in the art that compounds of Formula I can be used as precursors for preparing other compounds of Formula I by procedures well known to those skilled in the art. For example, a suitable compound of Formula I can be reduced, alkylated, substituted, esterified, hydrolyzed or the like.

As previously noted, the compounds of the present invention exhibit excellent insecticidal activity and are most active against insects of the orders Lepidoptera and Coleoptera.

In general, for the control of insects in agriculture, horticulture and forestry, the compounds of the present invention may be used at a dosage corresponding to from about 10 grams to about 10 kilograms of

the active substance per hectare and from about 100 grams to about 5 kilograms per hectare of the active substance is preferred. The exact amount of dosage for a given situation can be routinely determined and depends on a variety of factors, for example, the substance used, the kind of insect, the formulation used, the state of the crop infested with the insect and the prevailing weather conditions. The term "insecticidal" as employed in the specification and claims of this application is to be construed as any means which adversely affects the existence or growth of the target insects at any stage in their life cycle. Such means can comprise a complete killing action, eradication, arresting in growth, inhibition, reducing in number, reproductive inhibition (such as ovicidal or chemisterilant) or any combination thereof. The term "control" or "combat" as employed in the specification and claims of this application is to be construed as meaning "insecticidal" activity or protection of plants from insect damage. By "insecticidally effective amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research", (1969), Advances in Chemistry Series No. 86, written by Wade Van Valkenburg; and the Marcel Dekker, Inc. publication "Pesticide Formulations", (1973), edited by Wade Van Valkenburg. In these compositions and formulations, the active substance or substances are mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, conventional adjuvants such as surfactant, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behavior, feeding habits and physiology of the pest for the purpose of control.

The invert emulsions are mainly used for air application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl isobutyl ketone, cyclohexanone, isophorone, etc.), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as

cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.); and/or dispersing agents, such as lignin, sulfite waste liquors, methyl cellulose, etc.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists, etc., if desired, or in the form of particular dosage preparations for specific application made therefrom, such as solutions, emulsions, suspensions, powders, pastes, and granules which are thus ready for use.

As concerns commercially marketed preparations, these generally contemplate mixtures in which the active compound is present in an amount substantially between about 0.1% and 99% by weight, and preferably between about 1% and 75% by weight, of the mixture. Mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally, between about 0.0001% and about 99% by weight of the composition, preferably between about 0.001% and about 90% by weight of the composition, and more preferably between about 0.01% and about 75% by weight of the composition, which is effective for the purpose in question.

The active compounds can be applied as sprays by methods commonly employed, such as conventional high-gallonage hydraulic sprays, low gallonage sprays, ultra-lcw-volume sprays, airblast spray, aerial sprays, and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of combatting or controlling insects, which comprises contacting insects with an insecticidally effective amount of at least one active compound of the invention alone or together with a diluent or carrier vehicle, (i.e. in a composition or formulation) as noted above. The term "contacting" as employed in the specification and claims of this application is to be construed as applying to at least one of (a) such insects and (b) the corresponding habitat thereof (i.e., the locus to be protected, for example, to a growing crop or to an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the

active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example, as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

Representative preparation of compositions and formulations including the compounds of the present invention are set forth below as Examples A through I by way of illustration but not limitation.

## Example A

### Granular

| Ingredient | %/wt. |
|---|---|
| Toxicant and toxicant impurities | 0.25 |
| Triton X-305 (binder) (Octylphenyl-30-ethylene oxide ethanol) | 0.25 |
| Agsorb 24/48 (diluent) (Montmorillonite clay) | 99.50 |

Preparation: The toxicant and Triton X-305 are dissolved into methylene chloride and the mixture is added to the Agsorb with continuous mixing. The methylene chloride is then allowed to evaporate.

## Example B

### Dust

| Ingredient | %/wt. |
|---|---|
| Toxicant and toxicant impurities | 1.0 |
| Talc | 99.0 |

Preparation: Toxicant is dissolved in excess acetone and the mixture is impregnated onto the talc. The acetone is then permitted to evaporate.

## Example C

### Wettable Powder

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HiSil 233 (diluent) (Sodium silica) | 30.0 |

Preparation: The toxicant, optionally dissolved in a volatile solvent, is absorbed onto the Barden clay and HiSil carriers. The Duponal and Reax are then added and the entire dry mixture blended until homogeneous. The composition is then micronized to a fine particle size.

Example D

Emulsifiable Concentrate

| Ingredient | %/wt. |
|---|---|
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco 500-100 (solvent) (Aromatic solvent mixture principally comprising xylene, cumene and ethyle benzene having a boiling point range of 143-174°C (290-345°F) | 52.5 |

Preparation: All ingredients are mixed together with continuous agitation until a homogeneous clear solution is obtained.

Example E

Aerosol

| Ingredient | %/wt. |
|---|---|
| Toxicant and toxicant impurities | 0.5 |
| Freon 12 | 99.5 |

Preparation: The components are mixed and packaged under pressure in a suitable container equipped with a release spray valve.

Example F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 (preservative) (2-*n*-octyl-4-isothiazolin-3-one) | 0.05 |

Preparation: The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example H

Pellet

Same as Example G, Method A, with this addition: the bait composition is formed into 0.64 cm (1/4") diameter by 0.95 cm (3/8") long pellets using a suitable die and press apparatus.

Example I

Flowable

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| HiSil 233 (diluent) (Sodium silica) | 30.0 |
| Kelzan (thickener) (Xanthan gum) | 0.5 |
| Water | 31.2 |

Preparation: The toxicant is absorbed onto the HiSil carrier. The Duponal and Reax are then added and the entire dry mixture blended until homogeneous. The composition is then micronized to a fine particle size. The resulting powder is suspended in water and the Kelzan added.

Compositions and formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis( p -chlorophenyl)-1,1,1-trichloroethane and hexachloroepoxyoctahydrodimethanonaphthalene;

Carbamates, for example, N-methyl-1-naphthylcarbamates;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, O,O-diethyl-O-p -nitrophenylphosphorothioate; N-monomethylamide of O,O-dimethyldithiophosphorylacetic acid;

Diphenylsulfides, for example, p -chlorobenzyl or p -chlorophenyl sulfide and 2,4,4',5-tetrachlorodiphenylsulfide;

Diphenylsulfonates, for example, p -chlorophenylbenzenesulfonate;

Methylcarbinols, for example, 4,4-dichloro-1-trichloromethylbenzhydrol;

Quinoxaline compounds, such as methylquinoxaline dithiocarbonate;

Amidines such as N'-(4-chloro-2-methylphenyl) N,N-dimethylformamidine;

Pyrethroids such as Allethrin;

Biologicals such as Bacillus thuringiensis preparations;

Organic tin compounds such as tricyclohexyltin hydroxide;

Synergists such as piperonyl butoxide;

Insect growth regulators such as N-benzoyl-phenyl ureas, for example, diflubenzuron.

Fungicides such as:

Organic mercury compounds, for example, phenylmercuryacetate and methylmercurycyanoguanide;

Organic tin compounds, for example, triphenyltin hydroxide and triphenyltin acetate;

Alkylenebisdithiocarbamates, for example, zinc ethylenebisthiocarbamate and managanese ethylenebisdithiocarbamate; and

2,4-dinitro-6-(2-octyl-phenylcrotonate), 1-bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

Biological Activity

It has been found by biological evaluation that compounds according to the present invention have pesticidal activity and are capable of controlling larvae and adult forms of pests, especially insects from the orders Lepidoptera and Coleoptera and most especially insects from the order Lepidoptera. One skilled in the art will know how to determine the activity of a given compound against a given insect and the dosage required to obtain general or selective insecticidal effects. The compounds of the present invention in part affect the normal development of insects, particularly insects from the order Lepidoptera, by directly and/or indirectly influencing the moulting process.

As previously noted, the compounds of the present invention are particularly suitable for controlling plant destructive insects in crops of cultivated plants, such as, but not limited to, cotton, vegetables, corn and other cereals and the like; forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to fruit and/or citrus trees, raspberry bushes and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton X-155) and a modified phthalic glycerol alkyl resin (sold under the trademark Triton B-1956) was utilized at the equivalent of 1 ounce per 100 gal. of test solution as a surfactant.

Initial evaluations were made on one or more of the following pests:

| Symbol | Common Name | Latin Name |
|---|---|---|
| SAW | Southern Armyworm | Spodoptera eridania |
| MBB | Mexican Bean Beetle | Epilachna varivestis |

For the foliar bean beetle and armyworm tests, individual bean ( Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

The percent mortality for the bean beetle and armyworm evaluations are determined 96 hours after treatment. Evaluations are based on a scale of 0-100 percent in which 0 equals no activity and 100 equals total kill.

The rotating turntable consists of a fixed, continuously operating spray nozzle under which targets are rotated at a fixed speed and distance. If the target is a Petri dish (such as for the armyworm), the distance from the nozzle is 38.1 cm (15 inches). If the target is a Mason jar, the distance between the screened lid and the nozzle is 15.24 cm (6 inches) (25.4 cm (10 inches) from the base of the jar to the nozzle). The nozzle is located 8 inches from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the guage pressure of 68.5 $\times$ $10^3$ $N/m^2$ (10 psig) air pressure used and with liquid siphon feed 1.9 l/hr (0.5 gallons per hour) are delivered in a round spray pattern with a 21° spray angle. Targets are misted with spray droplets to the point that the droplets coalesce to form a uniform thin film insufficient to drown test organisms.

All treatments are maintained at 23.9-26.7° C (75-80° F) under continuous fluorescent light in a well-ventilated room.

The results of the initial insecticidal evaluations are given in Table IV.

TABLE IV

Initial Biological Evaluations

| Example No. | Foliar Application Test Species | |
|---|---|---|
| | SAW | MBB |
| 1 | 100 | - |
| 2 | 100 | - |
| 3 | 100 | - |
| 4 | 100 | - |
| 5 | 10 | - |
| 6 | 30 | - |
| 7 | 0 | - |
| 8 | 100 | 40 |
| 9 | 100 | 60 |
| 10 | 100 | 30 |
| 11 | 100 | 30 |
| 12 | 100 | 20 |
| 13 | 100 | 0 |
| 14 | 100 | 20 |
| 15 | 100 | 0 |
| 16 | 100 | 50 |
| 17 | 50 | 0 |
| 18 | 100 | 0 |
| 19 | 100 | 0 |
| 20 | 100 | 20 |
| 21 | 100 | 0 |

TABLE IV (contd.)

Initial Biological Evaluations

| Example No. | Foliar Application Test Species SAW | MBB |
|---|---|---|
| 22 | 100 | 0 |
| 23 | 100 | 30 |
| 24 | 100 | 40 |
| 25 | 30 | - |
| 26 | 40 | - |
| 27 | 100 | - |
| 28 | 100 | - |
| 29 | 100 | - |
| 30 | 0 | - |
| 31 | 100 | - |
| 32 | 100 | - |
| 33 | 20 | - |
| 34 | 0 | - |

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

The following words are trademarks which may be registered in some or all of the designated states: Triton, Agsorb, Duponal, Reax, Hisil, Sponto, Tenneco, Kathon and Kelzan.

## Claims

**1.** An insecticidally active compound which is a substituted hydrazine having the formula:

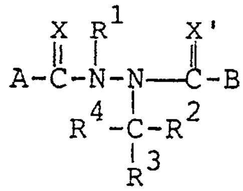

I

wherein

X and X′ are the same or different O, S or NR;

R' is hydrogen; $(C_1\text{-}C_6)$alkyl; $(C_1\text{-}C_6)$alkoxy-$(C_1\text{-}C_6)$alkyl having independently the stated number of carbon atoms in each alkyl group; $(C_1\text{-}C_6)$alkylthio-$(C_1\text{-}C_6)$alkyl having independently the stated number of carbon atoms in each alkyl group; $(C_2\text{-}C_6)$alkenyl; $(C_2\text{-}C_6)$alkynyl; or phen-$(C_1\text{-}C_4)$alkyl where the phenyl ring is unsubstituted or substituted with one to three of the same or different halo, cyano, nitro, hydroxy, $(C_1\text{-}C_4)$alkyl, halo-$(C_1\text{-}C_4)$alkyl $(C_1\text{-}C_4)$alkoxy, halo-$(C_1\text{-}C_4)$alkoxy, carboxy, $(C_1\text{-}C_4)$alkoxy-carbonyl, $(C_1\text{-}C_4)$alkanoyloxy or NZZ′;

$R^2$ and $R^3$ are the same or different hydrogen or $(C_1\text{-}C_4)$alkyl;

$R^4$ is $(C_1\text{-}C_4)$alkyl substituted with 1 to 4 fluoro; straight chain $(C_2\text{-}C_4)$alkenyl; carboxyl; $(C_1\text{-}C_3)$-alkoxy-carbonyl; cyano; cyano substituted $(C_1\text{-}C_4)$alkyl, tri$(C_1\text{-}C_4)$alkylsilyl having independently the stated number of carbon atoms in each alkyl group; or tri$(C_1\text{-}C_2)$alkylsilylmethyl having independently the stated number of carbon atoms in each alkyl group; provided that $R^2$ and $R^3$ are both alkyl when $R^4$ is carboxyl or alkoxycarbonyl; and

A and B are the same or different unsubstituted or substituted naphthyl where the substituents can be from one to three of the same or different halo; nitro; $(C_1\text{-}C_4)$alkoxy; $(C_1\text{-}C_4)$alkyl or NZZ′;

unsubstituted or substituted phenyl where the substituents can be from one to five of the same or different halo; nitroso; nitro; cyano; hydroxy; $(C_1\text{-}C_6)$alkyl; halo-$(C_1\text{-}C_6)$alkyl; cyano-$(C_1\text{-}C_6)$alkyl; hydroxy-$(C_1\text{-}C_6)$alkyl; $(C_1\text{-}C_6)$alkoxy; halo-$(C_1\text{-}C_6)$alkoxy; $(C_1\text{-}C_6)$alkoxy-$(C_1\text{-}C_6)$alkyl having independently the stated number of carbon atoms in each alkyl group; $(C_1\text{-}C_6)$alkoxy-$(C_1\text{-}C_6)$alkoxy having independently the stated number of carbon atoms in each alkyl group; -ORSR′ group; $-OCO_2R$ group; $-OCO_2H$ group; $(C_1\text{-}C_6)$alkanoyloxy-$(C_1\text{-}C_6)$alkyl having independently the stated number of carbon atoms in each alkyl group; $(C_2\text{-}C_6)$alkenyl optionally substituted with halo, cyano, $(C_1\text{-}C_4)$-alkyl, halo-$(C_1\text{-}C_4)$alkoxy, $(C_1\text{-}C_4)$alkylthio or $(C_1\text{-}C_4)$alkoxy; $(C_1\text{-}C_4)$alkadienyl; $(C_2\text{-}C_6)$alkenyloxy; $(C_2\text{-}C_6)$alkenyl-carbonyl; $(C_2\text{-}C_6)$alkenyloxy-carbonyloxy; $(C_2\text{-}C_6)$alkynyl optionally substituted with halo, cyano, nitro, hydroxy, $(C_1\text{-}C_4)$alkoxy, halo-$(C_1\text{-}C_4)$alkyl, $(C_1\text{-}C_4)$alkylthio or $(C_1\text{-}C_4)$alkyl; carboxy; $-RCO_2R'$ group; -COR; -COH; halo-$(C_1\text{-}C_6)$alkyl-carbonyl; $-CO_2R$ group; $(C_1\text{-}C_6)$haloalkoxy-carbonyl; -OCOR group; $-ORCO_2R'$ group; amino (-NZZ′); amino substituted with hydroxy, $(C_1\text{-}C_4)$-alkoxy or $(C_1\text{-}C_4)$alkylthio; -CONZZ′ group; $(C_2\text{-}C_6)$alkenyl-carbonylamino; hydroxy-$(C_1\text{-}C_6)$-alkylamino-carbonyl; -OCONZZ′ group; -NZCOZ′ group; $-NZCO_2Z'$ group; thiocyanato; isothiocyanato; $(C_1\text{-}C_6)$thiocyanatoalkyl; $(C_1\text{-}C_6)$alkylthio; halo-$(C_1\text{-}C_6)$alkylthio; -S(O)Z group; $-SO_2Z$ group; $-OSO_2R$ group; $-OSO_2H$ group; $-SO_2NZZ'$ group; -CSR group; -CSH group; -SCOR group; -SCOH group; -NSCSZ′ group; unsubstituted or substituted phenyl having one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, amino, $(C_1\text{-}C_4)$alkylamino or di$(C_1\text{-}C_4)$alkylamino having independently the stated number of carbon atoms in each alkyl group; phenoxy where the phenyl ring is unsubstituted or substituted with one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkyl-carbonyl, $(C_1\text{-}C_4)$-alkanoyloxy, amino, $(C_1\text{-}C_4)$alkylamino or di$(C_1\text{-}C_4)$alkylamino having independently the stated number of carbon atoms in each alkyl group; benzoyl where the phenyl ring is unsubstituted or substituted with one tő three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, amino, $(C_1\text{-}C_4)$-alkylamino or di$(C_1\text{-}C_4)$alkylamino having independently the stated number of carbon atoms in each alkyl group; benzoyloxy-$(C_1\text{-}C_6)$alkyl; phenylthio-$(C_1\text{-}C_6)$alkyl where the phenyl ring is unsubstituted or substituted with one to three of the same or different halo, cyano, nitro, hydroxy, $(C_1\text{-}C_4)$alkyl, halo-$(C_1\text{-}C_4)$alkyl, $(C_1\text{-}C_4)$alkoxy, carboxy, $(C_1\text{-}C_4)$alkoxy-carbonyl, $(C_1\text{-}C_4)$-alkanoyloxy, amino, $(C_1\text{-}C_4)$alkylamino or di$(C_1\text{-}C_4)$alkylamino having independently the stated number of carbon atoms in each alkyl group; -CR=N-R> group where R> is hydroxy, $(C_1\text{-}C_4)$alkyl, $(C_1\text{-}C_4)$alkoxy, amino (-

NZZ'), phenylamino, -COR, -COH or benzoyl; ($C_2$-$C_6$)oxiranyl; acetylthiosemi- carbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;

unsubstituted or substituted ($C_1$-$C_{10}$)alkyl having one to four of the same or different halo, cyano, nitro, hydroxy, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy, phenyl or -NZZ';

unsubstituted or substituted ($C_3$-$C_8$)cycloalkyl or unsubstituted or substituted ($C_3$-$C_8$)cyclo($C_1$-$C_4$)-alkyl having one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkanoyl, ($C_1$-$C_4$)alkoxy-carbonyl, ($C_1$-$C_4$)alkanoyloxy or -NZZ';

unsubstituted or substituted ($C_2$-$C_8$)alkenyl or unsubstituted or substituted ($C_2$-$C_8$)alkadienyl having as substituent(s) a furyl, thienyl or pyridyl or one to four of the same or different halo, cyano, nitro, hydroxy, ($C_1$-$C_4$)alkyl, ($C_3$-$C_6$)cycloalkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy or -NZZ';

unsubstituted or substituted ($C_3$-$C_8$)cycloalkenyl or unsubstituted or substituted ($C_3$-$C_8$)-cycloalkadienyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy or -NZZ';

unsubstituted or substituted ($C_2$-$C_8$)alkynyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)-alkoxy, carboxy, ($C_1$-$C_4$)alkoxy-carbonyl, ($C_1$-$C_4$)alkanoyloxy, phenyl or -NZZ';

phenalkyl having one to four carbon atoms in the alkyl group and wherein the alkyl group is unsubstituted or substituted with one to three of the same or different halo, cyano, hydroxy, ($C_1$-$C_4$)-alkoxy, ($C_1$-$C_4$)alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, cyano-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy, ($C_2$-$C_6$)alkenyl, halo-($C_2$-$C_6$)alkenyl, ($C_2$-$C_6$)alkynyl or -NZZ';

phenalkenyl having two to six carbon atoms in the alkenyl group and the alkenyl group is unsubstituted or substituted with one to three of the same or different halo, cyano, hydroxy, ($C_1$-$C_4$)-alkyl, halo-($C_1$-$C_4$)alkyl, ($C_1$-$C_4$)alkoxy, halo-($C_1$-$C_4$)alkoxy, ($C_1$-$C_4$)alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy or -NZZ';

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; -$RCO_2H$ group; $RCO_2R'$ group; -CONZZ' group; amino (-NZZ'); -NZCOZ' group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl or amino (-NZZ'); or
unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; -$RCO_2H$ group; -$RCO_2R'$ group; -CONZZ' group; amino (-NZZ'); -NZCOZ' group; ($C_1$-$C_6$)alkylthio; or unsubstituted or substituted phenyl having one to three of the same or different halo, nitro, ($C_1$-$C_6$)-alkyl, halo- ($C_1$-$C_6$)alkyl, ($C_1$-$C_6$)alkoxy, halo-($C_1$-$C_6$)- alkoxy, carboxy, ($C_1$-$C_4$)alkoxy-carbonyl or amino (-NZZ');
where R and R' are ($C_1$-$C_6$)alkyl; Z and Z' are hydrogen or ($C_1$-$C_4$)alkyl; "amino" means NZZ'; or an

agronomically acceptable salt thereof.

**2.** A compound according to claim 1 wherein
X and X′ are O or S;
$R^1$ is hydrogen; ($C_1$-$C_4$)alkyl; ($C_1$-$C_4$)alkoxy-($C_1$-$C_4$)alkyl having independently the stated number of carbon atoms in each alkyl group; methylthiomethyl; ($C_2$-$C_5$)alkenyl; ($C_2$-$C_5$)- alkynyl; or phen-($C_1$-$C_2$)alkyl where the phenyl ring is unsubstituted or substituted with one or two halo, nitro, ($C_1$-$C_4$)alkyl or ($C_1$-$C_4$)alkoxy;
$R^2$ and $R^3$ are the same or different hydrogen or ($C_1$-$C_3$)alkyl;
$R^4$ is ($C_1$-$C_3$)alkyl substituted with one to four fluoro; straight chain ($C_2$-$C_4$)alkenyl; carboxyl; ($C_1$-$C_2$)-alkoxy-carbonyl; cyano; cyano substituted ($C_1$-$C_3$)alkyl; tri($C_1$-$C_2$)alkylsilyl having independently the stated number of carbon atoms in each alkyl group; or tri($C_1$-$C_2$)alkylsilylmethyl having independently the stated number of carbon atoms in each alkyl group; and
A and B are the same or different unsubstituted naphthyl;

unsubstituted or substituted phenyl where the substituents can be from one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)alkoxy-($C_1$-$C_4$)alkyl having independently the stated number of carbon atoms in each alkyl group; -COZ; carboxy; ($C_1$-$C_4$)alkoxy-carbonyl; ($C_1$-$C_4$)alkanoyloxy; ($C_2$-$C_6$)alkenyl; ($C_2$-$C_6$)alkynyl; amino (-NZZ′); thiocyanato; ($C_1$-$C_4$)alkylthio; -CSR; -CSH; unsubstituted or substituted phenyl having one to two of the same or different halo, nitro, ($C_1$-$C_4$)alkyl, ($C_1$-$C_4$)alkoxy, carboxy, -NZZ′; phenoxy where the phenyl ring is unsubstituted or substituted with one or two of the same or different halo, nitro, ($C_1$-$C_4$)alkyl, ($C_1$-$C_4$)alkoxy, carboxy, -NZZ′; or when two adjacent positions on the phenyl ring are substituted with alkoxy groups, these groups may be joined to form, together with the carbon atoms to which they are attached, a 5- or 6-membered dioxolano or dioxano heterocyclic ring;

unsubstituted or substituted ($C_1$-$C_8$)alkyl having one to three of the same or different halo, cyano, ($C_1$-$C_4$)alkoxy, phenyl, ($C_1$-$C_4$)alkoxy-carbonyl or halo-($C_1$-$C_4$)alkoxy;

unsubstituted or substituted ($C_3$-$C_6$)cycloalkyl or unsubstituted or substituted ($C_3$-$C_6$)cycloalkyl($C_1$-$C_4$)alkyl having one or two of the same or different halo, ($C_1$-$C_4$)alkyl, halo-($C_1$-$C_4$)alkyl, ($C_1$-$C_4$)-alkoxy, ($C_1$-$C_4$)alkanoyl or halo-($C_1$-$C_4$)alkoxy;

unsubstituted or substituted ($C_2$-$C_6$)alkenyl having as substituent(s) a furyl or one to three of the same or different ($C_1$-$C_4$)alkyl, halo-($C_1$-$C_4$)alkyl, ($C_1$-$C_4$)alkoxy or halo-($C_1$-$C_4$)alkoxy;

unsubstituted or substituted ($C_3$-$C_6$)cycloalkenyl having as substituents one or two of the same or different halo, ($C_1$-$C_4$)alkyl, halo-($C_1$-$C_4$)alkyl, ($C_1$-$C_4$)alkoxy or halo-($C_1$-$C_4$)alkoxy;

unsubstituted or phenyl substituted alkynyl;

phenalkyl having one or two carbon atoms in the alkyl group and the alkyl group is unsubstituted or substituted with one or two of the same or different halo, ($C_1$-$C_4$)alkyl, halo-($C_1$-$C_4$)alkyl, ($C_1$-$C_4$)-alkoxy or halo-($C_1$-$C_4$)alkoxy and the phenyl ring is unsubstituted or substituted with one or two of the same or different halo, cyano, ($C_1$-$C_4$)alkyl, halo-($C_1$-$C_4$)alkyl, ($C_1$-$C_4$)alkoxy or halo-($C_1$-$C_4$)-alkoxy;

phenalkenyl having two or three carbon atoms in the alkenyl group and the alkenyl group is unsubstituted or substituted with halo, ($C_1$-$C_4$)alkyl, halo-($C_1$-$C_4$)alkyl, ($C_1$-$C_4$)alkoxy or halo-($C_1$-$C_4$)-alkoxy and the phenyl ring is unsubstituted or substituted with one or two of the same or different halo, cyano, ($C_1$-$C_4$)alkyl, halo-($C_1$-$C_4$)alkyl, ($C_1$-$C_4$)alkoxy or halo-($C_1$-$C_4$)alkoxy;

unsubstituted or substituted five-membered heterocycle selected from furyl, thienyl, triazolyl, pyrrolyl, and oxazolyl where the substituents can be one or two of the same or different halo; nitro; ($C_1$-$C_4$)alkyl; ($C_1$-$C_4$)alkoxy; -NZZ′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NZZ′; or

unsubstituted or substituted six-membered heterocycle having one or two nitrogen atoms and 4 to 5 nuclear carbon atoms where the substituents can be one or two of the same or different halo; nitro; $(C_1\text{-}C_4)$alkyl; $(C_1\text{-}C_4)$alkoxy; thio-$(C_1\text{-}C_4)$alkoxy; -NZZ′; or unsubstituted or substituted phenyl having one or two of the same or different halo, nitro, $(C_1\text{-}C_4)$alkyl, halo-$(C_1\text{-}C_4)$alkyl, $(C_1\text{-}C_4)$alkoxy, halo-$(C_1\text{-}C_4)$alkoxy, carboxy or -NZZ′;

where R and R′ are $(C_1\text{-}C_4)$alkyl; Z and Z′ are hydrogen or $(C_1\text{-}C_4)$alkyl.

**3.** A compound according to claim 2 wherein

X and X′ are O or S;

$R^1$ is hydrogen; $(C_1\text{-}C_4)$alkyl; $(C_1\text{-}C_4)$alkoxy-$(C_1\text{-}C_4)$alkyl having independently the stated number of carbon atoms in each alkyl group; $(C_2\text{-}C_5)$alkenyl; $(C_2\text{-}C_5)$alkynyl; or benzyl where the phenyl ring is unsubstituted or substituted with one halo, nitro, $(C_1\text{-}C_4)$alkyl or $(C_1\text{-}C_4)$alkoxy;

$R^2$ and $R^3$ are the same or different hydrogen or $(C_1\text{-}C_2)$alkyl;

$R^4$ is $(C_1\text{-}C_3)$alkyl substituted with 1 to 4 fluoro; straight chain $(C_2\text{-}C_4)$alkenyl; methoxycarbonyl; cyano; cyano substituted $(C_1\text{-}C_2)$alkyl; tri$(C_1\text{-}C_2)$alkylsilyl having independently the stated number of carbon atoms in each alkyl group; or tri$(C_1\text{-}C_2)$alkylsilylmethyl having independently the stated number of carbon atoms in each alkyl group; provided that $R^2$ and $R^3$ are both alkyl when $R^4$ is methoxycarbonyl; and

A and B are the same or different unsubstituted naphthyl;

unsubstituted or substituted phenyl having one to three of the same or different halo; nitro; cyano; $(C_1\text{-}C_4)$alkyl; halo-$(C_1\text{-}C_4)$alkyl; cyano-$(C_1\text{-}C_4)$alkyl; $(C_1\text{-}C_4)$alkoxy; $(C_1\text{-}C_4)$alkoxy-$(C_1\text{-}C_4)$alkyl having independently the stated number of carbon atoms in each alkyl group; -COZ; $(C_1\text{-}C_4)$alkoxy-carbonyl; $(C_1\text{-}C_4)$alkanoyloxy; thiocyanato; -NZZ′; $(C_1\text{-}C_4)$alkylthio; -CSZ; $-CS_2Z$; unsubstituted or substituted phenyl having one or two of the same or different halo, nitro, $(C_1\text{-}C_4)$alkyl, $(C_1\text{-}C_4)$alkoxy, carboxy, -NZZ′; or phenoxy where the phenyl ring is unsubstituted or substituted with one or two of the same or different halo, nitro, $(C_1\text{-}C_4)$alkyl, $(C_1\text{-}C_4)$alkoxy, carboxy, -NZZ′ or when two adjacent positions on the phenyl ring are substituted with alkoxy groups, these groups may be joined to form, together with the carbon atoms to which they are attached, a 5- or 6-membered dioxolano or dioxano heterocyclic ring;

unsubstituted or substituted $(C_1\text{-}C_6)$alkyl having one to three of the same or different halo, cyano, nitro, carboxy, $(C_1\text{-}C_4)$alkoxy, phenyl, $(C_1\text{-}C_4)$alkoxy-carbonyl or halo-$(C_1\text{-}C_4)$alkoxy;

unsubstituted or substituted $(C_3\text{-}C_6)$cycloalkyl or unsubstituted or substituted $(C_3\text{-}C_6)$cycloalkyl$(C_1\text{-}C_4)$alkyl, where the substituent is halo, $(C_1\text{-}C_4)$alkyl, $(C_1\text{-}C_4)$alkanoyl, $(C_1\text{-}C_4)$alkoxy or halo-$(C_1\text{-}C_4)$-alkoxy;

unsubstituted or substituted $(C_2\text{-}C_6)$alkenyl having one or two of the same or different halo, $(C_1\text{-}C_4)$-alkyl, $(C_1\text{-}C_4)$alkoxy or halo-$(C_1\text{-}C_4)$alkoxy;

unsubstituted or substituted $(C_4\text{-}C_6)$cycloalkenyl where the substituent is halo $(C_1\text{-}C_4)$alkyl, $(C_1\text{-}C_4)$-alkoxy or halo-$(C_1\text{-}C_4)$alkoxy;

unsubstituted or phenyl substituted alkynyl;

benzyl where the phenyl ring is unsubstituted or substituted with one or two of the same or different halo, methyl or ethyl;

unsubstituted or substituted five-membered heterocycle selected from furyl, thienyl, pyrrolyl and oxazolyl where the substituents can be one or two of the same or different halo, nitro, $(C_1\text{-}C_4)$alkyl or $(C_1\text{-}C_4)$alkoxy; or

unsubstituted or substituted six-membered heterocycle having one or two nitrogen atoms and four to five nuclear carbon atoms where the substituents can be one or two of the same or different halo, nitro, $(C_1\text{-}C_4)$alkyl, $(C_1\text{-}C_4)$alkoxy or thio-$(C_1\text{-}C_4)$alkoxy;

where Z and Z' are hydrogen or ($C_1$-$C_4$)alkyl.

4. A compound according to claim 3 wherein
X and X' are O;
$R^1$ is hydrogen; methyl; ethyl; ($C_1$-$C_2$)alkoxy-($C_1$-$C_2$)alkyl having independently the stated number of carbon atoms in each alkyl group ($C_2$-$C_5$)alkenyl; ($C_2$-$C_5$)alkynyl; or benzyl where the phenyl ring is unsubstituted or substituted with halo;
$R^2$ and $R^3$ are the same or different hydrogen or ($C_1$-$C_2$)alkyl;
$R^4$ is ($C_1$-$C_2$)alkyl substituted with 1 to 4 fluoro; straight chain ($C_2$-$C_4$)alkenyl; cyano; cyano substituted ($C_1$-$C_2$)alkyl; tri($C_1$-$C_2$)alkylsilyl having independently the stated number of carbon atoms in each alkyl group; or tri($C_1$-$C_2$)alkylsilylmethyl having independently the stated number of carbon atoms in each alkyl group; and
A and B are the same or different phenyl or substituted phenyl where the substituents can be from one to three of the same or different halo, nitro, ($C_1$-$C_4$)alkyl, ($C_1$-$C_4$)alkoxy or halo-($C_1$-$C_4$)alkyl;

unsubstituted or substituted ($C_1$-$C_6$)alkyl having as substituent(s) one to two of the same or different halo, phenyl or cyano;

unsubstituted ($C_4$-$C_6$)cycloalkyl;

unsubstituted or substituted ($C_2$-$C_6$)alkenyl having as substituent(s) one to three of the same or different halo or ($C_1$-$C_4$)alkyl;

unsubstituted ($C_4$-$C_6$)cycloalkenyl;

phenalkyl having one or two carbon atoms in the alkyl group and the phenyl ring is unsubstituted or substituted with one or two of the same or different halo, ($C_1$-$C_4$)alkyl or ($C_1$-$C_4$)alkoxy;

unsubstituted or substituted pyridyl or 2,5-pyrazinyl where the substituent can be halo, nitro, ($C_1$-$C_4$)-alkyl, ($C_1$-$C_4$)alkoxy or ($C_1$-$C_4$)thioalkoxy; or

unsubstituted furyl or thienyl or an unsubstituted or substituted pyrrolyl where the substituent can be ($C_1$-$C_4$)alkyl.

5. A compound according to claim 4 wherein
X and X' are O;
$R^1$ is hydrogen; methyl; methoxymethyl; ($C_2$-$C_4$)alkenyl; ($C_2$-$C_5$)alkynyl; benzyl; or 4-halobenzyl;
$R^2$ and $R^3$ are the same or different hydrogen, methyl or ethyl; $R^4$ is trifluoromethyl; 2,2,2-trifluoroethyl; straight chain ($C_2$-$C_4$)alkenyl; cyano; cyanomethyl; tri($C_1$-$C_2$)alkylsilyl having independently the stated number of carbon atoms in each alkyl group; or trimethylsilylmethyl; and
A and B are the same or different phenyl or substituted phenyl where the substituents can be one, two or three of the same or different chloro, fluoro, bromo, iodo, nitro, methyl, ethyl, methoxy or trifluoromethyl;

($C_2$-$C_3$) substituted alkenyl having one to three of the same or different chloro, bromo, methyl or ethyl;

cyclohexenyl;

benzyl;

unsubstituted or substituted pyridyl or 2,5-pyrazinyl where the substituent can be halo, methyl, ethyl or methoxy; or

unsubstituted furyl or thienyl or an unsubstituted or substituted pyrrolyl where the substituent can be ethyl or methyl.

6. A compound according to claim 2 wherein
X and X' are O;
$R^1$ is hydrogen or methyl;
$R^2$ and $R^3$ are hydrogen or $(C_1-C_3)$alkyl;
$R^4$ is trifluoromethyl, 2,2,2-trifluoroethyl, cyano, cyanomethyl, straight chain $(C_2-C_4)$alkenyl, carboxyl, methoxycarbonyl, tri$(C_1-C_2)$alkylsilyl having independently the stated number of carbon atoms in each alkyl group or tri$(C_1-C_2)$alkylsilylmethyl having independently the stated number of carbon atoms in each alkyl group; and
A and B are the same or different thienyl or unsubstituted phenyl or substituted phenyl where the substituents can be one to three of the same or different chloro, fluoro, nitro, methyl, ethyl or propyl.

7. A compound according to claim 6 wherein
X and X' are O;
$R^1$ is hydrogen;
$R^2$ and $R^3$ are hydrogen, methyl or ethyl;
$R^4$ is trifluoromethyl, cyano, allyl, methoxycarbonyl, or trimethylsilyl; and
A and B are the same or different unsubstituted or substituted phenyl having one or two of the same or different chloro, fluoro, nitro, methyl and ethyl; or unsubstituted thienyl.

8. A compound according to claim 7 where
X and X' are O;
$R^1$ is hydrogen; and
$R^2$ is hydrogen, $R^3$ is methyl, $R^4$ is trifluoromethyl, and A and B are the same or different unsubstituted or substituted phenyl where the substituents can be one or two of the same or different chloro, methyl or nitro; or
$R^2$ and $R^3$ are the same or different methyl or ethyl, $R^4$ is cyano, and A and B are the same or different unsubstituted or substituted phenyl where the substituents can be one or two of the same or different chloro, fluoro, methyl, ethyl or nitro; or
$R^2$ and $R^3$ are methyl, $R^4$ is allyl, and A and B are the same or different unsubstituted or methyl substituted phenyl; or
$R^2$ and $R^3$ are hydrogen, $R^4$ is trimethylsilyl, and A and B are the same or different substituted phenyl where the substituents can be one or two of the same or different methyl, ethyl or nitro.

9. A compound according to claim 8 wherein
X and X' are O;
$R^1$ is hydrogen; and
$R^2$ is hydrogen, $R^3$ is methyl, $R^4$ is trifluoromethyl, and A and B are unsubstituted phenyl, or A is 2,3-dimethylphenyl and B is 2,4-dichlorophenyl, 3,5-dimethylphenyl or 2-nitro-5-methylphenyl; or
$R^2$ and $R^3$ are methyl, $R^4$ is allyl, and A and B are both unsubstituted phenyl or 3-methylphenyl; or
$R^2$ and $R^3$ are hydrogen, $R^4$ is trimethylsilyl, and A is 4-ethylphenyl and B is 2-nitro-5-methylphenyl, or A and B are both 2-methylphenyl or 2-nitrophenyl.

10. A compound according to claim 8 wherein
X and X' are O;
$R^1$ is hydrogen;
$R^2$ and $R^3$ are methyl;
$R^4$ is cyano; and
A and B are the same or different unsubstituted or substituted phenyl where the substituents can be one or two of the same or different chloro, fluoro, methyl, ethyl or nitro.

11. An insecticidal composition comprising an agronomically acceptable diluent or carrier and an insecticidal compound according to any one of the preceding claims.

12. A composition according to claim 11 containing said insecticidal compound in an amount of from 0.0001 to 99% by weight of the composition.

13. A composition according to claim 12 wherein the amount of said insecticidal compound is from 0.01 to 99% by weight of the composition.

**14.** A method of controlling insects which comprises contacting said insects with an insecticidally effective amount of a compound according to any one of claims 1 to 10 optionally in a composition according to any one of claims 11 to 13.

**15.** A method according to claim 14 wherein said compound or composition is applied to insects or to growing plants or an area in which plants are to be grown at from 10 grams to 10 kilograms per hectare of the compound.

**16.** A method according to claim 15 wherein the application rate is from 100 grams to 5 kilograms per hectare of the compound.

**17.** A method according to any of claims 14 to 16 wherein said insects are from the order of Lepidoptera or Coleoptera.

Claims for the following Contracting State : AT

**1.** An insecticidally active composition which comprises a compound having the formula:

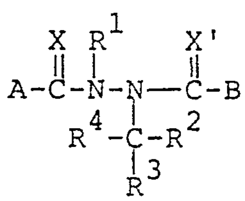

I

wherein

X and X' are the same or different 0, S or NR;

$R^1$ is hydrogen; $(C_1-C_6)$alkyl; $(C_1-C_6)$alkoxy-$(C_1-C_6)$alkyl having independently the stated number of carbon atoms in each alkyl group; $(C_1-C_6)$alkylthio-$(C_1-C_6)$alkyl having independently the stated number of carbon atoms in each alkyl group; $(C_2-C_6)$alkenyl; $(C_2-C_6)$alkynyl; or phen-$(C_1-C_4)$alkyl where the phenyl ring is unsubstituted or substituted with one to three of the same or different halo, cyano, nitro, hydroxy, $(C_1-C_4)$alkyl, halo-$(C_1-C_4)$alkyl $(C_1-c_4)$-alkoxy, halo-$(C_1-C_4)$alkoxy, carboxy, $(C_1-C_4)$alkoxy-carbonyl, $(C_1-C_4)$alkanoyloxy or NZZ';

$R^2$ and $R^3$ are the same or different hydrogen or $(C_1-C_4)$alkyl;

$R^4$ is $(C_1-C_4)$alkyl substituted with 1 to 4 fluoro; straight chain $(C_2-C_4)$alkenyl; carboxyl; $(C_1-C_3)$-alkoxy-carbonyl; cyano; cyano substituted $(C_1-C_4)$alkyl, tri$(C_1-C_4)$-alkylsilyl having independently the stated number of carbon atoms in each alkyl group; or tri$(C_1-C_2)$alkylsilylmethyl having independently the stated number of carbon atoms in each alkyl group; provided that $R^2$ and $R^3$ are both alkyl when $R^4$ is carboxyl or alkoxycarbonyl; and

A and B are the same or different unsubstituted or substituted naphthyl where the substituents can be from one to three of the same or different halo; nitro; $(C_1-C_4)$alkoxy; $(C_1-C_4)$alkyl or NZZ';

unsubstituted or substituted phenyl where the substituents can be from one to five of the same or different halo; nitroso; nitro; cyano; hydroxy; $(C_1-C_6)$alkyl; halo-$(C_1-C_6)$alkyl; cyano-$(C_1-C_6)$alkyl; hydroxy-$(C_1-C_6)$alkyl; $(C_1-C_6)$alkoxy; halo-$(C_1-C_6)$alkoxy; $(C_1-C_6)$alkoxy-$(C_1-C_6)$alkyl having independently the stated number of carbon atoms in each alkyl group; $(C_1-C_6)$alkoxy-$(C_1-C_6)$alkoxy having independently the stated number of carbon atoms in each alkyl group; -ORSR' group; $-OCO_2R$ group; $-OCO_2H$ group; $(C_1-C_6)$alkanoyloxy-$(C_1-C_6)$alkyl having independently the stated number of carbon atoms in each alkyl group; $(C_2-C_6)$alkenyl optionally substituted with halo, cyano, $(C_1-C_4)$-alkyl, halo-$(C_1-C_4)$alkoxy, $(C_1-C_4)$alkylthio or $(C_1-C_4)$alkoxy; $(C_3-C_5)$ alkadienyl; $(C_2-C_6)$alkenyloxy; $(C_2-C_6)$alkenyl-carbonyl; $(C_2-C_6)$alkenyloxy-carbonyloxy; $(C_2-C_6)$alkynyl optionally substituted with halo, cyano, nitro, hydroxy, $(C_1-C_4)$alkoxy, halo-$(C_1-C_4)$alkyl, $(C_1-C_4)$alkylthio or $(C_1-C_4)$alkyl; carboxy; $-RCO_2R'$ group; -COR; -COH; halo-$(C_1-C_6)$alkyl-carbonyl; $-CO_2R$ group; $(C_1-C_6)$haloalkoxy-carbonyl; -OCOR group; $-ORCO_2R'$ group; amino (-NZZ'); amino substituted with hydroxy, $(C_1-C_4)$-alkoxy or $(C_1-C_4)$alkylthio; -CONZZ' group; $(C_2-C_6)$alkenyl-carbonylamino; hydroxy-$(C_1-C_6)$-alkylamino-carbonyl; -OCONZZ' group; -NZCOZ' group; $-NZCO_2Z'$ group; thiocyanato;

isothiocyanato; ($C_1$-$C_6$)thiocyanatoalkyl; ($C_1$-$C_6$)alkylthio; halo-($C_1$-$C_6$)alkylthio; -S(O)Z group; -$SO_2$Z group; -$OSO_2$R group; -$OSO_2$H group; -$SO_2$NZZ' group; -CSR group; -CSH group; -SCOR group; -SCOH group; -NSCSZ' group; unsubstituted or substituted phenyl having one to three of the same or different halo, cyano, nitro, hydroxy, ($C_1$-$C_4$)alkyl, halo-($C_1$-$C_4$)alkyl, ($C_1$-$C_4$)alkoxy, carboxy, ($C_1$-$C_4$)alkoxy-carbonyl, ($C_1$-$C_4$)alkanoyloxy, amino, ($C_1$-$C_4$)alkylamino or di($C_1$-$C_4$)alkylamino having independently the stated number of carbon atoms in each alkyl group; phenoxy where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, carboxy, ($C_1$-$C_4$)alkyl-carbonyl, ($C_1$-$C_4$)-alkanoyloxy, amino, ($C_1$-$C_4$)alkylamino or di($C_1$-$C_4$)alkylamino having independently the stated number of carbon atoms in each alkyl group; benzoyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, carboxy, ($C_1$-$C_4$)alkoxy-carbonyl, ($C_1$-$C_4$)alkanoyloxy, amino, ($C_1$-$C_4$)-alkylamino or di($C_1$-$C_4$)alkylamino having independently the stated number of carbon atoms in each alkyl group; benzoyloxy-($C_1$-$C_6$)alkyl; phenylthio-($C_1$-$C_6$)alkyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, carboxy, ($C_1$-$C_4$)alkoxy-carbonyl, ($C_1$-$C_4$)-alkanoyloxy, amino, ($C_1$-$C_4$)alkylamino or di($C_1$-$C_4$)alkylamino having independently the stated number of carbon atoms in each alkyl group; -CR=N-R> group where R> is hydroxy, ($C_1$-$C_4$)alkyl, ($C_1$-$C_4$)alkoxy, amino (-NZZ'), phenylamino, -COR, -COH or benzoyl; ($C_2$-$C_6$)oxiranyl; acetylthiosemi- carbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;

unsubstituted or substituted ($C_1$-$C_{10}$)alkyl having one to four of the same or different halo, cyano, nitro, hydroxy, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy, phenyl or -NZZ';

unsubstituted or substituted ($C_3$-$C_8$)cycloalkyl or unsubstituted or substituted ($C_3$-$C_8$)cyclo($C_1$-$C_4$)-alkyl having one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkanoyl, ($C_1$-$C_4$)alkoxy-carbonyl, ($C_1$-$C_4$)alkanoyloxy or -NZZ';

unsubstituted or substituted ($C_2$-$C_8$)alkenyl or unsubstituted or substituted ($C_2$-$C_8$)($C_3$-$C_5$)-alkadienyl having as substituent(s) a furyl, thienyl or pyridyl or one to four of the same or different halo, cyano, nitro, hydroxy, ($C_1$-$C_4$)alkyl, ($C_3$-$C_6$)cycloalkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy or -NZZ';

unsubstituted or substituted ($C_3$-$C_8$)alkenyl or unsubstituted or substituted ($C_3$-$C_8$)cycloalkadienyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy or -NZZ';

unsubstituted or substituted ($C_2$-$C_8$)alkynyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)-alkoxy, carboxy, ($C_1$-$C_4$)alkoxy-carbonyl, ($C_1$-$C_4$)alkanoyloxy, phenyl or -NZZ';

phenalkyl having one to four carbon atoms in the alkyl group and wherein the alkyl group is unsubstituted or substituted with one to three of the same or different halo, cyano, hydroxy, ($C_1$-$C_4$)-alkoxy, ($C_1$-$C_4$)alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, cyano-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)-alkanoyloxy, ($C_2$-$C_6$)alkenyl, halo-($C_2$-$C_6$)alkenyl, ($C_2$-$C_6$)alkynyl or -NZZ';

phenalkenyl having two to six carbon atoms in the alkenyl group and the alkenyl group is unsubstituted or substituted with one to three of the same or different halo, cyano, hydroxy, ($C_1$-$C_4$)-alkyl, halo-($C_1$-$C_4$)alkyl, ($C_1$-$C_4$)alkoxy, halo-($C_1$-$C_4$)alkoxy, ($C_1$-$C_4$)alkoxy-carbonyl or -NZZ', and the phenyl ring is unsubstituted or substituted with one to three of the same or different halo, cyano,

nitro, hydroxy, $(C_1-C_4)$alkyl, halo-$(C_1-C_4)$alkyl, $(C_1-C_4)$alkoxy, halo-$(C_1-C_4)$alkoxy, carboxy, $(C_1-C_4)$-alkoxy-carbonyl, $(C_1-C_4)$alkanoyloxy or -NZZ';

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substituents can be from one to three of the same or different halo; nitro; hydroxy; $(C_1-C_6)$alkyl; $(C_1-C_6)$alkoxy; carboxy; $(C_1-C_6)$alkoxy-carbonyl; $-RC0_2H$ group; $RC0_2R'$ group; -C0NZZ' group; amino (-N'ZZ'); -NZC0Z' group; $(C_1-C_6)$alkylthio; or unsubstituted or substituted phenyl having one to three of the same or different halo, nitro, $(C_1-C_6)$alkyl, halo$(C_1-C_6)$alkyl, $(C_1-C_6)$alkoxy, halo-$(C_1-C_6)$-aloxy, carboxy, $(C_1-C_4)$alkoxy-carbonyl or amino (-NZZ'); or

unsubstituted or substituted six-membered heterocycle having one, two, three or four nitrogen atoms and two to five nuclear carbon atoms where the substituents can be from one to three of the same or different halo; nitro; hydroxy; $(C_1-C_6)$alkyl; $(C_1-C_6)$alkoxy; carboxy; $(C_1-C_6)$-alkoxy-carbonyl; $-RC0_2H$ group; $-RC0_2R'$ group; -C0NZZ' group; amino (-NZZ'); -NZC0Z' group; $(C_1-C_6)$alkylthio; or unsubstituted or substituted phenyl having one to three of the same or different halo, nitro, $(C_1-C_6)$-alkyl, halo- $(C_1-C_6)$alkyl, $(C_1-C_6)$alkoxy, halo-$(C_1-C_6)$- alkoxy, carboxy, $(C_1-C_4)$alkoxy-carbonyl or amino (-NZZ');

where R and R' are $(C_1-C_6)$alkyl; Z and Z' are hydrogen or $(C_1-C_4)$alkyl; "amino" means NZZ'; or an agronomically acceptable salt thereof; together with agronomically acceptable diluent or carrier.

**2.** A composition according to claim 1 wherein
X and X' are 0 or S;
$R^1$ is hydrogen; $(C_1-C_4)$alkyl; $(C_1-C_4)$alkoxy-$(C_1-C_4)$alkyl having independently the stated number of carbon atoms in each alkyl group; methylthiomethyl; $(C_2-C_5)$alkenyl; $(C_2-C_5)$-alkynyl; or phen-$(C_1-C_2)$alkyl where the phenyl ring is unsubstituted or substituted with one or two halo, nitro, $(C_1-C_4)$alkyl or $(C_1-C_4)$alkoxy;
$R^2$ and $R^3$ are the same or different hydrogen or $(C_1-C_3)$alkyl;
$R^4$ is $(C_1-C_3)$alkyl substituted with one to four fluoro; straight chain $(C_2-C_4)$alkenyl; carboyl; $(C_1-C_2)$-alkoy-carbonyl; cyano; cyano substituted $(C_1-C_3)$alkyl; tri$(C_1-C_2)$-alkylsilyl having independently the stated number of carbon atoms in each alkyl group; or tri$(C_1-C_2)$alkylsilylmethyl having independently the stated number of carbon atoms in each alkyl group; and
A and B are the same or different
unsubstituted naphthyl;
unsubstituted or substituted phenyl where the substituents can be from one to three of the same or different halo; nitro; cyano; $(C_1-C_4)$alkyl; halo-$(C_1-C_4)$alkyl; cyano-$(C_1-C_4)$alkyl; $(C_1-C_4)$alkoxy; $(C_1-C_4)$-alkoxy-$(C_1-C_4)$alkyl having independently the stated number of carbon atoms in each alkyl group; -C0Z; carboxy; $(c_1-c_4)$ - alkoy-carbonyl; $(C_1-C_4)$alkanoyloxy; $(6_2-C_6)$alkenyl; $(C_2-C_6)$alkynyl; amino (-NZZ'); thiocyanato; $(C_1-C_4)$alkylthio; -CSR; -CSH; unsubstituted or substituted phenyl having one to two of the same or different halo, nitro, $(C_1-C_4)$alkyl, $(C_1-C_4)$alkoxy, carboxy, -NZZ'; phenory where the phenyl ring is unsubstituted or substituted with one or two of the same or different halo, nitro, $(C_1-C_4)$alkyl, $(C_1-C_4)$alkoxy, carboxy, -NZZ'; or when two adjacent positions on the phenyl ring are substituted with alkoxy groups, these groups may be joined to form, together with the carbon atoms to which they are attached, a 5- or 6-membered dioyolano or dioxano heterocyclic ring;

unsubstituted or substituted $(C_1-C_8)$alkyl having one to three of the same or different halo, cyano, $(C_1-C_4)$alkoxy, phenyl, $(C_1-C_4)$alkoxy-carbonyl or halo-$(C_1-C_4)$alkoxy;

unsubstituted or substituted $(C_3-C_6)$cyclo-alkyl or unsubstituted or substituted $(C_3-C_6)$cycloalkyl-$(C_1C_4)$alkyl having one or two of the same or different halo, $(C_1-c_4)$-alkyl, halo-$(C_1-C_4)$alkyl, $(C_1-C_4)$-alkoxy, $(C_1-C_4)$alkanoyl or halo-$(C_1-C_4)$alkoxy;

unsubstituted or substituted $(C_2-C_6)$alkenyl having as substituent(s) a furyl or one to three of the same or different $(C_1-C_4)$-alkyl, halo-$(C_1-C_4)$alkyl, $(6_1-C_4)$alkoxy or halo-$(C_1-C_4)$alkoxy;
unsubstituted or substituted $(C_3-C_6)$cyclo-alkenyl having as substituents one or two of the same or different halo, $(c_1-c_4)$-alkyl, halo-$(C_1-C_4)$alkyl, $(C_1-C_4)$alkoxy or halo-$(C_1-C_4)$alkoxy;

unsubstituted or phenyl substituted alkynyl;

phenalkyl having one or two carbon atoms in the alkyl group and the alkyl group is unsubstituted or substituted with one or two of the same or different halo, $(C_1-c_4)$-alkyl, halo-$(C_1-C_4)$alkyl, $(C_1-C_4)$-alkoxy or halo-$(C_1-C_4)$alkoxy and the phenyl ring is unsubstituted or substituted with one or two of the same or different halo, cyano, $(C_1-C_4)$alkyl, halo-$(C_1-C_4)$alkyl, $(C_1-C_4)$-alkoxy or halo-$(C_1-C_4)$-alkoxy;

phenalkenyl having two or three carbon atoms in the alkenyl group and the alkenyl group is unsubstituted or substituted with halo, $(C_1-C_4)$alkyl, halo-$(C_1-C_4)$alkyl, $(C_1-C_4)$alkoxy or halo-$(C_1-C_4)$-alkoxy and the phenyl ring is unsubstituted or substituted with one or two of the same or different halo, cyano, $(C_1-C_4)$alkyl, halo-$(C_1-C_4)$-alkyl, $(C_1-C_4)$alkoxy or halo-$(C_1-C_4)$alkoxy;

unsubstituted or substituted five-membered heterocycle selected from furyl, thienyl, triazolyl, pyrrolyl, and oxazolyl where the substituents can be one or two of the same or different halo; nitro; $(C_1-C_4)$alkyl;
$(C_1-C_4)$alkoxy; -NZZ'; or unsubstituted or substituted phenyl having one or two of the same or different halo, nitro, $(C_1-c_4)$-alkyl, halo-$(C_1-C_4)$alkyl, $(C_1-C_4)$alkoxy, halo-$(C_1C_4)$alkoxy, carboxy or -NZZ'; or

unsubstituted or substituted six-membered heterocycle having one or two nitrogen atoms and 4 to 5 nuclear carbon atoms where the substituents can be one or two of the same or different halo; nitro; $(C_1-C_4)$-alkyl; $(C_1-C_4)$alkoxy; thio-$(C_1C_4)$alkoxy; -NZZ'; or unsubstituted or substituted phenyl having one or two of the same or different halo, nitro, $(C_1-C_4)$alkyl, halo$(C_1-C_4)$alkyl, $(C_1-C_4)$alkoxy, halo-$(C_1-C_4)$-alkoxy, carboxy or -NZZ';

where R and R' are $(C_1-C_4)$alkyl; Z and Z' are hydrogen or $(C_1-C_4)$alkyl.

**3.** A composition according to claim 2 wherein
X and X' are O or S;
$R^1$ is hydrogen; $(C_1-C_4)$alkyl; $(C_1-C_4)$alkoxy-$(C_1-C_4)$alkyl having independently the stated number of carbon atoms in each alkyl group; $(C_2-C_5)$alkenyl; $(C_2-C_5)$alkynyl; or benzyl where the phenyl ring is unsubstituted or substituted with one halo, nitro, $(C_1-C_4)$alkyl or $C_1-C_4)$alkoxy;
$R^2$ and $R^3$ are the same or different hydrogen or $(C_1-C_2)$alkyl;
$R^4$ is $(C_1-C_3)$alkyl substituted with 1 to 4 fluoro; straight chain $(C_2-C_4)$alkenyl; methoxycarbonyl; cyano; cyano substituted $(C_1-C_2)$alkyl; tri$(C_1-C_2)$alkylsilyl having independently the stated number of carbon atoms in each alkyl group; or tri$(C_1-C_2)$-alkylsilylmethyl having independently the stated number of carbon atoms in each alkyl group; provided that $R^2$ and $R^3$ are both alkyl when $R^4$ is methoxycarbonyl; and
A and B are the same or different
unsubstituted naphthyl;

unsubstituted or substituted phenyl having one to three of the same or different halo; nitro; cyano; $(C_1-C_4)$alkyl; halo-$(C_1-C_4)$-alkyl; cyano-$(C_1-C_4)$alkyl; $(C_1-C_4)$alkoxy; $(C_1-C_4)$alkoxy-$(C_1-C_4)$alkyl having independently the stated number of carbon atoms in each alkyl group; -C0Z; $(C_1-c_4)$-alkoxy-carbonyl; $(C_1-C_4)$alkanoyloxy; thiocyanato; -Nzz'; $(C_1-C_4)$alkylthio; -CSZ; $-CS_2Z$; unsubstituted or substituted phenyl having one or two of the same or different halo, nitro, $(C_1-C_4)$alkyl, $(C_1-C_4)$alkoxy, carboxy, -N'ZZ'; or phenoxy where the phenyl ring is unsubstituted or substituted with one or two of the same or different halo, nitro, $(C_1-C_4)$alkyl, $(C_1-C_4)$alkoxy, carboxy, -NZZ' or when two adjacent positions on the phenyl ring are substituted with alkoxy groups, these groups may be joined to form, together with the carbon atoms to which they are attached, a 5- or 6-membered dioxolano or dioxano heterocyclic ring;
unsubstituted or substituted $(C_1-C_6)$alkyl having one to three of the same or different halo, cyano, nitro, carboxy, $(C_1-C_4)$alkoxy, phenyl, $(C_1-C_4)$-alkoxy-carbonyl or halo-$(C_1-C_4)$alkoxy;

unsubstituted or substituted $(C_3-C_6)$cyclo-alkyl or unsubstituted or substituted $(C_3-C_6)$cycloalkyl$(C_1-C_4)$alkyl, where the substituent is halo, $(C_1-C_4)$alkyl, $(C_1-C_4)$alkanoyl, $(C_1-C_4)$alkoxy or halo$(C_1-C_4)$-alkoxy;

unsubstituted or substituted $(C_2-C_6)$-alkenyl having one or two of the same or different halo, $(C_1-C_4)$-alkyl, $(C_1-C_4)$alkoxy or halo-$(C_1-C_4)$alkoxy;

unsubstituted or substituted $(C_4-C_6)$cyclo-alkenyl where the substituent is halo $(C_1-C_4)$alkyl, $(C_1-C_4)$-alkoxy or halo$(C_1-C_4)$alkoxy;

unsubstituted or phenyl substituted alkynyl;

benzyl where the phenyl ring is unsubstituted or substituted with one or two of the same or different halo, methyl or ethyl;

unsubstituted or substituted five-membered heterocycle selected from furyl, thienyl, pyrrolyl and oxazolyl where the substituents can be one or two of the same or different halo, nitro, $(C_1-C_4)$alkyl or $(C_1-C_4)$alkoxy; or

unsubtituted or substituted six-membered heterocycle having one or two nitrogen atoms and four to five nuclear carbon atoms where the substituents can be one or two of the same or different halo, nitro, $(C_1-C_4)$-alkyl, $(C_1-C_4)$alkoxy or thio-$(C_1-C_4)$alkoxy;

where Z and Z' are hydrogen or $(C_1-C_4)$alkyl.

**4.** A composition according to claim 3 wherein
$X_1$ and X' are O;
$R^1$ is hydrogen; methyl; ethyl; $(C_1-C_2)$alkoxy-$(C_1-C_2)$alkyl having independently the stated number of carbon atoms in each alkyl group $(C_2-C_5)$alkenyl; $(C_2-C_5)$alkynyl; or benzyl where the phenyl ring is unsubstituted or substituted with halo;
$R^2$ and $R^3$ are the same or different hydrogen or $(C_1-C_2)$alkyl;
$R^4$ is $(C_1-C_2)$alkyl substituted with 1 to 4 fluoro; straight chain $(C_2-C_4)$alkenyl; cyano; cyano substituted $(C_1-C_2)$alkyl; tri$(C_1-C_2)$alkylsilyl having independently the stated number of carbon atoms in each alkyl group; or tri$(C_1C_2)$alkylsilylmethyl having independently the stated number of carbon atoms in each alkyl group; and
A and B are the same or different phenyl or substituted phenyl where the substituents can be from one to three of the same or different halo, nitro, $(C_1-C_4)$alkyl, $(C_1-C_4)$alkoxy or halo-$(C_1C_4)$alkyl;

unsubstituted or substituted $(C_1-C_6)$alkyl having as substituent(s) one to two of the same or different halo, phenyl or cyano;

unsubstituted $(C_4-C_6)$cycloalkyl;

unsubstituted or substituted $(C_2-C_6)$alkenyl having as substituent(s) one to three of the same or different halo or $(C_1-C_4)$alkyl;

unsubstituted $(C_4-C_6)$cycloalkenyl;

phenalkyl having one or two carbon atoms in the alkyl group and the phenyl ring is unsubstituted or substituted with one or two of the same or different halo, $(C_1-C_4)$-alkyl or $(C_1-C_4)$alkoxy;

unsubstituted or substituted pyridyl or 2,5-pyrazinyl where the substituent can be halo, nitro, $(C_1-C_4)$-alkyl, $(C_1-C_4)$alkoxy or $(C_1-C_4)$thioalkoxy; or

unsubstituted furyl or thienyl or an unsubstituted or substituted pyrrolyl where the substituent can be $(C_1-C_4)$alkyl.

**5.** A composition according to claim 4 wherein
$X_1$ and X' are 0;
$R^1$ is hydrogen; methyl; methoxymethyl; $(C_2-C_4)$-alkenyl; $(C_2-C_5)$alkynyl; benzyl; or 4-halobenzyl;
$R^2$ and $R^3$ are the same or different hydrogen, methyl or ethyl;

$R^4$ is trifluoromethyl; 2,2,2-trifluoroethyl; straight chain ($C_2$-$C_4$)alkenyl; cyano; cyanomethyl; tri($C_1$-$C_2$)alkylsilyl having independently the stated number of carbon atoms in each alkyl group; or trimethylsilylmethyl; and
A and B are the same or different phenyl or substituted phenyl where the substituents can be one, two or three of the same or different chloro, fluoro, bromo, iodo, nitro, methyl, ethyl, methoxy or trifluoromethyl;

($C_2$-$C_3$) substituted alkenyl having one to three of the same or different chloro, bromo, methyl or ethyl;

cyclohexenyl;

benzyl;

unsubstituted or substituted pyridyl or 2,5-pyrazinyl where the substituent can be halo, methyl, ethyl or methoxy; or

unsubstituted furyl or thienyl or an unsubstituted or substituted pyrrolyl where the substituent can be ethyl or methyl.

**6.** A composition according to claim 2 wherein
X and X' are O;
$R_1$ is hydrogen or methyl;
$R^2$ and $R^3$ are hydrogen or ($C_1$-$C_3$)alkyl;
$R^4$ is trifluoromethyl, 2,2,2-trifluoroethyl, cyano, cyanomethyl, straight chain ($C_2$-$C_4$)-alkenyl, carboyl, methoxycarbonyl, tri($C_1$-$C_2$)alkylsilyl having independently the stated number of carbon atoms in each alkyl group or tri($C_1$-$C_2$)alkylsilylmethyl having independently the stated number of carbon atoms in each alkyl group; and
A and B are the same or different thienyl or unsubstituted phenyl or substituted phenyl where the substituents can be one to three of the same or different chloro, fluoro, nitro, methyl, ethyl or propyl.

**7.** A composition according to claim 6 wherein
$X_1$ and X' are 0;
$R^1$ is hydrogen;
$R^2$ and $R^3$ are hydrogen, methyl or ethyl;
$R^4$ is trifluoromethyl, cyano, allyl, methoxycarbonyl, or trimethylsilyl; and
A and B are the same or different unsubstituted or substituted phenyl having one or two of the same or different chloro, fluoro, nitro, methyl and ethyl; or unsubstituted thienyl.

**8.** A composition according to claim 7 where
X and X' are O;
$R^1$ is hydrogen; and
$R^2$ is hydrogen, $R^3$ is methyl, $R^4$ is trifluoromethyl, and A and B are the same or different unsubstituted or substituted phenyl where the substituents can be one or two of the same or different chloro, methyl or nitro; or
$R^2$ and $R^3$ are the same or different methyl or ethyl, $R^4$ is cyano, and A and B are the same or different unsubstituted or substituted phenyl where the substituents can be one or two of the same or different chloro, fluoro, methyl, ethyl or nitro; or
$R^2$ and $R^3$ are methyl, $R^4$ is allyl, and A and B are the same or different unsubstituted or methyl substituted phenyl; or
$R^2$ and $R^3$ are hydrogen, $R^4$ is trimethylsilyl, and A and B are the same or different substituted phenyl where the substituents can be one or two of the same or different methyl, ethyl or nitro.

**9.** A composition according to claim 8 wherein
X and X' are 0;
$R^1$ is hydrogen; and
$R^2$ is hydrogen, $R^3$ is methyl, $R^4$ is trifluoromethyl, and A and B are unsubstituted phenyl, or A is 2,3-dimethylphenyl and B is 2,4-dichlorophenyl, 3,5-dimethylphenyl or 2-nitro-5-methylphenyl; or

$R^2$ and $R^3$ are methyl, $R^4$ is allyl, and A and B are both unsubstituted phenyl or 3-methyl-phenyl; or $R^2$ and $R^3$ are hydrogen, $R^4$ is trimethylsilyl, and A is 4-ethylphenyl and B is 2-nitro-5-methylphenyl, or A and B are both 2-methyl-phenyl or 2-nitrophenyl.

**10.** A composition according to claim 8 wherein
X and X' are 0;
$R^1$ is hydrogen;
$R^2$ and $R^3$ are methyl;
$R^4$ is cyano; and
A and B are the same or different unsubstituted or substituted phenyl where the substituents can be one or two of the same or different chloro, fluoro, methyl, ethyl or nitro.

**11.** A composition according to any preceding claim containing said insecticidal compound in an amount of from 0.0001 to 99% by weight of the composition.

**12.** A composition according to any preceding claim wherein the amount of said insecticidal compound is from 0.01 to 99% by weight of the composition.

**13.** A method of controlling insects which comprises contacting said insects with an insecticidally effective amount of a compound as defined in any one of claims 1 to 10 optionally in a composition according to any one of claims 1 to 12.

**14.** A method according to claim 13 wherein said compound or composition is applied to insects or to growing plants or an area in which plants are to be grown at from 10 grams to 10 kilograms per hectare of the compound.

**15.** A method according to claim 14 wherein the application rate is from 100 grams to 5 kilograms per hectare of the compound.

**16.** A method according to any of claims 13 to 15 wherein said insects are from the order of Lepidoptera or Coleoptera.

Claims for the following Contracting State : ES

**1.** The use of an insecticidally active compound which is a substituted hydrazine having the formula:

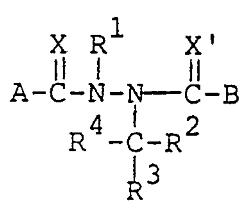

I

wherein
X and X' are the same or different 0, S or NR;
$R^1$ is hydrogen; $(C_1-C_6)$alkyl; $(C_1-C_6)$alkoxy-$(C_1-C_6)$alkyl having independently the stated number of carbon atoms in each alkyl group; $(C_1C_6)$alkylthio$(C_1C_6)$alkyl having independently the stated number of carbon atoms in each alkyl group; $(C_2-C_6)$alkenyl; $(C_2-C_6)$alkynyl; or phen-$(C_1-C_4)$alkyl where the phenyl ring is unsubstituted or substituted with one to three of the same or different halo, cyano, nitro, hydroxy, $(C_1-C_4)$alkyl, halo-$(C_1-C_4)$alkyl $(C_1-C_4)$-alkoxy, halo-$(C_1-C_4)$alkoxy, carboxy, $(C_1-C_4)$alkoxy-carbonyl, $(C_1-C_4)$alkanoyloxy or NZZ';
$R^2$ and $R^3$ are the same or different hydrogen or $(C_1-C_4)$alkyl;
$R^4$ is $(C_1-C_4)$alkyl substituted with 1 to 4 fluoro; straight chain $(C_2-C_4)$alkenyl; carboxyl; $(C_1-C_3)$-alkoxy-carbonyl; cyano; cyano substituted $(C_1-C_4)$alkyl, tri$(C_1-C_4)$-alkylsilyl having independently the stated number of carbon atoms in each alkyl group; or tri$(C_1C_2)$alkylsilylmethyl having independently the stated number of carbon atoms in each alkyl group; provided that $R^2$ and $R^3$ are both alkyl when $R^4$ is carboxyl or alkoxycarbonyl; and

A and B are the same or different unsubstituted or substituted naphthyl where the substituents can be from one to three of the same or different halo; nitro; ($c_1$-$C_4$) - alkoxy; ($C_1$-$C_4$)alkyl or NZZ';

unsubstituted or substituted phenyl where the substituents can be from one to five of the same or different halo; nitroso; nitro; cyano; hydroxy; ($C_1$-$C_6$)alkyl; halo-($C_1$-$C_6$)-alkyl; cyano($C_1C_6$)alkyl; hydroxy-($C_1C_6$)-alkyl; ($C_1$-$C_6$)alkoxy; halo($C_1$-$C_6$)alkoxy; ($C_1C_6$)alkoxy-($C_1C_6$)alkyl having independently the stated number of carbon atoms in each alkyl group; ($C_1$-$C_6$)alkoxy-($C_1$-$C_6$)alkoxy having independently the stated number of carbon atoms in each alkyl group; -0RSR' group; -0C0$_2$R group; -0C0$_2$H group; ($C_1C_6$)alkanoyloxy($C_1C_6$)alkyl having independently the stated number of carbon atoms in each alkyl group; ($C_2$-$C_6$)alkenyl optionally substituted with halo, cyano, ($C_1$-$C_4$)alkyl, halo-($C_1$-$C_4$)-alkoxy, ($C_1$-$C_4$)alkylthio or ($C_1$-$C_4$)alkoxy; ($C_{3\text{-}5}$) Alkadienyl; ($C_2$-$C_6$) alkenyloxy; ($C_2$-$C_6$)-alkenyl-carbonyl; ($C_2$-$C_6$)-alkenyloxy-carbonyloxy; ($C_2$-$C_6$)alkynyl optionally substituted with halo, cyano, nitro, hydroxy, ($C_1$-$C_4$)alkoxy, halo($C_1$-$C_4$)alkyl, ($C_1$-$C_4$)alkylthio or ($C_1$-$C_4$)-alkyl; carboxy; -RC0$_2$R' group; -C0R; -C0H; hal0($C_1$-$C_6$)alkyl-carbonyl; -C0$_2$R group; ($C_1C_6$)haloalkoxy-carbonyl; -0C0R group; -ORCO$_2$R' group; amino (-NZZ'); amino substituted with hydroxy, ($C_1$-$C_4$)alkoxy or ($C_1$-$C_4$)alkylthio; -C0NZZ' group; ($C_2$-$c_6$)-alkenyl-carbonylamino; hydroxy-($C_1C_6$)-alkylamino-carbonyl; -0C0NZZ' group; -N2C0Z' group; -N2C0$_2$Z' group; thiocyanato; isothiocyanato; ($C_1$-$C_6$)-thiocyanatoalkyl; ($C_1$-$C_6$)alkylthio; halo($C_1C_6$)alkylthio; -S(0)Z group; S0$_2$Z group; -OSO$_2$R group; 0S0$_2$H group; -SO$_2$NZZ' group; -CSR group; -CSH group; -SC0R group; -SC0H group; -NSCSZ' group; unsubstituted or substituted phenyl having one to three of the same or different halo, cyano, nitro, hydroxy, ($C_1$-$C_4$)alkyl, halo-($C_1C_4$)alkyl, ($c_1$-$c_4$)-alkoxy, carboxy, ($C_1$-$C_4$)alkoxy-carbonyl, ($C_1$-$C_4$)alkanoyloxy, amino, ($C_1$-$C_4$)-alkylamino or di($C_1$-$C_4$)alkylamino having independently the stated number of carbon atoms in each alkyl group; phenoxy where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)-alkoxy, carboxy, ($C_1$-$C_4$)alkyl-carbonyl, ($C_1$-$C_4$)alkanoyloxy, amino, ($C_1$-$C_4$)-alkylamino or di($C_1$-$C_4$)alkylamino having independently the stated number of carbon atoms in each alkyl group; benzoyl where the phenyl ring is unsubstituted or substituted with one to three of the same or different halo, cyano, nitro, hydroxy, ($C_1$-$C_4$)alkyl, halo($C_1$-$C_4$)alkyl, ($C_1$-$C_4$)-alkoxy, carboxy, ($C_1$-$C_4$)alkoxy-carbonyl, ($C_1$-$C_4$)alkanoyloxy, amino, ($C_1$-$C_4$)alkyl-amino or di($C_1$-$C_4$)alkylamino having independently the stated number of carbon atoms in each alkyl group; benzoyloxy($C_1$-$C_6$)alkyl; phenylthio($C_1$-$C_6$)alkyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)-alkoxy, carboxy, ($C_1$-$C_4$)alkoxy-carbonyl, ($C_1$-$C_4$)-alkanoyloxy, amino, ($C_1$-$C_4$)alkyl-amino or di($C_1$-$C_4$)alkylamino having independently the stated number of carbon atoms in each alkyl group; -CR=N-R'" group where R'" is hydroxy, ($C_1$-$C_4$)alkyl, ($C_1$-$C_4$)alkoxy, amino (-NZZ'), phenylamino, -C0R, -C0H or benzoyl; ($C_2$-$C_6$)oxiranyl; acetylthiosemi- carbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;

unsubstituted or substituted ($C_1$-$C_{10}$)alkyl having one to four of the same or different halo, cyano, nitro, hydroxy, ($C_1$-$C_4$)alkoxy, halo-($C_1$-$C_4$)alkoxy, carboxy, ($C_1$-$C_4$)-alkoxy-carbonyl, ($C_1$-$C_4$)-alkanoyloxy, phenyl or -NZZ';

unsubstituted or substituted ($C_3$-$C_8$)cyclo-alkyl or unsubstituted or substituted ($C_3$-$C_8$)cyclo($C_1$-$C_4$)-alkyl having one to four of the same or different halo, cyano, nitro, hydroxy, ($C_1$-$C_4$)alkyl, halo-($C_1$-$C_4$)-alkyl, ($C_1$-$C_4$)alkoxy, halo($C_1$-$C_4$)alkoxy, carboxy, ($C_1$-$C_4$)alkanoyl, ($C_1$-$C_4$)-alkoxy-carbonyl, ($C_1$-$C_4$)alkanoyloxy or -NZZ';

unsubstituted or substituted ($C_2$-$C_8$)alkenyl or unsubstituted or Substitued ($C_3$-$C_5$) -alkadienyl having as substituent(s) a furyl, thienyl or pyridyl or one to four of the same or different halo, cyano, nitro, hydroxy, ($C_1$-$C_4$)alkyl, ($C_3$-$C_6$)cycloalkyl, halo-($C_1$-$C_4$)alkyl, ($C_1$-$C_4$)alkoxy, halo($C_1$-$C_4$)alkoxy, carboxy, ($C_1$-$C_4$)-alkoxy-carbonyl, ($C_1$-$C_4$)alkanoyloxy or -NZZ';

unsubstituted or substituted ($C_3$-$C_8$)cyclo-alkenyl or unsubstituted or substituted (C8) cycloalkadienlyl having as substituent(s) one to four of the same or different halo, cyano, nitro, hydroxy, ($C_1$-$C_4$)-alkyl, halo-($C_1$-$C_4$)alkyl, ($C_1$-$C_4$)-alkoxy, halo-($C_1$-$C_4$)alkoxy, carboxy, ($C_1$-$C_4$)alkoxy-carbonyl, ($C_1$-$C_4$)alkanoyloxy or -NZZ';

unsubstituted or substituted ($C_2$-$C_8$)alkynyl having as substituent(s) one to four of the same or different halo, cyano, nitro, hydroxy, ($C_1$-$C_4$)alkyl, halo-($C_1$-$C_4$)alkyl, ($C_1$-$C_4$)alkoxy, halo-($C_1C_4$)-alkoxy, carboxy, ($C_1$-$C_4$)alkoxy-carbonyl, ($C_1$-$C_4$)alkanoyloxy, phenyl or -NZZ';

phenalkyl having one to four carbon atoms in the alkyl group and wherein the alkyl group is unsubstituted or substituted with one to three of the same or different halo, cyano, hydroxy, ($C_1$-$C_4$)-alkoxy, ($C_1$-$C_4$)-alkoxy-carbonyl or -NZZ', and the phenyl ring is unsubstituted or substituted with one to three of the same or different halo, cyano, nitro, hydroxy, ($C_1$-$C_4$)alkyl, halo-($C_1$-$C_4$)alkyl, cyano($C_1C_4$)alkyl, ($C_1$-$C_4$)-alkoxy, halo($C_1C_4$)alkoxy, carboxy, ($C_1C_4$)alkoxy-carbonyl, ($C_1$-$C_4$)-alkanoyloxy, ($C_2$-$C_6$)alkenyl, halo($C_2$-$C_6$)alkenyl, ($C_2$-$C_6$)alkynyl or -NZZ';

phenalkenyl having two to six carbon atoms in the alkenyl group and the alkenyl group is unsubstituted or substituted with one to three of the same or different halo, cyano, hydroxy, ($C_1$-$C_4$)-alkyl, halo($C_1$-$C_4$)alkyl, ($C_1$-$C_4$)alkoxy, halo-($C_1$-$C_4$)alkoxy, ($C_1$-$C_4$)-alkoxy-carbonyl or -NZZ', and the phenyl ring is unsubstituted or substituted with one to three of the same or different halo, cyano, nitro, hydroxy, ($C_1$-$C_4$)alkyl, halo($C_1$-$C_4$)alkyl, ($C_1$-$C_4$)alkoxy, halo-($C_1$-$C_4$)-alkoxy, carboxy, ($C_1$-$C_4$)-alkoxy-carbonyl, ($C_1$-$C_4$)alkanoyloxy or -NZZ';

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; -$RC0_2N$ group; $RC0_2R'$ group; -C0NZZ' group; amino (-NZZ'); -N2C0Z' group; ($C_1$-$C_6$)alkylthio; or unsubstituted or substituted phenyl having one to three of the same or different halo, nitro, ($C_1$-$C_6$)alkyl, halo($C_1$-$C_6$)alkyl, ($C_1$-$C_6$)alkoxy, halo-($C_1$-$C_6$)-alkoxy, carboxy, ($C_1$-$C_4$)alkoxy-carbonyl or amino (-NZZ'); or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; carboxy; ($C_1$-$C_6$)-alkoxy-carbonyl; -$RC0_2N$ group; -$RC0_2R'$ group; -C0NZZ' group; amino (-NZZ'); -NZC0Z' group; ($C_1$-$C_6$)alkylthio; or unsubstituted or substituted phenyl having one to three of the same or different halo, nitro, ($C_1$-$C_6$)-alkyl, halo- ($C_1$-$C_6$)alkyl, ($C_1$-$C_6$)alkoxy, halo-($C_1$-$C_6$) alkoxy, carboxy, ($C_1$-$C_4$)alkoxy-carbonyl or amino (-NZZ');

where R and R' are ($C_1$-$C_6$)alkyl; Z and Z' are hydrogen or ($C_1$-$C_4$)alkyl; "amino" means NZZ'; or an agronomically acceptable salt thereof; together with agronomically acceptable diluent or carrier to make an insecticidal composition.

2. The use according to claim 1 wherein, in the compound,
X and X' are 0 or S;
$R^1$ is hydrogen; ($C_1$-$C_4$)alkyl; ($C_1$-$C_4$)alkoxy-($C_1$-$C_4$)alkyl having independently the stated number of carbon atoms in each alkyl group; methylthiomethyl; ($C_2$-$C_5$)alkenyl; ($C_2$-$C_5$)- alkynyl; or phen-($C_1$-$C_2$)alkyl where the phenyl ring is unsubstituted or substituted with one or two halo, nitro, ($C_1$-$C_4$)alkyl or ($C_1$-$C_4$)alkoxy;
$R^2$ and $R^3$ are the same or different hydrogen or ($C_1$-$C_3$)alkyl;
$R^4$ is ($C_1$-$C_3$)alkyl substituted with one to four fluoro; straight chain ($C_2$-$C_4$)alkenyl; carboxyl; ($C_1$-$C_2$)-alkoxy-carbonyl; cyano; cyano substituted ($C_1$-$C_3$)alkyl; tri($C_1$-$C_2$)-alkylsilyl having independently the stated number of carbon atoms in each alkyl group; or tri($C_1$-$C_2$)alkylsilylmethyl having independently the stated number of carbon atoms in each alkyl group; and
A and B are the same or different
unsubstituted naphthyl;

unsubstituted or substituted phenyl where the substituents can be from one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)-alkoxy-($C_1$-$C_4$)alkyl having independently the stated number of carbon atoms in each alkyl group; -C0Z; carboxy; ($C_1$-$C_4$)-alkoxy-carbonyl; ($C_1$-$C_4$)alkanoyloxy; ($C_2$-$C_6$)alkenyl; ($C_2$-$C_6$)alkynyl; amino (-NZZ'); thiocyanato; ($C_1$-$C_4$)alkylthio; -CSR; -CSH; unsubstituted or substituted phenyl having

one to two of the same or different halo, nitro, $(C_1\text{-}C_4)$alkyl, $(C_1\text{-}C_4)$alkoxy, carboxy, -NZZ'; phenoxy where the phenyl ring is unsubstituted or substituted with one or two of the same or different halo, nitro, $(C_1\text{-}C_4)$alkyl, $(C_1\text{-}C_4)$alkoxy, carboxy, -NZZ'; or when two adjacent positions on the phenyl ring are substituted with alkoxy groups, these groups may be joined to form, together with the carbon atoms to which they are attached, a 5- or 6-membered dioxolano or dioxano heterocyclic ring;

unsubstituted or substituted $(C_1\text{-}C_8)$alkyl having one to three of the same or different halo, cyano, $(C_1\text{-}C_4)$alkoxy, phenyl, $(C_1\text{-}C_4)$alkoxy-carbonyl or halo$(C_1\text{-}C_4)$alkoxy;

unsubstituted or substituted $(C_3\text{-}C_6)$cyclo-alkyl or unsubstituted or substituted $(C_3\text{-}C_5)$cycloalkyl$(C_1\text{-}C_4)$alkyl having one or two of the same or different halo, $(C_1\text{-}C_4)$-alkyl, halo-$(C_1\text{-}C_4)$alkyl, $(C_1\text{-}C_4)$-alkoxy, $(C_1\text{-}C_4)$alkanoyl or halo-$(C_1\text{-}C_4)$alkoxy;
unsubstituted or substituted $(C_2\text{-}C_6)$alkenyl having as substituent(s) a furyl or one to three of the same or different $(C_1\text{-}C_4)$-alkyl, halo$(C_1\text{-}C_4)$alkyl, $(C_1\text{-}C_4)$alkoxy or halo-$(C_1\text{-}C_4)$alkoxy;

unsubstituted or substituted $(C_3\text{-}C_6)$cyclo-alkenyl having as substituents one or two of the same or different halo, $(C_1\text{-}C_4)$-alkyl, halo-$(C_1\text{-}C_4)$alkyl, $(C_1\text{-}C_4)$alkoxy or halo-$(C_1\text{-}C_4)$alkoxy;

unsubstituted or phenyl substituted alkynyl;

phenalkyl having one or two carbon atoms in the alkyl group and the alkyl group is unsubstituted or substituted with one or two of the same or different halo, $(C_1\text{-}C_4)$-alkyl, halo-$(C_1\text{-}C_4)$alkyl, $(C_1\text{-}C_4)$-alkoxy or halo-$(C_1\text{-}C_4)$alkoxy and the phenyl ring is unsubstituted or substituted with one or two of the same or different halo, cyano, $(C_1\text{-}C_4)$alkyl, halo$(C_1\text{-}C_4)$alkyl, $(C_1\text{-}C_4)$-alkoxy or halo-$(C_1\text{-}C_4)$-alkoxy;

phenalkenyl having two or three carbon atoms in the alkenyl group and the alkenyl group is unsubstituted or substituted with halo, $(C_1\text{-}C_4)$alkyl, halo$(C_1\text{-}C_4)$alkyl, $(C_1\text{-}C_4)$alkoxy or halo-$(C_1\text{-}C_4)$-alkoxy and the phenyl ring is unsubstituted or substituted with one or two of the same or different halo, cyano, $(C_1\text{-}C_4)$alkyl, halo-$(C_1\text{-}C_4)$-alkyl, $(C_1\text{-}C_4)$alkoxy or halo$(C_1\text{-}C_4)$alkoxy;

unsubstituted or substituted five-membered heterocycle selected from furyl, thienyl, triazolyl, pyrrolyl, and oxazolyl where the substituents can be one or two of the same or different halo; nitro; $(C_1\text{-}C_4)$alkyl; $(C_1\text{-}C_4)$alkoxy; -NZZ'; or unsubstituted or substituted phenyl having one or two of the same or different halo, nitro, $(C_1\text{-}C_4)$-alkyl, halo-$(C_1\text{-}C_4)$alkyl, $(C_1\text{-}C_4)$alkoxy, halo-$(C_1\text{-}C_4)$alkoxy, carboxy or -NZZ'; or

unsubstituted or substituted six-membered heterocycle having one or two nitrogen atoms and 4 to 5 nuclear carbon atoms where the substituents can be one or two of the same or different halo; nitro; $(C_1\text{-}C_4)$-alkyl; $(C_1\text{-}C_4)$alkoxy; thio-$(C_1\text{-}C_4)$alkoxy; -NZZ'; or unsubstituted or substituted phenyl having one or two of the same or different halo, nitro, $(C_1\text{-}C_4)$alkyl, halo$(C_1\text{-}C_4)$alkyl, $(C_1\text{-}C_4)$alkoxy, halo-$(C_1\text{-}C_4)$-alkoxy, carboxy or -NZZ';

where R and R' are $(C_1\text{-}C_4)$alkyl; Z and Z' are hydrogen or $(C_1\text{-}C_4)$alkyl.

**3.** The use according to claim 2 wherein, in the compound,
X and X' are O or S;
$R^1$ is hydrogen; $(C_1\text{-}C_4)$alkyl; $(C_1\text{-}C_4)$alkoxy-$(C_1\text{-}C_4)$alkyl having independently the stated number of carbon atoms in each alkyl group; $(C_2\text{-}C_5)$alkenyl; $(C_2\text{-}C_5)$alkynyl; or benzyl where the phenyl ring is unsubstituted or substituted with one halo, nitro, $(C_1\text{-}C_4)$alkyl or $(C_1\text{-}C_4)$alkoxy;
$R^2$ and $R^3$ are the same or different hydrogen or $(C_1\text{-}C_2)$alkyl;
$R^4$ is $(C_1\text{-}C_3)$alkyl substituted with 1 to 4 fluoro; straight chain $(C_2\text{-}C_4)$alkenyl; methoxycarbonyl; cyano; cyano substituted $(C_1\text{-}C_2)$alkyl; tri$(C_1\text{-}C_2)$alkylsilyl having independently the stated number of carbon atoms in each alkyl group; or tri$(C_1\text{-}C_2)$-alkylsilylmethyl having independently the stated number of carbon atoms in each alkyl group; provided that $R^2$ and $R^3$ are both alkyl when $R^4$ is methoxycarbonyl; and
A and B are the same or different
unsubstituted naphthyl;

unsubstituted or substituted phenyl having one to three of the same or different halo; nitro; cyano; $(C_1-C_4)$alkyl; halo-$(C_1-C_4)$-alkyl; cyano-$(C_1-C_4)$alkyl; $(C_1-C_4)$alkoxy; $(C_1-C_4)$alkoxy-$(C_1-C_4)$alkyl having independently the stated number of carbon atoms in each alkyl group; -COZ; $(C_1-C_4)$-alkoxy-carbonyl; $(C_1-C_4)$alkanoyloxy; thiocyanato; -NZZ'; $(C_1-C_4)$alkylthio; -CSZ; $-CS_2Z$; unsubstituted or substituted phenyl having one or two of the same or different halo, nitro, $(C_1-C_4)$alkyl, $(C_1-C_4)$alkoxy, carboxy, -NZZ'; or phenoxy where the phenyl ring is unsubstituted or substituted with one or two of the same or different halo, nitro, $(C_1-C_4)$alkyl, $(C_1-C_4)$alkoxy, carboxy, -NZZ' or when two adjacent positions on the phenyl ring are substituted with alkoxy groups, these groups may be joined to form, together with the carbon atoms to which they are attached, a 5- or 6-membered dioxolano or dioxano heterocyclic ring;

unsubstituted or substituted $(C_1-C_6)$alkyl having one to three of the same or different halo, cyano, nitro, carboxy, $(C_1-C_4)$alkoxy, phenyl, $(C_1-C_4)$-alkoxy-carbonyl or halo$(C_1-C_4)$alkoxy;

unsubstituted or substituted $(C_3-C_6)$cyclo-alkyl or unsubstituted or substituted $(C_3-C_6)$cycloalkyl$(C_1-C_4)$alkyl, where the substituent is halo, $(C_1-C_4)$alkyl, $(C_1-C_4)$alkanoyl, $(C_1-C_4)$alkoxy or halo$(C_1-C_4)$-alkoxy;

unsubstituted or substituted $(C_2-C_6)$-alkenyl having one or two of the same or different halo, $(C_1-C_4)$-alkyl, $(C_1-C_4)$alkoxy or halo$(C_1-C_4)$alkoxy;

unsubstituted or substituted $(C_4-C_6)$cyclo-alkenyl where the substituent is halo $(C_1-C_4)$alkyl, $(C_1-C_4)$-alkoxy or halo-$(C_1-C_4)$alkoxy;

unsubstituted or phenyl substituted alkynyl;

benzyl where the phenyl ring is unsubstituted or substituted with one or two of the same or different halo, methyl or ethyl;

unsubstituted or substituted five-membered heterocycle selected from furyl, thienyl, pyrrolyl and oxazolyl where the substituents can be one or two of the same or different halo, nitro, $(C_1-C_4)$alkyl or $(C_1-C_4)$alkoxy; or

unsubstituted or substituted six-membered heterocycle having one or two nitrogen atoms and four to five nuclear carbon atoms where the substituents can be one or two of the same or different halo, nitro, $(C_1-C_4)$-alkyl, $(C_1-C_4)$alkoxy or thio-$(C_1-C_4)$alkoxy;

where Z and Z' are hydrogen or $(C_1-C_4)$alkyl.

**4.** The use according to claim 3 wherein, in the compound,
X and X' are 0;
$R^1$ is hydrogen; methyl; ethyl; $(C_1-C_2)$alkoxy-$(C_1-C_2)$alkyl having independently the stated number of carbon atoms in each alkyl group $(C_2-C_5)$alkenyl; $(C_2-C_5)$alkynyl; or benzyl where the phenyl ring is unsubstituted or substituted with halo;
$R^2$ and $R^3$ are the same or different hydrogen or $(C_1-C_2)$alkyl;
$R^4$ is $(C_1-C_2)$alkyl substituted with 1 to 4 fluoro; straight chain $(C_2-C_4)$alkenyl; cyano; cyano substituted $(C_1-C_2)$alkyl; tri$(C_1-C_2)$alkylsilyl having independently the stated number of carbon atoms in each alkyl group; or tri$(C_1-C_2)$alkylsilylmethyl having independently the stated number of carbon atoms in each alkyl group; and
A and B are the same or different phenyl or substituted phenyl where the substituents can be from one to three of the same or different halo, nitro, $(C_1-C_4)$alkyl, $(C_1-C_4)$alkoxy or halo$(C_1-C_4)$alkyl;

unsubstituted or substituted $(C_1-C_6)$alkyl having as substituent(s) one to two of the same or different halo, phenyl or cyano;

unsubstituted $(C_4-C_6)$cycloalkyl;

unsubstituted or substituted ($C_2$-$C_6$)alkenyl having as substituent(s) one to three of the same or different halo or ($C_1$-$C_4$)alkyl;

unsubstituted ($C_4$-$C_6$)cycloalkenyl;

phenalkyl having one or two carbon atoms in the alkyl group and the phenyl ring is unsubstituted or substituted with one or two of the same or different halo, ($C_1$-$C_4$)-alkyl or ($C_1$-$C_4$)alkoxy;

unsubstituted or substituted pyridyl or 2,5-pyrazinyl where the substituent can be halo, nitro, ($C_1$-$C_4$)-alkyl, ($C_1$-$C_4$)alkoxy or ($C_1$-$C_4$)thioalkoxy; or

unsubstituted furyl or thienyl or an unsubstituted or substituted pyrrolyl where the substituent can be ($C_1$-$C_4$)alkyl.

**5.** The use according to claim 4 wherein, in the compound,
X and X' are 0;
$R^1$ is hydrogen; methyl; methoxymethyl; ($C_2$-$C_4$)-alkenyl; ($C_2$-$C_5$)alkynyl; benzyl; or 4-halobenzyl;
$R^2$ and $R^3$ are the same or different hydrogen, methyl or ethyl;
$R^4$ is trifluoromethyl; 2,2,2-trifluoroethyl; straight chain ($C_2$-$C_4$)alkenyl; cyano; cyanomethyl; tri($C_1$-$C_2$)alkylsilyl having independently the stated number of carbon atoms in each alkyl group; or trimethylsilylmethyl; and
A and B are the same or different phenyl or substituted phenyl where the substituents can be one, two or three of the same or different chloro, fluoro, bromo, iodo, nitro, methyl, ethyl, methoxy or trifluoromethyl;

($C_2$-$C_3$) substituted alkenyl having one to three of the same or different chloro, bromo, methyl or ethyl;

cyclohexenyl;

benzyl;

unsubstituted or substituted pyridyl or 2,5-pyrazinyl where the substituent can be halo, methyl, ethyl or methoxy; or

unsubstituted furyl or thienyl or an unsubstituted or substituted pyrrolyl where the substituent can be ethyl or methyl.

**6.** The use according to claim 2 wherein, in the compound,
X and Y' are 0;
$R^1$ is hydrogen or methyl;
$R^2$ and $R^3$ are hydrogen or ($C_1$-$C_3$)alkyl;
$R^4$ is trifluoromethyl, 2,2,2-trifluoroethyl, cyano, cyanomethyl, straight chain ($C_2$-$C_4$)-alkenyl, carboxyl, methoxycarbonyl, tri($C_1$-$C_2$)alkylsilyl having independently the stated number of carbon atoms in each alkyl group or tri($C_1$-$C_2$)alkylsilylmethyl having independently the stated number of carbon atoms in each alkyl group; and
A and B are the same or different thienyl or unsubstituted phenyl or substituted phenyl where the substituents can be one to three of the same or different chloro, fluoro, nitro, methyl, ethyl or propyl.

**7.** The use according to claim 6 wherein, in the compound,
X and X' are 0;
$R^1$ is hydrogen;
$R^2$ and $R^3$ are hygrogen, meyhyl or ethyl;
$R^4$ is trifluoromethyl, cyano, allyl, methoxycarbonyl, or trimethylsilyl; and
A and B are the same or different unsubstituted or substituted phenyl having one or two of the same or different chloro, fluoro, nitro, methyl and ethyl; or unsubstituted thienyl.

**8.** The use according to claim 7 wherein, in the compound,

X and X' are O;
$R^1$ is hydrogen; and
$R^2$ is hydrogen, $R^3$ is methyl, $R^4$ is trifluoromethyl, and A and B are the same or different unsubstituted or substituted phenyl where the substituents can be one or two of the same or different chloro, methyl or nitro; or
$R^2$ and $R^3$ are the same or different methyl or ethyl, $R^4$ is cyano, and A and B are the same or different unsubstituted or substituted phenyl where the substituents can be one or two of the same or different chloro, fluoro, methyl, ethyl or nitro; or
$R^2$ and $R^3$ are methyl, $R^4$ is allyl, and A and B are the same or different unsubstituted or methyl substituted phenyl; or
$R^2$ and $R^3$ are hydrogen, $R^4$ is trimethylsilyl, and A and B are the same or different substituted phenyl where the substituents can be one or two of the same or different methyl, ethyl or nitro.

**9.** The use according to claim 8 wherein, in the compound,
X and X' are 0;
$R^1$ is hydrogen; and
$R^2$ is hydrogen, $R^3$ is methyl, $R^4$ is trifluoromethyl, and A and B are unsubstituted phenyl, or A is 2,3-dimethylphenyl and B is 2,4-dichlorophenyl, 3,5-dimethylphenyl or 2-nitro-5-methylphenyl; or
$R^2$ and $R^3$ are methyl, $R^4$ is allyl, and A and B are both unsubstituted phenyl or 3-methyl-phenyl; or
$R^2$ and $R^3$ are hydrogen, $R^4$ is trimethylsilyl, and A is 4-ethylphenyl and B is 2-nitro-5-methylphenyl, or A and B are both 2-methyl-phenyl or 2-nitrophenyl.

**10.** The use according to claim 8 wherein, in the compound,
X and X' are O;
$R^1$ is hydrogen;
$R^2$ and $R^3$ are methyl;
$R^4$ is cyano; and
A and B are the same or different unsubstituted or substituted phenyl where the substituents can be one or two of the same or different chloro, fluoro, methyl, ethyl or nitro.

**11.** The use according to any preceding claim of the compound to make a composition containing said insecticidal compound in an amount of from 0.0001 to 99% by weight of the composition.

**12.** The use according to claim 11 of the compound to make a composition wherein the amount of said insecticidal compound is from 0.01 to 99% by weight of the composition.

**13.** The use of a compound as defined in any one of claims 1 to 10 optionally in a composition with agronomically acceptable diluent or carrier for controlling insects by contacting said insects with an insecticidally effective amount of the compound or composition.

**14.** A mechanical process for improving the commercial value and/or profitability of vendible crops from plants whose growth is affected or likely to be affected by insects comprising (1) charging to a container, fumigation device or mechanical dissemination device an insecticidal compound or salt as defined in any of claims 1 to 10, optionally in a mixture with agronomically acceptable diluent or carrier, (2) using the container, fumigator or mechanical dissemination device to apply the insecticidal compound or salt, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

**15.** The use or process according to claim 13 or 14 wherein said compound or composition is applied to insects or to growing plants or an area in which plants are to be grown at from 10 grams to 10 kilograms per hectare of the compound.

**16.** The use or process according to claim 13, 14 or 15 wherein the application rate is from 100 grams to 5 kilograms per hectare of the compound.

17. The use or process according to any of claims 13 to 16 wherein said insects are from the order of Lepidoptera or Coleoptera.

## Revendications

1. Composé à activité insecticide, qui est une hydrazine substituée répondant à la formule :

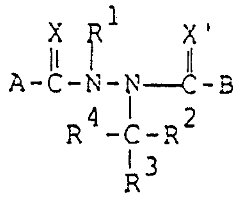

I

dans laquelle :

X et X', identiques ou différents, représentent chacun O, S ou NR ;

$R^1$ représente un atome d'hydrogène ; un groupe alkyle en $C_1$ à $C_6$ ; alcoxy (en $C_1$ à $C_6$)-alkyle (en $C_1$ à $C_6$) ayant, indépendamment, le nombre indiqué des atomes de carbone dans chaque groupe alkyle ; alkyl (en $C_1$ à $C_6$)-thio-alkyle (en $C_1$ à $C_6$) ayant, indépendamment, le nombre indiqué d'atomes de carbone dans chaque groupe alkyle ; alcényle en $C_2$ à $C_6$ ; alcynyle en $C_2$ à $C_6$ ; ou phényle-alkyle (en $C_1$ à $C_4$) dans lequel le noyau phényle n'est pas substitué ou est substitué par un à trois substituants, identiques ou différents, qui sont chacun un reste halogéno, cyano, nitro, hydroxy, alkyle en $C_1$ à $C_4$, halogéno-alkyle (en $C_1$ à $C_4$) alcoxy (en $C_1$ à $C_4$), halogéno-alcoxy en $C_1$ à $C_4$, carboxy, alcoxy (en $C_1$ à $C_4$)-carbonyle, alcanoyloxy en $C_1$ à $C_4$ ou NZZ' ;

$R^2$ et $R^3$, identiques ou différents, représentent chacun un atome d'hydrogène ou un groupe alkyle en $C_1$ à $C_4$ ;

$R^4$ représente un groupe alkyle en $C_1$ à $C_4$, substitué par un à quatre atomes de fluor ; un groupe alcényle (en $C_2$ à $C_4$) linéaire ; un groupe carboxyle ; alcoxy (en $C_1$ à $C_3$)-carbonyle ; cyano ; alkyle (en $C_1$ à $C_4$) à substituant(s) cyano ; un groupe trialkyl (en $C_1$ à $C_4$) silyle, dont chaque groupe alkyle présente, indépendamment, le nombre indiqué des atomes de carbone ; ou un groupe trialkyl (en $C_1$ à $C_2$) silylméthyle dont chaque groupe alkyle présente, indépendamment, le nombre indiqué des atomes de carbone ; à la condition que $R^2$ et $R^3$ représentent chacun un groupe alkyle lorsque $R^4$ représente un groupe carboxyle ou alcoxycarbonyle ; et

A et B, identiques ou différents, représentent chacun : un groupe naphtyle, substitué ou non substitué, dans lequel les substituants peuvent être constitués par un à trois des restes suivants, identiques ou différents : un reste halogéno, nitro, alcoxy en $C_1$ à $C_4$, alkyle en $C_1$ à $C_4$ ou NZZ' ;

un groupe phényle, substitué ou non substitué, dans lequel les substituants peuvent être constitués par un à cinq des restes suivants, identiques ou différents : un reste halogéno ; nitroso ; nitro ; cyano ; hydroxy ; alkyle en $C_1$ à $C_6$ ; halogénoalkyle en $C_1$ à $C_6$ ; cyanoalkyle en $C_1$ à $C_6$ ; hydroxyalkyle en $C_1$ à $C_6$ ; alcoxy en $C_1$ à $C_6$ ; halogéno-alcoxy en $C_1$ à $C_6$ ; alcoxy (en $C_1$ à $C_6$)-alkyle (en $C_1$ à $C_6$) ayant indépendamment le nombre indiqué d'atomes de carbone dans chaque groupe alkyle ; alcoxy (en $C_1$ à $C_6$)-alcoxy (en $C_1$ à $C_6$) ayant indépendamment le nombre indiqué d'atomes de carbone dans chaque groupe alkyle ; un groupe -ORSR' ; un groupe $-OCO_2R$ ; un groupe $-OCO_2H$ ; un groupe alcanoyl (en $C_1$ à $C_6$)-oxy-alkyle en $C_1$ à $C_6$ ayant indépendamment le nombre indiqué d'atomes de carbone dans chaque groupe alkyle ;

un groupe alcényle en $C_2$ à $C_6$, éventuellement substitue par un groupe halogéno, cyano, alkyle en $C_1$ à $C_4$, halogénoalcoxy en $C_1$ à $C_4$, alkylthio en $C_1$ à $C_4$ ou alcoxy en $C_1$ à $C_4$ ; alcadiényle en $C_3$ à $C_5$ ; alcényloxy en $C_2$ à $C_6$ ; alcényl (en $C_2$ à $C_6$)-carbonyle ; alcényloxy (en $C_2$ à $C_6$)-carbonyloxy ; alcynyle en $C_2$ à $C_6$ (éventuellement substitué par un atome d'halogène, un groupe cyano, nitro, hydroxy, alcoxy en $C_1$ à $C_4$, halogéno-alkyle en $C_1$ à $C_4$, alkylthio en $C_1$ à $C_4$ ou alkyle en $C_1$ à $C_4$) ; un groupe carboxy ; un groupe $-RCO_2R'$ ; un groupe -COR ; -COH ; halogéno-alkyl (en $C_1$ à $C_6$)-carbonyle ; un groupe $-CO_2R$ ; halogéno-alcoxy (en $C_1$ à $C_6$) carbonyle ; un groupe -OCOR ; un groupe $-ORCO_2R'$ ; un groupe amino (-NZZ') ; un groupe amino (substitué par un reste hydroxy,

alcoxy en $C_1$ à $C_4$ ou alkylthio en $C_1$ à $C_4$) ; un groupe -CONZZ' ; un groupe alcényl (en $C_2$ à $C_6$)-carbonylamino ; hydroxy-alkylamino (en $C_1$ à $C_6$)-carbonyle ; un groupe -OCONZZ'; un groupe -NZCOZ' ; un groupe -$NZCO_2Z'$ ; un groupe thiocyanato ; isothiocyanato ; thiocyanatoalkyle (en $C_1$ à $C_6$) ; alkylthio (en $C_1$ à $C_6$) ; halogéno-alkylthio (en $C_1$ à $C_6$) ; un groupe -S(O)Z ; un groupe -$SO_2Z$; un groupe -$OSO_2R$ ; un groupe -$OSO_2H$ ; un groupe -$SO_2NZZ'$ ; un groupe -CSR ; un groupe -CSH ; un groupe -SCOR ; un groupe -SCOH ; un groupe -NSCSZ' ; un groupe phényle, non substitué ou bien substitué comportant un à trois des restes suivants, identiques ou différents : un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe phénoxy dans lequel le noyau phényle n'est pas substitué ou est substitué par un à trois restes, identiques ou différents, qui sont des restes halogéno, cyano, nitro, hydroxy, alkyle en $C_1$ à $C_4$, halogéno-alkyle en $C_1$ à $C_4$, alcoxy (en $C_1$ à $C_4$), carboxy, alkyl (en $C_1$ à $C_4$)-carbonyle, alcanoyloxy en $C_1$ à $C_4$, amino, alkyl (en$C_1$ à $C_4$)-amino ou dialkyl (en $C_1$ à $C_4$)-amino ayant, indépendamment, le nombre indiqué d'atomes de carbone dans chaque groupe alkyle ; un groupe benzoyle dont le noyau phényle n'est pas substitué ou est substitué par un à trois substituants, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; benzoyloxy-alkyle (en $C_1$ à $C_6$) ; phénylthio-alkyle (en $C_1$ à $C_6$), dont le noyau phényle n'est pas substitué ou est substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe -CR=N-R''', dans lequel R''' représente un groupe hydroxy, alkyle en $C_1$ à $C_4$, alcoxy en $C_1$ à $C_4$, amino (-NZZ'), phénylamino, -COR, -COH ou benzoyle ; un groupe oxirannyle en $C_2$ à $C_6$ ; acétylthiosemi-carbazone ; pyrrolyle ; oxazolyle, non substitué ou substitué par un ou deux groupes méthyles ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_{10}$, non substitué ou substitué comportant alors un à quatre substituants identiques ou différents, qui sont chacun un reste halogéno, cyano, nitro, hydroxy, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou -NZZ' ;

un groupe cycloalkyle en $C_3$ à $C_8$, non substitué ou substitué, ou un groupe cyclo (en $C_3$ à $C_8$)-alkyle (en $C_1$ à $C_4$), non substitué ou substitué comportant un à quatre substituants, identiques ou différents, qui sont chacun un reste halogéno, cyano, nitro, hydroxy, alkyle en $C_1$ à $C_4$, halogénoalkyle en $C_1$ à $C_4$, alcoxy en $C_1$ à $C_4$, halogéno-alcoxy en $C_1$ à $C_4$, carboxy, alcanoyle en $C_1$ à $C_4$, alcoxy (en $C_1$ à $C_4$)-carbonyle, alcanoyloxy en $C_1$ à $C_4$ ou -NZZ' ;

un groupe alcényle en $C_2$ à $C_8$, non substitué ou substitué ou un groupe alcadiényle en $C_3$ à $C_5$, non substitué ou substitué et ayant comme substituant(s) un groupe furyle, thiényle ou pyridyle ou bien ayant un à quatre substituants, identiques ou différents, qui sont chacun un reste halogéno, cyano, nitro, hydroxy, alkyle en $C_1$ à $C_4$, cycloalkyle en $C_3$ à $C_6$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$ ou -NZZ' ;

un groupe cycloalcényle en $C_3$ à $C_8$, non substitué ou substitué, ou un groupe cycloalcadiényle (en $C_8$), non substitué ou substitué et ayant alors comme substituant(s) un à quatre restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$ ou -NZZ' ;

un groupe alcynyle en $C_2$ à $C_8$, non substitué ou substitué et ayant alors comme substituant(s) un à

quatre restes, identiques ou différents, qui sont chacun un reste halogeno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou NZZ' ;

un groupe phénylalkyle comportant 1 à 4 atomes de carbone dans le groupe alkyle et dans lequel le groupe alkyle n'est pas substitué ou est substitué par un à trois restes, identiques ou différents, qui sont chacun un reste halogéno, cyano, hydroxy, alcoxy en $C_1$ à $C_4$, alcoxy (en $C_1$ à $C_4$)-carbonyle ou -NZZ', et le noyau phényle n'est pas substitué ou est substitué par un à trois restes, identiques ou différents, qui sont chacun un reste halogéno, cyano, nitro, hydroxy, alkyle en $C_1$ à $C_4$, halogénoalkyle en $C_1$ à $C_4$, cyano-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, alcényle en $C_2$ à $C_6$, halogéno-alcényle en $C_2$ à $C_6$, alcynyle en $C_2$ à $C_6$ ou -NZZ' ; un groupe phénylalcényle ayant deux à six atomes de carbone dans le groupe alcényle, ce groupe alcényle n'étant pas substitué ou étant substitué par un à trois restes, identiques ou différents, qui sont chacun un reste halogéno, cyano, hydroxy, alkyle en $C_1$ à $C_4$, halogénoalkyle en $C_1$ à $C_4$, alcoxy (en $C_1$ à $C_4$), halogéno-alcoxy en $C_1$ à $C_4$, alcoxy (en $C_1$ à $C_4$)-carbonyle ou -NZZ', et le noyau phényle n'étant pas substitué ou étant substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogénoalcoxy en $C_1$ à $C_4$, carboxy, alcoxy (en $C_1$ à $C_4$)-carbonyle, alcanoyloxy en $C_1$ à $C_4$ ou -NZZ' ; un hétérocycle pentagonal, non substitué ou substitué, qui est choisi parmi un groupe furyle, thiényle, triazolyle, pyrrolyle, isopyrrolyle, pyrazolyle, isoimidazolyle, thiazolyle, isothiazolyle, oxazolyle et isooxazolyle, les substituants pouvant être un à trois radicaux identiques ou différents qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle ; un groupe $-RCO_2H$ ; un groupe $RCO_2R'$ ; un groupe -CONZZ' ; un groupe amino (-NZZ') ; un groupe -NZCOZ' ; un groupe alkylthio en $C_1$ à $C_6$; ou un groupe phényle, non substitué ou substitué comportant un à trois restes, identiques ou différents, qui sont chacun un reste halogéno, nitro, alkyle en $C_1$ à $C_6$, halogénoalkyle en $C_1$ à $C_6$, alcoxy en $C_1$ à $C_6$, halogéno-alcoxy en $C_1$ à $C_6$, carboxy, alcoxy (en $C_1$ à $C_4$)-carbonyle ou amino (-NZZ') ; ou bien

un hétérocycle hexagonal, non substitué ou substitué, comportant un, deux, trois ou quatre atomes d'azote et deux à cinq atomes de carbone de noyau, dans lequel les substituants peuvent être constitués par un à trois restes, identiques ou différents, qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle, un groupe $-RCO_2H$ ; un groupe $-RCO_2R'$ ; un groupe -CONZZ' ; un groupe amino (NZZ') ; un groupe -NZCOZ' ; un groupe alkylthio en $C_1$ à $C_6$ ; ou bien un groupe phényle non substitué ou substitué, comportant un à trois restes identiques ou différents qui sont chacun un reste halogéno, nitro, alkyle en $C_1$ à $C_6$, halogéno-alkyle en $C_1$ à $C_6$, alcoxy en $C_1$ à $C_6$, halogéno-alcoxy (en $C_1$ à $C_6$), carboxy, alcoxy (en $C_1$ à $C_4$)-carbonyle ou amino (-NZZ') ;

les symboles R et R' représentant chacun un groupe alkyle en $C_1$ à $C_6$ ; Z et Z' représentant chacun un atome d'hydrogène ou un groupe alkyle en $C_1$ à $C_4$ ; le terme "amino" désignant un groupe NZZ' ; ou un sel agronomiquement acceptable de ce composé.

**2.** Composé selon la revendication 1, dans lequel
X et X' représentent O ou S ;
$R^1$ représente un atome d'hydrogène ; un groupe alkyle en $C_1$ à $C_4$, alcoxy (en $C_1$ à $C_4$)-alkyle (en $C_1$ à $C_4$), ayant, indépendamment, le nombre indiqué d'atomes de carbone dans chaque groupe alkyle ; un groupe méthylthiométhyle ; alcényle en $C_2$ à $C_5$ ; alcynyle en $C_2$ à $C_5$ ; ou phényl-alkyle en $C_1$ ou $C_2$, dans lequel le noyau phényle n'est pas substitué ou est substitué par un ou deux restes halogéno, nitro, alkyle en $C_1$ à $C_4$ ou alcoxy en $C_1$ à $C_4$ ;
$R^2$ et $R^3$ sont identiques ou différents et représentent chacun un atome d'hydrogène ou un groupe alkyle en $C_1$ à $C_3$ ;
$R^4$ représente un groupe alkyle en $C_1$ à $C_3$, substitué par un à quatre atomes de fluor ; un groupe alcényle linéaire en $C_2$ à $C_4$ ; carboxyle ; alcoxy (en $C_1$ ou $C_2$)-carbonyle ; cyano ; alkyle (en $C_1$ à $C_3$) substitué par un groupe cyano ; trialkyl (en $C_1$ à $C_2$)-silyle ayant, indépendamment, le nombre indique d'atomes de carbone dans chaque groupe alkyle ; ou trialkyl (en $C_1$ ou $C_2$)-silylméthyle ayant, indépendamment, le nombre indiqué d'atomes de carbone dans chaque groupe alkyle ; et
A et B, identiques ou différents, representent chacun :

un groupe naphtyle non substitué ;
un groupe phényle, non substitué ou bien substitué et dans lequel les substituants peuvent être constitués par un à trois restes différents ou identiques qui sont chacun un reste halogéno ; nitro ; cyano ; alkyle en $C_1$ à $C_4$ ; halogéno-alkyle en $C_1$ à $C_4$ ; cyano-alkyle en $C_1$ à $C_4$ ; alcoxy en $C_1$ à $C_4$ ; alcoxy (en $C_1$ à $C_4$) alkyle (en $C_1$ à $C_4$) ayant, indépendamment, le nombre indiqué d'atomes de carbone dans chaque groupe alkyle ; -COZ ; carboxy ; alcoxy (en $C_1$ à $C_4$)-carbonyle ; alcanoyloxy en $C_1$ à $C_4$ ; alcényle en $C_2$ à $C_6$ ; alcynyle en $C_2$ à $C_6$ ; amino (-NZZ') ; thiocyanato ; alkylthio en $C_1$ à $C_4$ ; -CSR ; -CSH ; un groupe phényle non substitué, ou substitué ayant un ou deux restes, identiques ou différents qui sont chacun un reste halogéno, nitro, alkyle en $C_1$ à $C_4$, alcoxy en $C_1$ à $C_4$, carboxy, -NZZ' ; un groupe phénoxy dans lequel le noyau phényle n'est pas substitué ou est substitué par un ou deux restes, identiques ou différents, qui sont chacun un reste halogéno, nitro, alkyle en $C_1$ à $C_4$, alcoxy en $C_1$ à $C_4$, carboxy, -NZZ' ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_8$, non substitué ou substitué, comportant un à trois restes identiques ou différents qui sont chacun un reste halogéno, cyano, alcoxy en $C_1$ à $C_4$, phényle, alcoxy (en $C_1$ à $C_4$)-carbonyle ou halogénoalcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, comportant un ou deux substituants identiques ou différents qui sont chacun un radical halogéno, alkyle en $C_1$ à $C_4$, halogéno-alkyle en $C_1$ à $C_4$, alcoxy en $C_1$ à $C_4$, alcanoyle en $C_1$ à $C_4$ ou halogéno-alcoxy en $C_1$ à $C_4$ ; un groupe alcényle en $C_2$ à $C_6$, non substitué ou substitué, comportant comme substituant(s) un reste furyle ou un à trois restes, identiques ou différents, qui sont chacun un reste alkyle en $C_1$ à $C_4$, halogéno-alkyle en $C_1$ à $C_4$, alcoxy en $C_1$ à $C_4$ ou halogénoalcoxy en $C_1$ à $C_4$ ; un groupe cycloalkényle (en $C_3$ à $C_6$) non-substitué ou substitué, ayant comme substituants un ou deux restes identiques ou différents qui sont halogéno, alkyle en $C_1$ à $C_4$, halogénoalkyle en $C_1$ à $C_4$, alcoxy en $C_1$ à $C_4$ ou halogénoalcoxy en $C_1$ à $C_4$ ;

un groupe alcynyle non substitué ou substitué par un groupe phényle ;

un groupe phénylalkyle ayant 1 ou 2 atomes de carbone dans le groupe alkyle, ce groupe alkyle n'étant pas substitué ou étant substitué par un ou deux restes, identiques ou différents, qui sont chacun un reste halogéno, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un groupe phénylalcényle ayant 2 ou 3 atomes de carbone dans le groupe alcényle, ce groupe alcényle n'étant pas substitué ou étant substitué par un reste halogé-no, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un hétérocycle pentagonal, non substitué ou substitué, choisi parmi un groupe furyle, thiényle, triazolyle, pyrrolyle et oxazolyle, les substituants pouvant être un ou deux restes identiques ou différents qui sont chacun un reste halogéno ; nitro ; alkyle en $C_1$ à $C_4$ ; alcoxy en $C_1$ à $C_4$ ; -NZZ' ; ou bien un groupe phényle, non substitué ou substitué comportant un ou deux restes identiques ou différents qui sont chacun un reste halogeno, nitro, alkyle en $C_1$ à $C_4$, halogéno-alkyle en $C_1$ à $C_4$, alcoxy en $C_1$ à $C_4$, halogeno-alcoxy en $C_1$ à $C_4$, carboxy ou -NZZ' ; ou
un hetérocycle hexagonal non substitué ou substitué, comportant un ou deux atomes d'azote et quatre à cinq atomes de carbone de noyau, les substituants pouvant être constitués par un ou deux restes identiques ou différents qui sont chacun un reste halogéno ; nitro ; alkyle en $C_1$ à $C_4$ ; alcoxy en $C_1$ à $C_4$ ; thioalcoxy en $C_1$ à $C_4$ ; -NZZ' ; ou un groupe phényle non substitué ou substitué comportant un ou deux restes identiques ou différents qui sont chacun un reste halogeno, nitro,

alkyle en $C_1$ à $C_4$, halogéno-alkyle en $C_1$ à $C_4$, alcoxy en $C_1$ à $C_4$, halogéno-alcoxy en $C_1$ à $C_4$, carboxy ou -NZZ' ;

les symboles R et R' représentant chacun un groupe alkyle en $C_1$ à $C_4$ ; et Z et Z' représentant chacun un atome d'hydrogène ou un groupe alkyle en $C_1$ à $C_4$.

**3.** Composé selon la revendication 2, dans lequel

X et X' représentent O ou S ;

$R^1$ represente un atome d'hydrogène, un groupe alkyle en $C_1$ à $C_4$ ; alcoxy (en $C_1$ à $C_4$)-alkyle en $C_1$ à $C_4$ comportant, independamment, le nombre indiqué d'atomes de carbone dans chaque groupe alkyle ; alcényle en $C_2$ à $C_5$ ; alcynyle en $C_2$ à $C_5$ ; ou benzyle dans lequel le noyau phényle n'est pas substitué ou est substitué par un reste halogéno, nitro, alkyle en $C_1$ à $C_4$ ou alcoxy en $C_1$ à $C_4$ ;

$R^2$ et $R^3$ , identiques ou différents, représentent chacun un atome d'hydrogène ou un groupe alkyle en $C_1$ ou $C_2$ ;

$R^4$ représente un groupe alkyle en $C_1$ à $C_3$, substitué par un à quatre restes fluoro ; un groupe alcényle linéaire en $C_2$ à $C_4$ ; un groupe méthoxycarbonyle ; cyano ; alkyle en $C_1$ ou $C_2$ à substituant(s) cyano ; trialkyl (en $C_1$ ou $C_2$) silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; à la condition que $R^2$ et $R^3$ représentent chacun un groupe alkyle lorsque $R^4$ représente un groupe méthoxycarbonyle ; et

A et B sont identiques ou différents et représentent chacun :

un groupe naphtyle non substitué ;

un groupe phényle, non substitué ou substitué, comportant un à trois restes identiques ou différents, qui sont chacun un reste halogéno ; nitro ; cyano ; alkyle en $C_1$ à $C_4$ ; halogéno-alkyle en $C_1$ à $C_4$ ; cyano-alkyle en $C_1$ à $C_4$ ; alcoxy en $C_1$ à $C_4$ ; alcoxy (en $C_1$ à $C_4$)-alkyle (en $C_1$ à $C_4$) ayant, indépendamment, le nombre indiqué d'atomes de carbone dans chaque groupe alkyle ; -COZ ; alcoxy (en $C_1$ à $C_4$)-carbonyle ; alcanoyloxy (en $C_1$ à $C_4$) ; thiocyanato ; -NZZ' ; alkylthio en $C_1$ à $C_4$ ; -CSZ ; $-CS_2Z$ ; un groupe phényle non substitué ou substitué comportant un ou deux restes, identiques ou différents, qui sont chacun un reste halogéno, nitro, alkyle en $C_1$ à $C_4$, alcoxy en $C_1$ à $C_4$, carboxy, -NZZ' ; ou bien un groupe phénoxy dans lequel le noyau phényle n'est pas substitué ou est substitué par un ou deux restes identiques ou différents qui sont chacun un reste halogéno, nitro, alkyle en $C_1$ à $C_4$, alcoxy en $C_1$ à $C_4$, carboxy, -NZZ' ou bien, lorsque deux positions adjacentes du noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_6$, non substitué ou substitué et comportant alors un à trois restes identiques ou différents qui sont chacun un reste halogéno, cyano, nitro, carboxy, alcoxy en $C_1$ à $C_4$, phényle, alcoxy (en $C_1$ à $C_4$)-carbonyle ou halogéno-alcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, dans lesquels le substituant est un reste halogéno, alkyle en $C_1$ à $C_4$, alcanoyle en $C_1$ à $C_4$, alcoxy en $C_1$ à $C_4$ ou halogéno-alcoxy en $C_1$ à $C_4$ ;

un groupe alcényle en $C_2$ à $C_6$, non substitué ou substitué et comportant alors un ou deux restes, identiques ou diiférents, qui sont chacun un reste halogéno, alkyle en $C_1$ à $C_4$, alcoxy en $C_1$ à $C_4$ ou halogéno-alcoxy en $C_1$ à $C_4$ ;

un groupe cycloalcényle en $C_4$ à $C_6$, non substitué, ou substitué et dans lequel le substituant est un reste halogéno, alkyle en $C_1$ à $C_4$, alcoxy en $C_1$ à $C_4$ ou halogéno-alcoxy en $C_1$ à $C_4$ ;

un groupe alcynyle, non substitué ou substitué par un groupe phényle ;

un groupe benzyle, dans lequel le noyau phényle n'est pas substitué ou est substitué par un ou deux restes, identiques ou différents, qui sont chacun un reste halogéno, méthyle ou éthyle ;

un hétérocycle pentagonal, choisi parmi un groupe furyle, thienyle, pyrrolyle et oxazolyle, non substitué ou bien substitué et dans lequel les substituants peuvent être un ou deux restes, identiques ou différents, qui sont chacun un reste halogéno, nitro, alkyle en $C_1$ à $C_4$ ou alcoxy en $C_1$ à $C_4$ ; ou

un hétérocycle hexagonal comportant un ou deux atomes d'azote et quatre ou cinq atomes de carbone de noyau, l'hétérocycle n'étant pas substitué ou étant substitué, les substituants pouvant être un ou deux restes identiques ou différents qui sont chacun un reste halogéno, nitro, alkyle en $C_1$ à $C_4$, alcoxy en $C_1$ à $C_4$ ou thio-alcoxy en $C_1$ à $C_4$ ;

et Z et Z' représentent chacun un atome d'hydrogène ou un groupe alkyle en $C_1$ à $C_4$.

**4.** Composé selon la revendication 3, dans lequel
X et X' représentent O ;
$R^1$ représente un atome d'hydrogène ; un groupe méthyle, ethyle, alcoxy (en $C_1$ ou $C_2$)-alkyle (en $C_1$ ou $C_2$) ayant, indépendamment, le nombre indiqué d'atomes de carbone dans chaque groupe alkyle ; alcényle en $C_2$ à $C_5$ ; alcynyle en $C_2$ à $C_5$ ; ou benzyle, dans lequel le noyau phényle n'est pas substitué ou est substitué par un reste halogéno ;
$R^2$ et $R^3$, identiques ou différents, représentent chacun un atome d'hydrogène ou un groupe alkyle en $C_1$ ou $C_2$ ;
$R^4$ représente un groupe alkyle en $C_1$ ou $C_2$, substitué par un à quatre atomes de fluor ; un groupe alcényle linéaire en $C_2$ à $C_4$ ; cyano ; alkyle en $C_1$ ou $C_2$ à substituant(s) cyano ; trialkyl (en $C_1$ ou $C_2$)-silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; et
A et B sont identiques ou différents et représentent chacun un groupe phényle ou phényle substitué, dans lequel les substituants peuvent être constitués par un à trois restes, identiques ou différents, qui sont chacun un reste halogéno, nitro, alkyle en $C_1$ à $C_4$, alcoxy en $C_1$ à $C_4$ ou halogéno-alkyle en $C_1$ à $C_4$ ;

un groupe alkyle en $C_1$ à $C_6$, non substitué ou substitué, ayant comme substituant(s) un ou deux restes identiques ou différents qui sont chacun un reste halogéno, phényle ou cyano ;

un groupe cycloalkyle en $C_4$ à $C_6$, qui n'est pas substitué ;

un groupe alcényle en $C_2$ à $C_6$, non substitué ou substitué et ayant alors comme substituant(s) un à trois restes, identiques ou différents, qui sont chacun un reste halogéno ou alkyle en $C_1$ à $C_4$ ;

un groupe cycloalcényle en $C_4$ à $C_6$, non substitué ; un groupe phénylalkyle ayant un ou deux atomes de carbone dans le groupe alkyle et dont le noyau phényle n'est pas substitué ou est substitué par un ou deux restes identiques ou différents qui sont chacun un reste halogéno, alkyle en $C_1$ à $C_4$ ou alcoxy en $C_1$ à $C_4$ ;

un groupe pyridyle ou 2,5-pyrazinyle, non substitué ou substitué, dans lesquels le substituant peut être un reste halogéno, nitro, alkyle en $C_1$ à $C_4$, alcoxy en $C_1$ à $C_4$ ou thioalcoxy en $C_1$ à $C_4$ ; ou

un groupe furyle ou thiényle non substitué ou un groupe pyrrolyle non substitué ou substitué, dans lesquels le substituant peut être un groupe alkyle en $C_1$ à $C_4$.

**5.** Composé selon la revendication 4, dans lequel
X et X' représentent O ;
$R^1$ représente un atome d'hydrogène, un groupe méthyle ; méthoxyméthyle ; alcényle en $C_2$ à $C_4$ ; alcynyle en $C_2$ à $C_5$ ; benzyle ou 4-halogénobenzyle ;
$R^2$ et $R^3$ sont identiques ou différents et représentent chacun un atome d'hydrogène ou un groupe méthyle ou éthyle ;
$R^4$ représente un groupe trifluorométhyle ; 2,2,2-trifluoroéthyle ; alcényle linéaire en $C_2$ à $C_4$ ; cyano ; cyanométhyle ; trialkyl (en $C_1$ ou $C_2$) silyle ayant, indépendamment, le nombre indiqué d'atomes

de carbone dans chaque groupe alkyle ; ou triméthylsilylméthyle ; et
A et B, identiques ou différents, représentent chacun un groupe phényle ou un groupe phényle substitué dont les substituants peuvent être constitués par un, deux ou trois restes identiques ou différents qui sont chacun un reste chloro, fluoro, bromo, iodo, nitro, méthyle, éthyle, méthoxy ou trifluorométhyle ;

un groupe alcényle en $C_2$ ou $C_3$, substitué, comportant un à trois restes identiques ou différents qui sont chacun un reste chloro, bromo, méthyle ou éthyle ;

un groupe cyclohéxényle ;

un groupe benzyle ;

un groupe pyridyle ou 2,5-pyrazinyle, non substitués ou substitués dans lesquels le substituant peut être un reste halogéno, méthyle, éthyle ou méthoxy ; ou bien

un groupe furyle ou thiényle, non substitués ou un groupe pyrrolyle non substitué ou substitué dans lequel le substituant peut être un reste éthyle ou méthyle.

**6.** Composé selon la revendication 2, dans lequel
X et X' représentent chacun O ;
$R^1$ représente un atome d'hydrogène ou un groupe méthyle ;
$R^2$ et $R^3$ représentent chacun un atome d'hydrogène ou un groupe alkyle en $C_1$ à $C_3$ ;
$R^4$ représente un groupe trifluorométhyle, 2,2,2-trifluoroéthyle, cyano, cyanométhyle, alcényle linéaire en $C_2$ à $C_4$, carboxyle, méthoxycarbonyle, trialkyl (en $C_1$ ou $C_2$) silyle ayant, indépendamment, le nombre indiqué d'atomes de carbone dans chaque groupe alkyle ou trialkyl (en $C_1$ ou $C_2$) silylméthyle ayant, indépendamment, le nombre indiqué d'atomes de carbone dans chaque groupe alkyle ; et
A et B, identiques ou différents, représentent chacun un groupe thiényle ou phényle non substitué ou bien phényle substitué dans lequel les substituants peuvent être constitués par un à trois restes, identiques ou différents, qui sont chacun un reste chloro, fluoro, nitro, méthyle, éthyle ou propyle.

**7.** Composé selon la revendication 6, dans lequel
X et X' représentent chacun O ;
$R^1$ représente un atome d'hydrogène ;
$R^2$ et $R^3$ représentent chacun un atome d'hydrogène ou un groupe méthyle ou éthyle ;
$R^4$ représente un groupe trifluorométhyle, cyano, allyle, méthoxycarbonyle ou triméthylsilyle ; et
A et B, identiques ou différents, représentent chacun un groupe phényle non substitué ou bien substitué et comportant un ou deux restes identiques ou différents qui sont chacun un reste chloro, fluoro, nitro, méthyle et éthyle ; ou bien un groupe thiényle non substitué.

**8.** Composé selon la revendication 7, dans lequel
X et X' représentent O ;
$R^1$ représente un atome d'hydrogène ; et
$R^2$ représente un atome d'hydrogène, $R^3$ représente un groupe méthyle, $R^4$ représente un groupe trifluorométhyle, et A et B, identiques ou différents, représentent chacun un groupe phényle, non substitué ou substitué, dans lequel les substituants peuvent être un ou deux restes, identiques ou différents, qui sont chacun un reste chloro, méthyle ou nitro ; ou bien
$R^2$ et $R^3$, identiques ou différents, représentent chacun un groupe méthyle ou éthyle, $R^4$ représente un groupe cyano, et A et B, identiques ou différents, représentent chacun un groupe phényle non substitué ou bien substitué et dans lequel les substituants peuvent être formés par un ou deux restes, identiques ou différents, chaque reste étant un reste chloro, fluoro, méthyle, éthyle ou nitro ; ou bien
$R^2$ et $R^3$ représentent chacun un groupe méthyle, $R^4$ represente un groupe allyle, et A et B, identiques ou différents, représentent chacun un groupe phényle non substitué ou un groupe phényle substitué par un reste méthyle ; ou bien
$R^2$ et $R^3$ représentent chacun un atome d'hydrogène, $R^4$ représente un groupe triméthylsilyle, et A et B, identiques ou différents, représentent chacun un groupe phényle substitué dans lequel les

substituants peuvent être formés par un ou deux restes, identiques ou différents, chaque reste étant un reste méthyle, éthyle ou nitro.

**9.** Composé selon la revendication 8, dans lequel
X et X' représentent chacun O ;
$R^1$ représente un atome d'hydrogène ; et
$R^2$ représente un atome d'hydrogène, $R^3$ représente un groupe méthyle, $R^4$ représente un groupe trifluorométhyle, et A et B représentent chacun un groupe phényle non substitué, ou bien A représente un groupe 2,3-diméthylphényle et B représente un groupe 2,4-dichlorophényle, 3,5-diméthylphényle ou 2-nitro-5-méthylphényle ; ou
$R^2$ et $R^3$ représentent chacun un groupe méthyle, $R^4$ représente un groupe allyle, et A et B représentent chacun un groupe phényle non substitué ou bien chacun un groupe 3-méthyl-phényle ; ou bien
$R^2$ et $R^3$ représentent chacun un atome d'hydrogène, $R^4$ représente un groupe triméthylsilyle, et A représente un groupe 4-éthylphényle et B représente un groupe 2-nitro-5-méthylphényle, ou bien A et B représentent chacun un groupe 2-méthylphényle ou bien chacun un groupe 2-nitrophényle.

**10.** Composé selon la revendication 8, dans lequel
X et X' représentent chacun O ;
$R^1$ représente un atome d'hydrogène ;
$R^2$ et $R^3$ représentent chacun un groupe méthyle ;
$R^4$ représente un groupe cyano ; et
A et B, identiques ou différents, représentent chacun un groupe phényle non substitué ou bien un groupe phényle substitué dans lequel les substituants peuvent être constitués par un ou deux restes, identiques ou différents, dont chacun est un reste chloro, fluoro, méthyle, éthyle ou nitro.

**11.** Composition insecticide, comprenant un diluant, véhicule ou excipient agronomiquement acceptable et un composé insecticide selon l'une quelconque des revendications précédentes.

**12.** Composition selon la revendication 11, contenant ledit composé insecticide en une quantité de 0,0001 à 99 % du poids de la composition.

**13.** Composition selon la revendication 12, dans laquelle la quantité dudit composé insecticide est de 0,01 à 99 % du poids de la composition.

**14.** Procédé pour maîtriser des insectes, qui comprend la mise en contact desdits insectes avec une quantité à efficacité insecticide d'un composé selon l'une quelconque des revendications 1 à 10, éventuellement dans une composition selon l'une quelconque des revendications 11 à 13.

**15.** Procédé selon la revendication 14, dans lequel on applique ledit composé ou ladite composition à des insectes ou à des plantes en cours de croissance ou bien à une zone dans laquelle les plantes doivent être cultivées, l'application étant réalisée en une quantité de 10 g à 10 kg du composé par hectare.

**16.** Procédé selon la revendication 15, dans lequel le taux d'application est de 100 g à 5 kg du composé par hectare.

**17.** Procédé selon l'une quelconque des revendications 14 à 16, dans lequel lesdits insectes appartiennent à l'ordre des Lépidoptères ou des Coléoptères.

Revendications pour l'Etat contractant suivant : AT

**1.** Composition à activité insecticide, qui comprend un composé répondant à la formule :

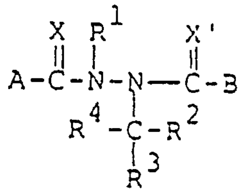

I

dans laquelle :

X et X', identiques ou différents, représentent chacun O, S ou NR ;

$R^1$ represente un atome d'hydrogène ; un groupe alkyle en $C_1$ à $C_6$ ; alcoxy (en $C_1$ à $C_6$)-alkyle (en $C_1$ à $C_6$) ayant, independamment, le nombre indiqué des atomes de carbone dans chaque groupe alkyle ; alkyl (en $C_1$ à $C_6$)-thio-alkyle (en $C_1$ à $C_6$) ayant, independamment, le nombre indique d'atomes de carbone dans chaque groupe alkyle ; alcenyle en $C_2$ à $C_6$ ; alcynyle en $C_2$ à $C_6$ ; ou phenyl-alkyle (en $C_1$ à $C_4$) dans lequel le noyau phényle n'est pas substitué ou est substitué par un à trois substituants, identiques ou différents, qui sont chacun un reste halogéno, cyano, nitro, hydroxy, alkyle en $C_1$ à $C_4$, halogéno-alkyle (en $C_1$ à $C_4$) alcoxy (en $C_1$ à $C_4$), halogéno-alcoxy en $C_1$ à $C_4$, carboxy, alcoxy (en $C_1$ à $C_4$)-carbonyle, alcanoyloxy en $C_1$ à $C_4$ ou NZZ' ;

$R^2$ et $R^3$, identiques ou différents, représentent chacun un atome d'hydrogène ou un groupe alkyle en $C_1$ à $C_4$ ;

$R^4$ représente un groupe alkyle en $C_1$ à $C_4$, substitué par un à quatre atomes de fluor ; un groupe alcényle (en $C_2$ à $C_4$) linéaire ; un groupe carboxyle ; alcoxy (en $C_1$ à $C_3$)-carbonyle ; cyano; alkyle (en $C_1$ à $C_4$) à substituant(s) cyano ; un groupe trialkyl (en $C_1$ à $C_4$) silyle, dont chaque groupe alkyle présente, indépendamment, le nombre indiqué des atomes de carbone ; ou un groupe trialkyl (en $C_1$ à $C_2$) silylméthyle dont chaque groupe alkyle présente, indépendamment, le nombre indiqué des atomes de carbone ; à la condition que $R^2$ et $R^3$ représentent chacun un groupe alkyle lorsque $R^4$ représente un groupe carboxyle ou alcoxycarbonyle ; et

A et B, identiques ou différents, représentent chacun :

un groupe naphtyle, substitué ou non substitué, dans lequel les substituants peuvent être constitués par un à trois des restes suivants, identiques ou différents : un reste halogéno, nitro, alcoxy en $C_1$ à $C_4$, alkyle en $C_1$ à $C_4$ ou NZZ' ;

un groupe phényle, substitué ou non substitué, dans lequel les substituants peuvent être constitués par un à cinq des restes suivants, identiques ou différents : un reste halogéno ; nitroso ; nitro ; cyano ; hydroxy ; alkyle en $C_1$ à $C_6$ ; halogénoalkyle en $C_1$ à $C_6$ ; cyanoalkyle en $C_1$ à $C_6$ ; hydroxyalkyle en $C_1$ à $C_6$ ; alcoxy en $C_1$ à $C_6$ ; halogéno-alcoxy en $C_1$ à $C_6$ ; alcoxy (en $C_1$ à $C_6$)-alkyle (en $C_1$ à $C_6$) ayant indépendamment le nombre indiqué d'atomes de carbone dans chaque groupe alkyle ; alcoxy (en $C_1$ à $C_6$)-alcoxy (en $C_1$ à $C_6$) ayant indépendamment le nombre indiqué d'atomes de carbone dans chaque groupe alkyle ; un groupe -ORSR' ; un groupe $-OCO_2R$ ; un groupe $-OCO_2H$ ; un groupe alcanoyl (en $C_1$ à $C_6$)-oxy-alkyle en $C_1$ à $C_6$ ayant indépendamment le nombre indiqué d'atomes de carbone dans chaque groupe alkyle ;

un groupe alcényle en $C_2$ à $C_6$, éventuellement substitué par un groupe halogéno, cyano, alkyle en $C_1$ à $C_4$, halogénoalcoxy en $C_1$ à $C_4$, alkylthio en $C_1$ à $C_4$ ou alcoxy en $C_1$ à $C_4$ ; alcadiényle en $C_3$ à $C_5$ ; alcényloxy en $C_2$ à $C_6$ ; alcényl (en $C_2$ à $C_6$)-carbonyle ; alcényloxy (en $C_2$ à $C_6$)-carbonyloxy ; alcynyle en $C_2$ à $C_6$ (éventuellement substitué par un atome d'halogène, un groupe cyano, nitro, hydroxy, alcoxy en $C_1$ à $C_4$, halogéno-alkyle en $C_1$ à $C_4$, alkylthio en $C_1$ à $C_4$ ou alkyle en $C_1$ à $C_4$) ; un groupe carboxy ; un groupe $-RCO_2R'$ ; un groupe -COR ; -COH ; halogéno-alkyl (en $C_1$ à $C_6$)-carbonyle ; un groupe $-CO_2R$ ; halogéno-alcoxy (en $C_1$ à $C_6$) carbonyle ; un groupe -OCOR ; un groupe $-ORCO_2R'$ ; un groupe amino (-NZZ') ; un groupe amino (substitué par un reste hydroxy, alcoxy en $C_1$ à $C_4$ ou alkylthio en $C_1$ à $C_4$) ; un groupe -CONZZ' ; un groupe alcényl (en $C_2$ à $C_6$)-carbonylamino ; hydroxy-alkylamino (en $C_1$ à $C_6$)-caronyle ; un groupe -OCONZZ' ; un groupe -NZCOZ' ; un groupe $-NZCO_2Z'$ ; un groupe thiocyanato ; isothiocyanato ; thiocyanatoalkyle (en $C_1$ à $C_6$) ; alkylthio (en $C_1$ à $C_6$) ; halogéno-alkylthio (en $C_1$ à $C_6$) ; un groupe -S(O)Z ; un groupe $-SO_2Z$; un groupe $-OSO_2R$ ; un groupe $-OSO_2H$ ; un groupe $-SO_2NZZ'$ ; un groupe -CSR ; un groupe -CSH ; un groupe -SCOR ; un groupe -SCOH ; un groupe -NSCSZ' ; un groupe phényle, non

substitué ou bien substitué comportant un à trois des restes suivants, identiques ou différents : un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe phénoxy dans lequel le noyau phényle n'est pas substitué ou est substitué par un à trois restes, identiques ou différents, qui sont des restes halogéno, cyano, nitro, hydroxy, alkyle en $C_1$ à $C_4$, halogéno-alkyle en $C_1$ à $C_4$, alcoxy (en $C_1$ à $C_4$), carboxy, alkyl (en $C_1$ à $C_4$)-carbonyle, alcanoyloxy en $C_1$ à $C_4$, amino, alkyl (en$C_1$ à $C_4$)-amino ou dialkyl (en $C_1$ à $C_4$)-amino ayant, indépendamment, le nombre indiqué d'atomes de carbone dans chaque groupe alkyle ; un groupe benzoyle dont le noyau phényle n'est pas substitué ou est substitué par un à trois substituants, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; benzoyloxy-alkyle (en $C_1$ à $C_6$) ; phénylthio-alkyle (en $C_1$ à $C_6$), dont le noyau phényle n'est pas substitué ou est substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe -CR=N-R''', dans lequel R''' représente un groupe hydroxy, alkyle en $C_1$ à $C_4$, alcoxy en $C_1$ à $C_4$, amino (-NZZ'), phénylamino, -COR, -COH ou benzoyle ; un groupe oxirannyle en $C_2$ à $C_6$ ; acétylthiosemi-carbazone ; pyrrolyle ; oxazolyle, non substitué ou substitué par un ou deux groupes méthyles ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_{10}$, non substitué ou substitué comportant alors un à quatre substituants identiques ou différents, qui sont chacun un reste halogéno, cyano, nitro, hydroxy, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou -NZZ' ;

un groupe cycloalkyle en $C_3$ à $C_8$, non substitué ou substitué, ou un groupe cyclo (en $C_3$ à $C_8$)-alkyle (en $C_1$ à $C_4$), non substitué ou substitué comportant un à quatre substituants, identiques ou différents, qui sont chacun un reste halogéno, cyano, nitro, hydroxy, alkyle en $C_1$ à $C_4$, halogénoalkyle en $C_1$ à $C_4$, alcoxy en $C_1$ à $C_4$, halogéno-alcoxy en $C_1$ à $C_4$, carboxy, alcanoyle en $C_1$ à $C_4$, alcoxy (en $C_1$ à $C_4$)-carbonyle, alcanoyloxy en $C_1$ à $C_4$ ou -NZZ' ;

un groupe alcényle en $C_2$ à $C_8$, non substitué ou substitué ou un groupe alcadiényle en $C_3$ à $C_5$, non substitué ou substitué et ayant comme substituant(s) un groupe furyle, thiényle ou pyridyle ou bien ayant un à quatre substituants, identiques ou différents, qui sont chacun un reste halogéno, cyano, nitro, hydroxy, alkyle en $C_1$ à $C_4$, cycloalkyle en $C_3$ à $C_6$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$ ou -NZZ' ;

un groupe cycloalcényle en $C_3$ à $C_8$, non substitué ou substitué, ou un groupe cycloalcadiényle (en $C_8$), non substitué ou substitue et ayant alors comme substituant(s) un à quatre restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$ ou -NZZ' ;

un groupe alcynyle en $C_2$ à $C_8$, non substitué ou substitué et ayant alors comme substituant(s) un à quatre restes, identiques ou différents, qui sont chacun un reste halogeno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou NZZ' ;

un groupe phénylalkyle comportant 1 à 4 atomes de carbone dans le groupe alkyle et dans lequel le groupe alkyle n'est pas substitué ou est substitué par un à trois restes, identiques ou différents, qui

sont chacun un reste halogéno, cyano, hydroxy, alcoxy en $C_1$ à $C_4$, alcoxy (en $C_1$ à $C_4$)-carbonyle ou -NZZ', et le noyau phényle n'est pas substitué ou est substitué par un à trois restes, identiques ou différents, qui sont chacun un reste halogéno, cyano, nitro, hydroxy, alkyle en $C_1$ à $C_4$, halogénoalkyle en $C_1$ à $C_4$, cyanoalkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, alcényle en $C_2$ à $C_6$, halogéno-alcényle en $C_2$ à $C_6$, alcynyle en $C_2$ à $C_6$ ou -NZZ' ; un groupe phénylalcényle ayant deux à six atomes de carbone dans le groupe alcényle, ce groupe alcényle n'étant pas substitué ou étant substitué par un à trois restes, identiques ou différents, qui sont chacun un reste halogéno, cyano, hydroxy, alkyle en $C_1$ à $C_4$, halogénoalkyle en $C_1$ à $C_4$, alcoxy (en $C_1$ à $C_4$), halogéno-alcoxy en $C_1$ à $C_4$, alcoxy (en $C_1$ à $C_4$)-carbonyle ou -NZZ', et le noyau phényle n'étant pas substitué ou étant substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogénoalcoxy en $C_1$ à $C_4$, carboxy, alcoxy (en $C_1$ à $C_4$)-carbonyle, alcanoyloxy en $C_1$ à $C_4$ ou -NZZ' ;

un hétérocycle pentagonal, non substitué ou substitué, qui est choisi parmi un groupe furyle, thiényle, triazolyle, pyrrolyle, isopyrrolyle, pyrazolyle, isoimidazolyle, thiazolyle, isothiazolyle, oxazolyle et isooxazolyle, les substituants pouvant être un à trois radicaux identiques ou différents qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle ; un groupe $-RCO_2H$ ; un groupe $RCO_2R'$ ; un groupe -CONZZ' ; un groupe amino (-NZZ') ; un groupe -NZCOZ' ; un groupe alkylthio en $C_1$ à $C_6$; ou un groupe phényle, non substitué ou substitué comportant un à trois restes, identiques ou différents, qui sont chacun un reste halogéno, nitro, alkyle en $C_1$ à $C_6$, halogénoalkyle en $C_1$ à $C_6$, alcoxy en $C_1$ à $C_6$, halogéno-alcoxy en $C_1$ à $C_6$, carboxy, alcoxy (en $C_1$ à $C_4$)-carbonyle ou amino (-NZZ') ; ou bien

un hétérocycle hexagonal, non substitué ou substitué, comportant un, deux, trois ou quatre atomes d'azote et deux à cinq atomes de carbone de noyau , dans lequel les substituants peuvent être constitués par un à trois restes, identiques ou différents, qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle, un groupe $-RCO_2H$ ; un groupe $-RCO_2R'$ ; un groupe -CONZZ' ; un groupe amino (NZZ') ; un groupe -NZCOZ' ; un groupe alkylthio en $C_1$ à $C_6$ ; ou bien un groupe phényle non substitué ou substitué, comportant un à trois restes identiques ou différents qui sont chacun un reste halogéno, nitro, alkyle en $C_1$ à $C_6$, halogéno-alkyle en $C_1$ à $C_6$, alcoxy en $C_1$ à $C_6$, halogéno-alcoxy (en $C_1$ à $C_6$), carboxy, alcoxy (en $C_1$ à $C_4$)-carbonyle ou amino (-NZZ') ;

les symboles R et R' représentant chacun un groupe alkyle en $C_1$ à $C_6$ ; Z et Z' représentant chacun un atome d'hydrogène ou un groupe alkyle en $C_1$ à $C_4$ ; le terme "amino" désignant un groupe NZZ' ; ou un sel agronomiquement acceptable de ce composé ; avec un diluant ou véhicule ou excipient agronomiquement acceptable.

**2.** Composition selon la revendication 1, dans laquelle :

X et X' représentent O ou S ;

$R^1$ représente un atome d'hydrogène ; un groupe alkyle en $C_1$ à $C_4$, alcoxy (en $C_1$ à $C_4$)-alkyle (en $C_1$ à $C_4$), ayant, indépendamment, le nombre indiqué d'atomes de carbone dans chaque groupe alkyle ; un groupe méthylthiométhyle ; alcényle en $C_2$ à $C_5$ ; alcynyle en $C_2$ à $C_5$ ; ou phényl-alkyle en $C_1$ à $C_2$, dans lequel le noyau phényle n'est pas substitué ou est substitué par un ou deux restes halogéno, nitro, alkyle en $C_1$ à $C_4$ ou alcoxy en $C_1$ à $C_4$ ;

$R^2$ et $R^3$ sont identiques ou différents et représentent chacun un atome d'hydrogène ou un groupe alkyle en $C_1$ à $C_3$ ;

$R^4$ représente un groupe alkyle en $C_1$ à $C_3$, substitué par un à quatre atomes de fluor ; un groupe alcényle linéaire en $C_2$ à $C_4$ ; carboxyle ; alcoxy (en $C_1$ ou $C_2$)-carbonyle ; cyano ; alkyle (en $C_1$ à $C_3$) substitué par un groupe cyano ; trialkyl (en $C_1$ à $C_2$)-silyle ayant, indépendamment, le nombre indiqué d'atomes de carbone dans chaque groupe alkyle ; ou trialkyl (en $C_1$ ou $C_2$)-silylméthyle ayant, indépendamment, le nombre indiqué d'atomes de carbone dans chaque groupe alkyle ; et

A et B, identiques ou différents, représentent chacun :

un groupe naphtyle non substitué ;

un groupe phényle, non substitué ou bien substitué et dans lequel les substituants peuvent être constitués par un à trois restes différents ou identiques qui sont chacun un reste halogéno ; nitro ; cyano ; alkyle en $C_1$ à $C_4$ ; halogéno-alkyle en $C_1$ à $C_4$ ; cyanoalkyle en $C_1$ à $C_4$ ; alcoxy en $C_1$ à

$C_4$ ; alcoxy (en $C_1$ à $C_4$) alkyle (en $C_1$ à $C_4$) ayant, indépendamment, le nombre indiqué d'atomes de carbone dans chaque groupe alkyle ; -COZ ; carboxy ; alcoxy (en $C_1$ à $C_4$)-carbonyle ; alcanoyloxy en $C_1$ à $C_4$ ; alcényle en $C_2$ à $C_6$ ; alcynyle en $C_2$ à $C_6$ ; amino (-NZZ') ; thiocyanato ; alkylthio en $C_1$ à $C_4$ ; -CSR ; -CSH ; un groupe phényle non substitué, ou substitué ayant un ou deux restes, identiques ou différents qui sont chacun un reste halogéno, nitro, alkyle en $C_1$ à $C_4$, alcoxy en $C_1$ à $C_4$, carboxy, -NZZ' ; un groupe phénoxy dans lequel le noyau phényle n'est pas substitué ou est substitué par un ou deux restes, identiques ou différents, qui sont chacun un reste halogéno, nitro, alkyle en $C_1$ à $C_4$, alcoxy en $C_1$ à $C_4$, carboxy, -NZZ' ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_8$, non substitué ou substitué, comportant un à trois restes identiques ou différents qui sont chacun un reste halogéno, cyano, alcoxy en $C_1$ à $C_4$, phényle, alcoxy (en $C_1$ à $C_4$)-carbonyle ou halogénoalcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, comportant un ou deux substituants identiques ou différents qui sont chacun un radical halogéno, alkyle en $C_1$ à $C_4$, halogéno-alkyle en $C_1$ à $C_4$, alcoxy en $C_1$ à $C_4$, alcanoyle en $C_1$ à $C_4$ ou halogéno-alcoxy en $C_1$ à $C_4$ ; un groupe alcényle en $C_2$ à $C_6$, non substitué ou substitué, comportant comme substituant(s) un reste furyle ou un à trois restes, identiques ou différents, qui sont chacun un reste alkyle en $C_1$ à $C_4$, halogéno-alkyle en $C_1$ à $C_4$, alcoxy en $C_1$ à $C_4$ ou halogénoalcoxy en $C_1$ à $C_4$ ; un groupe cycloalkényle (en $C_3$ à $C_6$) non-substitué ou substitué, ayant comme substituants un ou deux restes identiques ou différents qui sont halogéno, alkyle en $C_1$ à $C_4$, halogénoalkyle en $C_1$ à $C_4$, alcoxy en $C_1$ à $C_4$ ou halogénoalcoxy en $C_1$ à $C_4$ ;

un groupe alcynyle non substitué ou substitué par un groupe phényle ;

un groupe phénylalkyle ayant 1 ou 2 atomes de carbone dans le groupe alkyle, ce groupe alkyle n'étant pas substitué ou étant substitué par un ou deux restes, identiques ou différents, qui sont chacun un reste halogéno, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un groupe phénylalcényle ayant 2 ou 3 atomes de carbone dans le groupe alcényle, ce groupe alcényle n'étant pas substitué ou étant substitué par un reste halogéno, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un hétérocycle pentagonal, non substitué ou substitue, choisi parmi un groupe furyle, thiényle, triazolyle, pyrrolyle et oxazolyle, les substituants pouvant être un ou deux restes identiques ou différents qui sont chacun un reste halogéno ; nitro ; alkyle en $C_1$ à $C_4$ ; alcoxy en $C_1$ à $C_4$ ; -NZZ' ; ou bien un groupe phényle, non substitué ou substitué comportant un ou deux restes identiques ou différents qui sont chacun un reste halogeno, nitro, alkyle en $C_1$ à $C_4$, halogéno-alkyle en $C_1$ à $C_4$, alcoxy en $C_1$ à $C_4$, halogeno-alcoxy en $C_1$ à $C_4$, carboxy ou -NZZ' ; ou

un hétérocycle hexagonal non substitué ou substitué, comportant un ou deux atomes d'azote et quatre à cinq atomes de carbone de noyau, les substituants pouvant être constitués par un ou deux restes identiques ou différents qui sont chacun un reste halogéno ; nitro ; alkyle en $C_1$ à $C_4$ ; alcoxy en $C_1$ à $C_4$ ; thioalcoxy en $C_1$ à $C_4$ ; -NZZ' ; ou un groupe phényle non substitué ou substitué comportant un ou deux restes identiques ou différents qui sont chacun un reste halogéno, nitro, alkyle en $C_1$ à $C_4$, halogéno-alkyle en $C_1$ à $C_4$, alcoxy en $C_1$ à $C_4$, halogéno-alcoxy en $C_1$ à $C_4$, carboxy ou -NZZ' ;

les symboles R et R' représentant chacun un groupe alkyle en $C_1$ à $C_4$ ; et Z et Z' représentant chacun un atome d'hydrogène ou un groupe alkyle en $C_1$ à $C_4$.

**3.** Composition selon la revendication 2, dans laquelle :

X et X' représentent O ou S ;
$R^1$ représente un atome d'hydrogène, un groupe alkyle en $C_1$ à $C_4$ ; alcoxy (en $C_1$ à $C_4$) alkyle en $C_1$ à $C_4$ comportant, indépendamment, le nombre indiqué d'atomes de carbone dans chaque groupe alkyle ; alcényle en $C_2$ à $C_5$ ; alcynyle en $C_2$ à $C_5$ ; ou benzyle dans lequel le noyau phényle n'est pas substitué ou est substitué par un reste halogéno, nitro, alkyle en $C_1$ à $C_4$ ou alcoxy en $C_1$ à $C_4$ ;
$R^2$ et $R^3$, identiques ou différents, représentent chacun un atome d'hydrogène ou un groupe alkyle en $C_1$ ou $C_2$ ;
$R^4$ représente un groupe alkyle en $C_1$ à $C_3$, substitué par un à quatre restes fluoro ; ùn groupe alcényle liné-aire en $C_2$ à $C_4$ ; un groupe méthoxycarbonyle ; cyano ; alkyle en $C_1$ ou $C_2$ à substituant(s) cyano ; trialkyl (en $C_1$ ou $C_2$) silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; à la condition que $R^2$ et $R^3$ représentent chacun un groupe alkyle lorsque $R^4$ représente un groupe méthoxycarbonyle ; et
A et B sont identiques ou différents et représentent chacun :
un groupe naphtyle non substitué ;

un groupe phényle, non substitué ou substitué, comportant un à trois restes identiques ou différents, qui sont chacun un reste halogéno ; nitro ; cyano ; alkyle en $C_1$ à $C_4$ ; halogéno-alkyle en $C_1$ à $C_4$ ; cyano-alkyle en $C_1$ à $C_4$ ; alcoxy en $C_1$ à $C_4$ ; alcoxy (en $C_1$ à $C_4$)-alkyle (en $C_1$ à $C_4$) ayant, indépendamment, le nombre indiqué d'atomes de carbone dans chaque groupe alkyle ; -COZ ; alcoxy (en $C_1$ à $C_4$)-carbonyle ; alcanoyloxy (en $C_1$ à $C_4$) ; thiocyanato ; -NZZ' ; alkylthio en $C_1$ à $C_4$ ; -CSZ ; $-CS_2Z$ ; un groupe phényle non substitué ou substitué comportant un ou deux restes, identiques ou différents, qui sont chacun un reste halogéno, nitro, alkyle en $C_1$ à $C_4$, alcoxy en $C_1$ à $C_4$, carboxy, -NZZ' ; ou bien un groupe phénoxy dans lequel le noyau phényle n'est pas substitué ou est substitué par un ou deux restes identiques ou différents qui sont chacun un reste halogéno, nitro, alkyle en $C_1$ à $C_4$, alcoxy en $C_1$ à $C_4$, carboxy, -NZZ' ou bien, lorsque deux positions adjacentes du noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_6$, non substitué ou substitué et comportant alors un à trois restes identiques ou différents qui sont chacun un reste halogéno, cyano, nitro, carboxy, alcoxy en $C_1$ à $C_4$, phényle, alcoxy (en $C_1$ à $C_4$)-carbonyle ou halogéno-alcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, dans lesquels le substituant est un reste halogéno, alkyle en $C_1$ à $C_4$, alcanoyle en $C_1$ à $C_4$, alcoxy en $C_1$ à $C_4$ ou halogéno-alcoxy en $C_1$ à $C_4$ ;

un groupe alcényle en $C_2$ à $C_6$, non substitué ou substitué et comportant alors un ou deux restes, identiques ou différents, qui sont chacun un reste halogéno, alkyle en $C_1$ à $C_4$, alcoxy en $C_1$ à $C_4$ ou halogéno-alcoxy en $C_1$ à $C_4$ ;

un groupe cycloalcényle en $C_4$ à $C_6$, non substitué, ou substitué et dans lequel le substituant est un reste halogéno, alkyle en $C_1$ à $C_4$, alcoxy en $C_1$ à $C_4$ ou halogéno-alcoxy en $C_1$ à $C_4$ ;

un groupe alcynyle, non substitué ou substitué par un groupe phényle ;

un groupe benzyle, dans lequel le noyau phényle n'est pas substitué ou est substitué par un ou deux restes, identiques ou différents, qui sont chacun un reste halogéno, méthyle ou éthyle ;

un hétérocycle pentagonal, choisi parmi un groupe furyle, thienyle, pyrrolyle et oxazolyle, non

substitué ou bien substitué et dans lequel les substituants peuvent être un ou deux restes, identiques ou différents, qui sont chacun un reste halogéno, nitro, alkyle en $C_1$ à $C_4$ ou alcoxy en $C_1$ à $C_4$ ; ou

un hétérocycle hexagonal comportant un ou deux atomes d'azote et quatre ou cinq atomes de carbone de noyau, l'hétérocycle n'étant pas substitué ou étant substitué, les substituants pouvant être un ou deux restes identiques ou différents qui sont chacun un reste halogéno, nitro, alkyle en $C_1$ à $C_4$, alcoxy en $C_1$ à $C_4$ ou thio-alcoxy en $C_1$ à $C_4$ ;

et Z et Z' representent chacun un atome d'hydrogène ou un groupe alkyle en $C_1$ à $C_4$.

**4.** Composition selon la revendication 3, dans laquelle :

X et X' représentent O ;

$R^1$ représente un atome d'hydrogène ; un groupe méthyle, éthyle, alcoxy (en $C_1$ ou $C_2$) alkyle (en $C_1$ ou $C_2$) ayant, indépendamment, le nombre indiqué d'atomes de carbone dans chaque groupe alkyle ; alcényle en $C_2$ à $C_5$ ; alcynyle en $C_2$ à $C_5$ ; ou benzyle, dans lequel le noyau phényle n'est pas substitué ou est substitué par un reste halogéno ;

$R^2$ et $R^3$, identiques ou différents, représentent chacun un atome d'hydrogène ou un groupe alkyle en $C_1$ ou $C_2$ ;

$R^4$ représente un groupe alkyle en $C_1$ ou $C_2$, substitué par un à quatre atomes de fluor ; un groupe alcényle linéaire en $C_2$ à $C_4$ ; cyano ; alkyle en $C_1$ ou $C_2$ à substituant(s) cyano ; trialkyl (en $C_1$ ou $C_2$) silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; et

A et B sont identiques ou différents et representent chacun un groupe phényle ou phenyle substitué, dans lequel les substituants peuvent être constitues par un à trois restes, identiques ou différents, qui sont chacun un reste halogeno, nitro, alkyle en $C_1$ à $C_4$, alcoxy en $C_1$ à $C_4$ ou halogeno-alkyle en $C_1$ à $C_4$ ;

un groupe alkyle en $C_1$ à $C_6$, non substitué ou substitué, ayant comme substituant(s) un ou deux restes identiques ou différents qui sont chacun un reste halogéno, phényle ou cyano ;

un groupe cycloalkyle en $C_4$ à $C_6$, qui n'est pas substitué ;

un groupe alcényle en $C_2$ à $C_6$, non substitué ou substitué et ayant alors comme substituant(s) un à trois restes, identiques ou différents, qui sont chacun un reste halogéno ou alkyle en $C_1$ à $C_4$ ;

un groupe cycloalcényle en $C_4$ à $C_6$, non substitué ; un groupe phénylalkyle ayant un ou deux atomes de carbone dans le groupe alkyle et dont le noyau phényle n'est pas substitué ou est substitué par un ou deux restes identiques ou différents qui sont chacun un reste halogéno, alkyle en $C_1$ à $C_4$ ou alcoxy en $C_1$ à $C_4$ ;

un groupe pyridyle ou 2,5-pyrazinyle, non substitué ou substitué, dans lesquels le substituant peut être un reste halogéno, nitro, alkyle en $C_1$ à $C_4$, alcoxy en $C_1$ à $C_4$ ou thioalcoxy en $C_1$ à $C_4$ ; ou

un groupe furyle ou thienyle non substitué ou un groupe pyrrolyle non substitué ou substitué, dans lesquels le substituant peut être un groupe alkyle en $C_1$ à $C_4$.

**5.** Composition selon la revendication 4, dans laquelle :

X et X' représentent O ;

$R^1$ représente un atome d'hydrogène, un groupe méthyle ; méthoxyméthyle ; alcényle en $C_2$ à $C_4$ ; alcynyle en $C_2$ à $C_5$ ; benzyle ou 4-halogénobenzyle ;

$R^2$ et $R^3$ sont identiques ou différents et représentent chacun un atome d'hydrogène ou un groupe méthyle ou éthyle ;

$R^4$ représente un groupe trifluorométhyle ; 2,2,2-trifluoroéthyle ; alcényle linéaire en $C_2$ à $C_4$ ; cyano ; cyanométhyle ; trialkyl (en $C_1$ ou $C_2$) silyle ayant, indépendamment, le nombre indiqué d'atomes de carbone dans chaque groupe alkyle ; ou triméthylsilylméthyle ; et

A et B, identiques ou différents, représentent chacun un groupe phényle ou un groupe phényle substitué dont les substituants peuvent être constitués par un, deux ou trois restes identiques ou différents qui sont chacun un reste chloro, fluoro, bromo, iodo, nitro, méthyle, éthyle, méthoxy ou trifluorométhyle ;

un groupe alcényle en $C_2$ ou $C_3$, substitué, comportant un à trois restes identiques ou différents qui sont chacun un reste chloro, bromo, méthyle ou éthyle ;
un groupe cyclohéxényle ;

un groupe benzyle ;

un groupe pyridyle ou 2,5-pyrazinyle, non substitués ou substitués dans lesquels le substituant peut être un reste halogéno, méthyle, éthyle ou méthoxy ; ou bien

un groupe furyle ou thiényle, non substitués ou un groupe pyrrolyle non substitué ou substitué dans lequel le substituant peut être un reste éthyle ou méthyle.

**6.** Composition selon la revendication 2, dans laquelle :

X et X' représentent chacun O ;
$R^1$ représente un atome d'hydrogène ou un groupe méthyle ;
$R^2$ et $R^3$ représentent chacun un atome d'hydrogène ou un groupe alkyle en $C_1$ à $C_3$ ;
$R^4$ représente un groupe trifluorométhyle, 2,2,2-trifluoroéthyle, cyano, cyanométhyle, alcényle linéaire en $C_2$ à $C_4$, carboxyle, méthoxycarbonyle, trialkyl (en $C_1$ ou $C_2$) silyle ayant, indépendamment, le nombre indiqué d'atomes de carbone dans chaque groupe alkyle ou trialkyl (en $C_1$ ou $C_2$) silylméthyle ayant, indépendamment, le nombre indique d'atomes de carbone dans chaque groupe alkyle ; et
A et B, identiques ou différents, représentent chacun un groupe thiényle ou phényle non substitué ou bien phényle substitué dans lequel les substituants peuvent être constitues par un à trois restes, identiques ou différents, qui sont chacun un reste chloro, fluoro, nitro, méthyle, ethyle ou propyle.

**7.** Composition selon la revendication 6, dans laquelle :

X et X' représentent chacun O ;
$R^1$ représente un atome d'hydrogène ;
$R^2$ et $R^3$ representent chacun un atome d'hydrogène ou un groupe méthyle ou éthyle ;
$R^4$ représente un groupe trifluorométhyle, cyano, allyle, méthoxycarbonyle ou triméthylsilyle ; et
A et B, identiques ou différents, représentent chacun un groupe phényle non substitué ou bien substitué et comportant un ou deux restes identiques ou différents qui sont chacun un reste chloro, fluoro, nitro, méthyle et éthyle ; ou bien un groupe thiényle non substitué.

**8.** Composition selon la revendication 7, dans laquelle :
X et X' représentent O ;
$R^1$ représente un atome d'hydrogène ; et
$R^2$ represente un atome d'hydrogène, $R^3$ représente un groupe méthyle, $R^4$ représente un groupe trifluorométhyle, et A et B, identiques ou différents, représentent chacun un groupe phényle, non substitué ou substitué, dans lequel les substituants peuvent être un ou deux restes, identiques ou différents, qui sont chacun un reste chloro, methyle ou nitro ; ou bien
$R^2$ et $R^3$, identiques ou différents, représentent chacun un groupe méthyle ou éthyle, $R^4$ représente un groupe cyano, et A et B, identiques ou différents, représentent chacun un groupe phényle non substitué ou bien substitué et dans lequel les substituants peuvent être formés par un ou deux restes, identiques ou différents, chaque reste étant un reste chloro, fluoro, méthyle, éthyle ou nitro ; ou bien
$R^2$ et $R^3$ représentent chacun un groupe méthyle, $R^4$ représente un groupe allyle, et A et B, identiques ou différents, représentent chacun un groupe phényle non substitué ou un groupe phényle substitué par un reste méthyle ; ou bien
$R^2$ et $R^3$ représentent chacun un atome d'hydrogène, $R^4$ représente un groupe triméthylsilyle, et A et B, identiques ou différents, représentent chacun un groupe phényle substitué dans lequel les

substituants peuvent être formés par un ou deux restes, identiques ou différents, chaque reste étant un reste méthyle, éthyle ou nitro.

**9.** Composition selon la revendication 8, dans laquelle :

X et X' représentent chacun O ;
$R^1$ représente un atome d'hydrogène ; et
$R^2$ représente un atome d'hydrogène, $R^3$ représente un groupe méthyle, $R^4$ représente un groupe trifluorométhyle, et A et B représentent chacun un groupe phényle non substitué, ou bien A représente un groupe 2,3-diméthylphényle et B représente un groupe 2,4-dichlorophényle, 3,5-diméthylphényle ou 2-nitro-5-méthylphényle ; ou
$R^2$ et $R^3$ représentent chacun un groupe méthyle, $R^4$ représente un groupe allyle, et A et B représentent chacun un groupe phényle non substitué ou bien chacun un groupe 3-méthyl-phényle ; ou bien
$R^2$ et $R^3$ représentent chacun un atome d'hydrogène, $R^4$ représente un groupe triméthylsilyle, et A représente un groupe 4-éthylphényle et B représente un groupe 2-nitro-5-méthylphényle, ou bien A et B représentent chacun un groupe 2-méthylphényle ou bien chacun un groupe 2-nitrophényle.

**10.** Composition selon la revendication 8, dans laquelle :
X et X' représentent chacun O ;
$R^1$ représente un atome d'hydrogène ;
$R^2$ et $R^3$ représentent chacun un groupe méthyle ;
$R^4$ représente un groupe cyano ; et
A et B, identiques ou différents, représentent chacun un groupe phényle non substitué ou bien un groupe phényle substitué dans lequel les substituants peuvent être constitués par un ou deux restes, identiques ou différents, dont chacun est un reste chloro, fluoro, méthyle, éthyle ou nitro.

**11.** Composition selon l'une quelconque des revendications précédentes, contenant ledit composé insecticide présent en une quantité de 0,0001 à 99 % du poids de la composition.

**12.** Composition selon l'une quelconque des revendications précédentes, dans laquelle la quantité dudit composé insecticide est de 0,01 à 99 % du poids de la composition.

**13.** Procédé pour maîtriser des insectes, qui comprend la mise en contact desdits insectes avec une quantité, efficace du point de vue insecticide, d'un composé tel que défini dans l'une quelconque des revendications 1 à 10, éventuellement dans une composition selon l'une quelconque des revendications 1 à 12.

**14.** Procédé selon la revendication 13, dans lequel on applique aux insectes, ou aux plantes en cours de croissance ou de culture ou à une zone dans laquelle les plantes doivent être cultivées, ledit composé ou ladite composition en une quantité de 10 g à 10 kg du composé par hectare.

**15.** Procédé selon la revendication 14, dans lequel le taux d'application est de 100 g à 5 kg du composé par hectare.

**16.** Procédé selon l'une quelconque des revendications 13 à 15, dans lequel lesdits insectes appartiennent à l'ordre des Lépidoptères ou des Coléoptères.

Revendications pour l'Etat contractant suivant : ES

**1.** Utilisation d'un composé à activité insecticide, qui est une hydrazine substituée répondant à la formule :

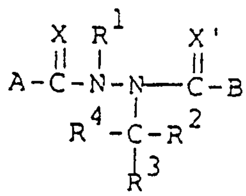

I

dans laquelle :

X et X', identiques ou différents, representent chacun O, S ou NR ;
$R^1$ représente un atome d'hydrogène ; un groupe alkyle en $C_1$ à $C_6$ ; alcoxy (en $C_1$ à $C_6$)-alkyle (en $C_1$ à $C_6$) ayant, indépendamment, le nombre indiqué des atomes de carbone dans chaque groupe alkyle ; alkyle (en $C_1$ à $C_6$)-thio-alkyle (en $C_1$ à $C_6$) ayant, indépendamment, le nombre indique d'atomes de carbone dans chaque groupe alkyle ; alcényle en $C_2$ à $C_6$ ; alcynyle en $C_2$ à $C_6$ ; ou phenyl-alkyle (en $C_1$ à $C_4$) dans lequel le noyau phényle n'est pas substitué ou est substitué par un à trois substituants, identiques ou différents, qui sont chacun un reste halogeno, cyano, nitro, hydroxy, alkyle en $C_1$ à $C_4$, halogeno-alkyle(en $C_1$ à $C_4$) alcoxy (en $C_1$ à $C_4$), halogeno-alcoxy en $C_1$ à $C_4$, carboxy, alcoxy (en $C_1$ à $C_4$)-carbonyle, alcanoyloxy en $C_1$ à $C_4$ ou NZZ' ;
$R^2$ et $R^3$, identiques ou différents, représentent chacun un atome d'hydrogène ou un groupe alkyle en $C_1$ à $C_4$ ;
$R^4$ représente un groupe alkyle en $C_1$ à $C_4$, substitué par un à quatre atomes de fluor ; un groupe alcényle (en $C_2$ à $C_4$) linéaire ; un groupe carboxyle ; alcoxy (en $C_1$ à $C_3$)-carbonyle ; cyano ; alkyle (en $C_1$ à $C_4$) à substituant(s) cyano ; un groupe trialkyl (en $C_1$ à $C_4$) silyle, dont chaque groupe alkyle présente, indépendamment, le nombre indiqué des atomes de carbone ; ou un groupe trialkyl (en $C_1$ à $C_2$) silylméthyle dont chaque groupe alkyle présente, indépendamment, le nombre indiqué des atomes de carbone ; à la condition que $R^2$ et $R^3$ représentent chacun un groupe alkyle lorsque $R^4$ représente un groupe carboxyle ou alcoxycarbonyle ; et
A et B, identiques ou différents, représentent chacun :

un groupe naphtyle, substitué ou non substitué, dans lequel les substituants peuvent être constitués par un à trois des restes suivants, identiques ou différents : un reste halogéno, nitro, alcoxy en $C_1$ à $C_4$, alkyle en $C_1$ à $C_4$ ou NZZ' ;

un groupe phényle, substitué ou non substitué, dans lequel les substituants peuvent être constitués par un à cinq des restes suivants, identiques ou différents : un reste halogéno ; nitroso ; nitro ; cyano ; hydroxy ; alkyle en $C_1$ à $C_6$ ; halogénoalkyle en $C_1$ à $C_6$ ; cyanoalkyle en $C_1$ à $C_6$ ; hydroxyalkyle en $C_1$ à $C_6$ ; alcoxy en $C_1$ à $C_6$ ; halogéno-alcoxy en $C_1$ à $C_6$ ; alcoxy (en $C_1$ à $C_6$)-alkyle (en $C_1$ à $C_6$) ayant indépendamment le nombre indiqué d'atomes de carbone dans chaque groupe alkyle ; alcoxy (en $C_1$ à $C_6$)-alcoxy (en $C_1$ à $C_6$) ayant indépendamment le nombre indiqué d'atomes de carbone dans chaque groupe alkyle ; un groupe -ORSR' ; un groupe $-OCO_2R$ ; un groupe $-OCO_2H$ ; un groupe alcanoyl (en $C_1$ à $C_6$)-oxy-alkyle en $C_1$ à $C_6$ ayant indépendamment le nombre indiqué d'atomes de carbone dans chaque groupe alkyle ;

un groupe alcényle en $C_2$ à $C_6$, éventuellement substitué par un groupe halogéno, cyano, alkyle en $C_1$ à $C_4$, halogénoalcoxy en $C_1$ à $C_4$, alkylthio en $C_1$ à $C_4$ ou alcoxy en $C_1$ à $C_4$ ; alcadiényle en $C_3$ à $C_5$ ; alcényloxy en $C_2$ à $C_6$ ; alcényl (en $C_2$ à $C_6$)-carbonyle ; alcényloxy (en $C_2$ à $C_6$)-carbonyloxy ; alcynyle en $C_2$ à $C_6$ (éventuellement substitué par un atome d'halogène, un groupe cyano, nitro, hydroxy, alcoxy en $C_1$ à $C_4$, halogéno-alkyle en $C_1$ à $C_4$, alkylthio en $C_1$ à $C_4$ ou alkyle en $C_1$ à $C_4$) ; un groupe carboxy ; un groupe $-RCO_2R'$ ; un groupe -COR ; -COH ; halogéno-alkyl (en $C_1$ à $C_6$)-carbonyle ; un groupe $-CO_2R$ ; halogéno-alcoxy (en $C_1$ à $C_6$) carbonyle ; un groupe -OCOR ; un groupe $-ORCO_2R'$ ; un groupe amino (-NZZ') ; un groupe amino (substitué par un reste hydroxy, alcoxy en $C_1$ à $C_4$ ou alkylthio en $C_1$ à $C_4$) ; un groupe -CONZZ' ; un groupe alcényl (en $C_2$ à $C_6$)-carbonylamino ; hydroxy-alkylamino (en $C_1$ à $C_6$)-carbonyle ; un groupe -OCONZZ' ; un groupe -NZCOZ' ; un groupe $-NZCO_2Z'$ ; un groupe thiocyanato ; isothiocyanato ; thiocyanatoalkyle (en $C_1$ à $C_6$) ; alkylthio (en $C_1$ à $C_6$) ; halogéno-alkylthio (en $C_1$ à $C_6$) ; un groupe -S(O)Z ; un groupe $-SO_2Z$; un groupe $-OSO_2R$ ; un groupe $-OSO_2H$ ; un groupe $-SO_2NZZ'$ ; un groupe -CSR ; un groupe -CSH ; un groupe -SCOR ; un groupe -SCOH ; un groupe -NSCSZ' ; un groupe phényle, non

substitué ou bien substitué comportant un à trois des restes suivants, identiques ou différents : un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe phénoxy dans lequel le noyau phényle n'est pas substitué ou est substitué par un à trois restes, identiques ou différents, qui sont des restes halogéno, cyano, nitro, hydroxy, alkyle en $C_1$ à $C_4$, halogéno-alkyle en $C_1$ à $C_4$, alcoxy (en $C_1$ à $C_4$), carboxy, alkyl (en $C_1$ à $C_4$)-carbonyle, alcanoyloxy en $C_1$ à $C_4$, amino, alkyl (en$C_1$ à $C_4$)-amino ou dialkyl (en $C_1$ à $C_4$)-amino ayant, indépendamment, le nombre indiqué d'atomes de carbone dans chaque groupe alkyle ; un groupe benzoyle dont le noyau phényle n'est pas substitué ou est substitué par un à trois substituants, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; benzoyloxy-alkyle (en $C_1$ à $C_6$) ; phénylthio-alkyle (en $C_1$ à $C_6$), dont le noyau phényle n'est pas substitué ou est substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, carboxy, alcoxy (en $C_1$ à $C_4$)-carbonyle, alcanoyloxy en $C_1$ à $C_4$, amino, alkyl (en $C_1$ à $C_4$)-amino ou dialkyl (en $C_1$ à $C_4$)-amino ayant, indépendamment, le nombre indiqué d'atomes de carbone dans chaque groupe alkyle ; un groupe -CR=N-R''', dans lequel R''' représente un groupe hydroxy, alkyle en $C_1$ à $C_4$, alcoxy en $C_1$ à $C_4$, amino (-NZZ'), phénylamino, -COR, -COH ou benzoyle ; un groupe oxirannyle en $C_2$ à $C_6$ ; acétylthiosemi-carbazone ; pyrrolyle ; oxazolyle, non substitué ou substitué par un ou deux groupes méthyles ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_{10}$, non substitué ou substitué comportant alors un à quatre substituants identiques ou différents, qui sont chacun un reste halogéno, cyano, nitro, hydroxy, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou -NZZ' ;

un groupe cycloalkyle en $C_3$ à $C_8$, non substitué ou substitué, ou un groupe cyclo (en $C_3$ à $C_8$)-alkyle (en $C_1$ à $C_4$), non substitué ou substitué comportant un à quatre substituants, identiques ou différents, qui sont chacun un reste halogéno, cyano, nitro, hydroxy, alkyle en $C_1$ à $C_4$, halogénoalkyle en $C_1$ à $C_4$, alcoxy en $C_1$ à $C_4$, halogéno-alcoxy en $C_1$ à $C_4$, carboxy, alcanoyle en $C_1$ à $C_4$, alcoxy (en $C_1$ à $C_4$)-carbonyle, alcanoyloxy en $C_1$ à $C_4$ ou -NZZ' ;

un groupe alcényle en $C_2$ à $C_8$, non substitué ou substitué ou un groupe alcadiényle en $C_3$ à $C_5$, non substitué ou substitué et ayant comme substituant(s) un groupe furyle, thiényle ou pyridyle ou bien ayant un à quatre substituants, identiques ou différents, qui sont chacun un reste halogéno, cyano, nitro, hydroxy, alkyle en $C_1$ à $C_4$, cycloalkyle en $C_3$ à $C_6$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carboxyle, alcanoyloxy en $C_1$ à $C_4$ ou -NZZ' ;

un groupe cycloalcényle en $C_3$ à $C_8$, non substitué ou substitué, ou un groupe cycloalcadiényle (en $C_8$), non substitué ou substitué et ayant alors comme substituant(s) un à quatre restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$ ou -NZZ' ;

un groupe alcynyle en $C_2$ à $C_8$, non substitué ou substitué et ayant alors comme substituant(s) un à quatre restes, identiques ou différents, qui sont chacun un reste halogeno, cyano, nitro, hydroxy, alkyle en $C_1$ à $C_4$, halogéno-alkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, phényle ou NZZ' ;

un groupe phénylalkyle comportant 1 à 4 atomes de carbone dans le groupe alkyle et dans lequel le groupe alkyle n'est pas substitué ou est substitué par un à trois restes, identiques ou différents, qui

sont chacun un reste halogéno, cyano, hydroxy, alcoxy en $C_1$ à $C_4$, alcoxy (en $C_1$ à $C_4$)-carbonyle ou -NZZ', et le noyau phényle n'est pas substitué ou est substitué par un à trois restes, identiques ou différents, qui sont chacun un reste halogéno, cyano, nitro, hydroxy, alkyle en $C_1$ à $C_4$, halogénoalkyle en $C_1$ à $C_4$, cyanoalkyle en $C_1$ à $C_4$, alcoxy en $C_1$ à $C_4$, halogéno-alcoxy en $C_1$ à $C_4$, carboxy, alcoxy (en $C_1$ à $C_4$)-carbonyle, alcanoyloxy en $C_1$ à $C_4$, alcényle en $C_2$ à $C_6$, halogéno-alcényle en $C_2$ à $C_6$, alcynyle en $C_2$ à $C_6$ ou -NZZ' ; un groupe phénylalcényle ayant deux à six atomes de carbone dans le groupe alcényle, ce groupe alcényle n'étant pas substitué ou étant substitué par un à trois restes, identiques ou différents, qui sont chacun un reste halogéno, cyano, hydroxy, alkyle en $C_1$ à $C_4$, halogénoalkyle en $C_1$ à $C_4$, alcoxy (en $C_1$ à $C_4$), halogéno-alcoxy en $C_1$ à $C_4$, alcoxy (en $C_1$ à $C_4$)-carbonyle ou -NZZ', et le noyau phényle n'étant pas substitué ou étant substitué par un à trois restes, identiques ou différents, qui sont chacun un reste halogéno, cyano, nitro, hydroxy, alkyle en $C_1$ à $C_4$, halogéno-alkyle en $C_1$ à $C_4$, alcoxy en $C_1$ à $C_4$, halogénoalcoxy en $C_1$ à $C_4$, carboxy, alcoxy (en $C_1$ à $C_4$)-carbonyle, alcanoyloxy en $C_1$ à $C_4$ ou -NZZ' ;

un hétérocycle pentagonal, non substitué ou substitué, qui est choisi parmi un groupe furyle, thiényle, triazolyle, pyrrolyle, isopyrrolyle, pyrazolyle, isoimidazolyle, thiazolyle, isothiazolyle, oxazolyle et isooxazolyle, les substituants pouvant être un à trois radicaux identiques ou différents qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle ; un groupe $-RCO_2H$ ; un groupe $RCO_2R'$ ; un groupe -CONZZ' ; un groupe amino (-NZZ') ; un groupe -NZCOZ' ; un groupe alkylthio en $C_1$ à $C_6$; ou un groupe phényle, non substitué ou substitué comportant un à trois restes, identiques ou différents, qui sont chacun un reste halogéno, nitro, alkyle en $C_1$ à $C_6$, halogénoalkyle en $C_1$ à $C_6$, alcoxy en $C_1$ à $C_6$, halogéno-alcoxy en $C_1$ à $C_6$, carboxy, alcoxy (en $C_1$ à $C_4$)-carbonyle ou amino (-NZZ') ; ou bien

un hétérocycle hexagonal, non substitué ou substitué, comportant un, deux, trois ou quatre atomes d'azote et deux à cinq atomes de carbone de noyau, dans lequel les substituants peuvent être constitués par un à trois restes, identiques ou différents, qui sont chacun un reste halogéno ; nitro ; hydroxy ; alkyle en $C_1$ à $C_6$ ; alcoxy en $C_1$ à $C_6$ ; carboxy ; alcoxy (en $C_1$ à $C_6$)-carbonyle, un groupe $-RCO_2H$ ; un groupe $-RCO_2R'$ ; un groupe -CONZZ' ; un groupe amino (NZZ') ; un groupe -NZCOZ' ; un groupe alkylthio en $C_1$ à $C_6$ ; ou bien un groupe phényle non substitué ou substitué, comportant un à trois restes identiques ou différents qui sont chacun un reste halogéno, nitro, alkyle en $C_1$ à $C_6$, halogéno-alkyle en $C_1$ à $C_6$, alcoxy en $C_1$ à $C_6$, halogéno-alcoxy (en $C_1$ à $C_6$), carboxy, alcoxy (en $C_1$ à $C_4$)-carbonyle ou amino (-NZZ') ;

les symboles R et R' représentant chacun un groupe alkyle en $C_1$ à $C_6$ ; Z et Z' représentant chacun un atome d'hydrogène ou un groupe alkyle en $C_1$ à $C_4$ ; le terme "amino" désignant un groupe NZZ' ; ou un sel agronomiquement acceptable de cette hydrazine, avec un diluant, véhicule ou excipient agronomiquement acceptable, pour préparer une composition insecticide.

**2.** Utilisation selon la revendication 1, dans laquelle, dans le composé :

X et X' représentent O ou S ;
$R^1$ représente un atome d'hydrogène ; un groupe alkyle en $C_1$ à $C_4$, alcoxy (en $C_1$ à $C_4$)-alkyle (en $C_1$ à $C_4$), ayant, indépendamment, le nombre indiqué d'atomes de carbone dans chaque groupe alkyle ; un groupe méthylthiométhyle ; alcényle en $C_2$ à $C_5$ ; alcynyle en $C_2$ à $C_5$ ; ou phényl-alkyle en $C_1$ ou $C_2$, dans lequel le noyau phenyle n'est pas substitué ou est substitué par un ou deux restes halogéno, nitro, alkyle en $C_1$ à $C_4$ ou alcoxy en $C_1$ à $C_4$ ;
$R^2$ et $R^3$ sont identiques ou différents et représentent chacun un atome d'hydrogène ou un groupe alkyle en $C_1$ à $C_3$ ;
$R^4$ représente un groupe alkyle en $C_1$ à $C_3$, substitué par un à quatre atomes de fluor ; un groupe alcényle linéaire en $C_2$ à $C_4$ ; carboxyle ; alcoxy (en $C_1$ ou $C_2$)-carbonyle ; cyano ; alkyle (en $C_1$ à $C_3$) substitué par un groupe cyano ; trialkyl (en $C_1$ à $C_2$)-silyle ayant, indépendamment, le nombre indiqué d'atomes de carbone dans chaque groupe alkyle ; ou trialkyl (en $C_1$ ou $C_2$)-silylméthyle ayant, indépendamment, le nombre indiqué d'atomes de carbone dans chaque groupe alkyle ; et
A et B, identiques ou différents, représentent chacun :

un groupe naphtyle non substitué ;

un groupe phényle, non substitué ou bien substitué et dans lequel les substituants peuvent être constitués par un à trois restes différents ou identiques qui sont chacun un reste halogéno ; nitro ; cyano ; alkyle en $C_1$ à $C_4$ ; halogéno-alkyle en $C_1$ à $C_4$ ; cyano-alkyle en $C_1$ à $C_4$ ; alcoxy en $C_1$ à $C_4$ : alcoxy (en $C_1$ à $C_4$) alkyle (en $C_1$ à $C_4$) ayant, indépendamment, le nombre indiqué d'atomes de carbone dans chaque groupe alkyle ; -COZ ; carboxy ; alcoxy (en $C_1$ à $C_4$)-carbonyle ; alcanoyloxy en $C_1$ à $C_4$ ; alcényle en $C_2$ à $C_6$ ; alcynyle en $C_2$ à $C_6$ ; amino (-NZZ') ; thiocyanato ; alkylthio en $C_1$ à $C_4$ ; -CSR ; -CSH ; un groupe phényle non substitué, ou substitué ayant un ou deux restes, identiques ou différents qui sont chacun un reste halogéno, nitro, alkyle en $C_1$ à $C_4$, alcoxy en $C_1$ à $C_4$, carboxy, -NZZ' ; un groupe phénoxy dans lequel le noyau phényle n'est pas substitué ou est substitué par un ou deux restes, identiques ou différents, qui sort chacun un reste halogéno, nitro, alkyle en $C_1$ à $C_4$, alcoxy en $C_1$ à $C_4$, carboxy, -NZZ' ; ou bien, lorsque deux positions adjacentes sur le noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_8$, non substitué ou substitué, comportant un à trois restes identiques ou différents qui sont chacun un reste halogéno, cyano, alcoxy en $C_1$ à $C_4$, phényle, alcoxy (en $C_1$ à $C_4$)-carbonyle ou halogénoalcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, comportant un ou deux substituants identiques ou différents qui sont chacun un radical halogéno, alkyle en $C_1$ à $C_4$, halogéno-alkyle en $C_1$ à $C_4$, alcoxy en $C_1$ à $C_4$, alcanoyle en $C_1$ à $C_4$ ou halogéno-alcoxy en $C_1$ à $C_4$ ; un groupe alcényle en $C_2$ à $C_6$, non substitué ou substitué, comportant comme substituant(s) un reste furyle ou un à trois restes, identiques ou différents, qui sont chacun un reste alkyle en $C_1$ à $C_4$, halogéno-alkyle en $C_1$ à $C_4$, alcoxy en $C_1$ à $C_4$ ou halogénoalcoxy en $C_1$ à $C_4$ ; un groupe cycloalkényle (en $C_3$ à $C_6$) non-substitué ou substitué, ayant comme substituants un ou deux restes identiques ou différents qui sont halogéno, alkyle en $C_1$ a $C_4$, halogénoalkyle en $C_1$ à $C_4$, alcoxy en $C_1$ à $C_4$ ou halogénoalcoxy en $C_1$ à $C_4$;
un groupe alcynyle non substitué ou substitué par un groupe phényle ;

un groupe phénylalkyle ayant 1 ou 2 atomes de carbone dans le groupe alkyle, ce groupe alkyle n'étant pas substitué ou étant substitué par un ou deux restes, identiques ou différents, qui sont chacun un reste halogéno, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un groupe phénylalcényle ayant 2 ou 3 atomes de carbone dans le groupe alcényle, ce groupe alcényle n'étant pas substitué ou étant substitué par un reste halogéno, alkyle en $C_1$ à $C_4$, halogéno-alkyle en $C_1$ à $C_4$, alcoxy en $C_1$ à $C_4$ ou halogéno-alcoxy en $C_1$ à $C_4$, et le noyau phényle n'étant pas substitué ou étant substitué par un ou deux restes identiques ou différents qui sont chacun un reste halogéno, cyano, alkyle en $C_1$ à $C_4$, halogéno-alkyle en $C_1$ à $C_4$, alcoxy en $C_1$ à $C_4$ ou halogéno-alcoxy en $C_1$ à $C_4$ ;

un hétérocycle pentagonal, non substitué ou substitue, choisi parmi un groupe furyle, thiényle, triazolyle, pyrrolyle et oxazolyle, les substituants pouvant être un ou deux restes identiques ou différents qui sont chacun un reste halogéno ; nitro ; alkyle en $C_1$ à $C_4$ ; alcoxy en $C_1$ à $C_4$ ; -NZZ' ; ou bien un groupe phényle, non substitué ou substitué comportant un ou deux restes identiques ou différents qui sont chacun un reste halogéno, nitro, alkyle en $C_1$ à $C_4$, halogéno-alkyle en $C_1$ à $C_4$, alcoxy en $C_1$ à $C_4$, halogeno-alcoxy en $C_1$ à $C_4$, carboxy ou -NZZ' ; ou

un hétérocycle hexagonal non substitué ou substitué, comportant un ou deux atomes d'azote et quatre à cinq atomes de carbone de noyau, les substituants pouvant être constitués par un ou deux restes identiques ou différents qui sont chacun un reste halogéno ; nitro ; alkyle en $C_1$ à $C_4$ ; alcoxy en $C_1$ à $C_4$ ; thioalcoxy en $C_1$ à $C_4$ ; -NZZ' ; ou un groupe phényle non substitué ou substitué comportant un ou deux restes identiques ou différents qui sont chacun un reste halogéno, nitro, alkyle en $C_1$ à $C_4$, halogéno-alkyle en $C_1$ à $C_4$, alcoxy en $C_1$ à $C_4$, halogéno-alcoxy en $C_1$ à $C_4$,

carboxy ou -NZZ' ;

les symboles R et R' représentant chacun un groupe alkyle en $C_1$ à $C_4$ ; et Z et Z' représentant chacun un atome d'hydrogène ou un groupe alkyle en $C_1$ à $C_4$.

**3.** Utilisation selon la revendication 2, dans laquelle, dans le compose :

X et X' représentent O ou S ;
$R^1$ représente un atome d'hydrogène, un groupe alkyle en $C_1$ à $C_4$ ; alcoxy (en $C_1$ à $C_4$) alkyle en $C_1$ à $C_4$ comportant, indépendamment, le nombre indiqué d'atomes de carbone dans chaque groupe alkyle ; alcényle en $C_2$ à $C_5$ ; alcynyle en $C_2$ à $C_5$ ; ou benzyle dans lequel le noyau phényle n'est pas substitué ou est substitué par un reste halogéno, nitro, alkyle en $C_1$ à $C_4$ ou alcoxy en $C_1$ à $C_4$ ;
$R^2$ et $R^3$, identiques ou différents, représentent chacun un atome d'hydrogène ou un groupe alkyle en $C_1$ ou $C_2$ ;
$R^4$ représente un groupe alkyle en $C_1$ à $C_3$, substitué par un à quatre restes fluoro ; un groupe alcényle linéaire en $C_2$ à $C_4$ ; un groupe méthoxycarbonyle ; cyano ; alkyle en $C_1$ ou $C_2$ à substituant(s) cyano ; trialkyle (en $C_1$ ou $C_2$) silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; à la condition que $R^2$ et $R^3$ représentent chacun un groupe alkyle lorsque $R^4$ représente un groupe méthoxycarbonyle ; et
A et B sont identiques ou différents et représentent chacun :
un groupe naphtyle non substitué ;

un groupe phényle, non substitué ou substitué, comportant un à trois restes identiques ou différents, qui sont chacun un reste halogéno ; nitro ; cyano ; alkyle en $C_1$ à $C_4$ ; halogéno-alkyle en $C_1$ à $C_4$ ; cyano-alkyle en $C_1$ à $C_4$ ; alcoxy en $C_1$ à $C_4$ ; alcoxy (en $C_1$ à $C_4$)-alkyle (en $C_1$ à $C_4$) ayant, indépendamment, le nombre indiqué d'atomes de carbone dans chaque groupe alkyle ; -COZ ; alcoxy (en $C_1$ à $C_4$)-carbonyle ; alcanoyloxy (en $C_1$ à $C_4$) ; thiocyanato ; -NZZ' ; alkylthio en $C_1$ à $C_4$ ; -CSZ ; $-CS_2Z$ ; un groupe phényle non substitué ou substitué ccmportant un ou deux restes, identiques ou différents, qui sont chacun un reste halogéno, nitro, alkyle en $C_1$ à $C_4$, alcoxy en $C_1$ à $C_4$, carboxy, -NZZ' ; ou bien un groupe phénoxy dans lequel le noyau phényle n'est pas substitué ou est substitué par un ou deux restes identiques ou différents qui sont chacun un reste halogéno, nitro, alkyle en $C_1$ à $C_4$, alcoxy en $C_1$ à $C_4$, carboxy, -NZZ' ou bien, lorsque deux positions adjacentes du noyau phényle sont substituées par des groupes alcoxy, ces groupes peuvent être reliés pour former, avec les atomes de carbone auxquels ils sont fixés, un noyau hétérocyclique dioxolano ou dioxano pentagonal ou hexagonal ;

un groupe alkyle en $C_1$ à $C_6$, non substitué ou substitué et comportant alors un à trois restes identiques ou différents qui sont chacun un reste halogéno, cyano, nitro, carboxy, alcoxy en $C_1$ à $C_4$, phényle, alcoxy (en $C_1$ à $C_4$)-carbonyle ou halogéno-alcoxy (en $C_1$ à $C_4$) ;

un groupe cycloalkyle en $C_3$ à $C_6$, non substitué ou substitué, ou un groupe cycloalkyl (en $C_3$ à $C_6$)-alkyle (en $C_1$ à $C_4$), non substitué ou substitué, dans lesquels le substituant est un reste halogéno, alkyle en $C_1$ à $C_4$, alcanoyle en $C_1$ à $C_4$, alcoxy en $C_1$ à $C_4$ ou halogéno-alcoxy en $C_1$ à $C_4$ ;

un groupe alcényle en $C_2$ à $C_6$, non substitué ou substitué et comportant alors un ou deux restes, identiques ou différents, qui sont chacun un reste halogéno, alkyle en $C_1$ à $C_4$, alcoxy en $C_1$ à $C_4$ ou halogéno-alcoxy en $C_1$ à $C_4$ ;

un groupe cycloalcényle en $C_4$ à $C_6$, non substitué, ou substitué et dans lequel le substituant est un reste halogéno, alkyle en $C_1$ à $C_4$, alcoxy en $C_1$ à $C_4$ ou halogéno-alcoxy en $C_1$ à $C_4$ ;

un groupe alcynyle, non substitue ou substitué par un groupe phényle ;

un groupe benzyle, dans lequel le noyau phényle n'est pas substitué ou est substitué par un ou deux restes, identiques ou différents, qui sont chacun un reste halogéno, méthyle ou éthyle ;

un hétérocycle pentagonal, choisi parmi un groupe furyle, thienyle, pyrrolyle et oxazolyle, non substitué ou bien substitué et dans lequel les substituants peuvent être un ou deux restes, identiques ou différents, qui sont chacun un reste halogéno, nitro, alkyle en $C_1$ à $C_4$ ou alcoxy en $C_1$ à $C_4$ ; ou

un hétérocycle hexagonal comportant un ou deux atomes d'azote et quatre ou cinq atomes de carbone de noyau, l'hétérocycle n'étant pas substitué ou étant substitué, les substituants pouvant être un ou deux restes identiques ou différents qui sont chacun un reste halogéno, nitro, alkyle en $C_1$ à $C_4$, alcoxy en $C_1$ à $C_4$ ou thio-alcoxy en $C_1$ à $C_4$ ;

et Z et Z' representent chacun un atome d'hydrogène ou un groupe alkyle en $C_1$ à $C_4$.

**4.** Utilisation selon la revendication 3, dans laquelle, dans le composé :

X et X' représentent O ;
$R^1$ représente un atome d'hydrogène ; un groupe méthyle, éthyle, alcoxy (en $C_1$ ou $C_2$) alkyle (en $C_1$ ou $C_2$) ayant, indépendamment, le nombre indiqué d'atomes de carbone dans chaque groupe alkyle ; alcényle en $C_2$ à $C_5$ ; alcynyle en $C_2$ à $C_5$ ; ou benzyle, dans lequel le noyau phényle n'est pas substitué ou est substitué par un reste halogéno ;
$R^2$ et $R^3$, identiques ou différents, représentent chacun un atome d'hydrogène ou un groupe alkyle en $C_1$ ou $C_2$ ;
$R^4$ représente un groupe alkyle en $C_1$ ou $C_2$, substitué par un à quatre atomes de fluor ; un groupe alcényle linéaire en $C_2$ à $C_4$ ; cyano ; alkyle en $C_1$ ou $C_2$ à substituant(s) cyano ; trialkyl (en $C_1$ ou $C_2$) silyle ayant, indépendamment, le nombre indiqué d'atomes de carbone dans chaque groupe alkyle ; ou trialkyl (en $C_1$ ou $C_2$) silylméthyle ayant, indépendamment, le nombre indiqué d'atomes de carbone dans chaque groupe alkyle ; et
A et B sont identiques ou différents et représentent chacun un groupe phényle ou phényle substitué, dans lequel les substituants peuvent être constitués par un à trois restes, identiques ou différents, qui sont chacun un reste halogéno, nitro, alkyle en $C_1$ à $C_4$, alcoxy en $C_1$ à $C_4$ ou halogéno-alkyle en $C_1$ à $C_4$ ;

un groupe alkyle en $C_1$ à $C_6$, non substitué ou substitué, ayant comme substituant(s) un ou deux restes identiques ou différents qui sont chacun un reste halogéno, phényle ou cyano ;

un groupe cycloalkyle en $C_4$ à $C_6$, qui n'est pas substitué ;

un groupe alcényle en $C_2$ à $C_6$, non substitué ou substitué et ayant alors comme substituant(s) un à trois restes, identiques ou différents, qui sont chacun un reste halogéno ou alkyle en $C_1$ à $C_4$ ;

un groupe cycloalcényle en $C_4$ à $C_6$, non substitué ; un groupe phénylalkyle ayant un ou deux atomes de carbone dans le groupe alkyle et dont le noyau phényle n'est pas substitué ou est substitué par un ou deux restes identiques ou différents qui sont chacun un reste halogéno, alkyle en $C_1$ à $C_4$ ou alcoxy en $C_1$ à $C_4$ ;

un groupe pyridyle ou 2,5-pyrazinyle, non substitué ou substitué, dans lesquels le substituant peut être un reste halogéno, nitro, alkyle en $C_1$ à $C_4$, alcoxy en $C_1$ à $C_4$ ou thioalcoxy en $C_1$ à $C_4$ ; ou

un groupe furyle ou thiényle non substitué ou un groupe pyrrolyle non substitué ou substitué, dans lesquels le substituant peut être un groupe alkyle en $C_1$ à $C_4$.

**5.** Utilisation selon la revendication 4, dans laquelle, dans le composé :
X et X' représentent O ;
$R^1$ représente un atome d'hydrogène, un groupe méthyle ; méthoxyméthyle ; alcényle en $C_2$ à $C_4$ ; alcynyle en $C_2$ à $C_5$ ; benzyle ou 4-halogénobenzyle ;
$R^2$ et $R^3$ sont identiques ou différents et représentent chacun un atome d'hydrogène ou un groupe méthyle ou éthyle ;
$R^4$ représente un groupe trifluorométhyle ; 2,2,2-trifluoroéthyle ; alcényle linéaire en $C_2$ à $C_4$ ; cyano

; cyanométhyle ; trialkyl (en $C_1$ ou $C_2$) silyle ayant, indépendamment, le nombre indiqué d'atomes de carbone dans chaque groupe alkyle ; ou triméthylsilylméthyle ; et
A et B, identiques ou différents, représentent chacun un groupe phényle ou un groupe phényle substitué dont les substituants peuvent être constitués par un, deux ou trois restes identiques ou différents qui sont chacun

un reste chloro, fluoro, bromo, iodo, nitro, méthyle, éthyle, méthoxy ou trifluorométhyle ;

un groupe alcényle en $C_2$ ou $C_3$, substitué, comportant un à trois restes identiques ou différents qui sont chacun un reste chloro, bromo, méthyle ou éthyle ;
un groupe cyclohéxényle ;

un groupe benzyle ;

un groupe pyridyle ou 2,5-pyrazinyle, non substitués ou substitués dans lesquels le substituant peut être un reste halogéno, méthyle, éthyle ou méthoxy ; ou bien

un groupe furyle ou thiényle, non substitués ou un groupe pyrrolyle non substitué ou substitué dans lequel le substituant peut être un reste éthyle ou méthyle.

**6.** Utilisation selon la revendication 2, dans laquelle, dans le composé :
X et X' représentent chacun O ;
$R^1$ représente un atome d'hydrogène ou un groupe méthyle ;
$R^2$ et $R^3$ représentent chacun un atome d'hydrogène ou un groupe alkyle en $C_1$ à $C_3$ ;
$R^4$ représente un groupe trifluorométhyle, 2,2,2-trifluoroéthyle, cyano, cyanométhyle, alcényle linéaire en $C_2$ à $C_4$, carboxyle, méthoxycarbonyle, trialkyl (en $C_1$ ou $C_2$) silyle ayant, indépendamment, le nombre indiqué d'atomes de carbone dans chaque groupe alkyle ou trialkyl (en $C_1$ ou $C_2$) silylméthyle ayant, indépendamment, le nombre indiqué d'atomes de carbone dans chaque groupe alkyle ; et
A et B, identiques ou différents, représentent chacun un groupe thiényle ou phényle non substitué ou bien phényle substitué dans lequel les substituants peuvent être constitués par un à trois restes, identiques ou différents, qui sont chacun un reste chloro, fluoro, nitro, méthyle, ethyle ou propyle.

**7.** Utilisation selon la revendication 6, dans laquelle, dans le composé :
X et X' représentent chacun O ;
$R^1$ représente un atome d'hydrogène ;
$R^2$ et $R^3$ représentent chacun un atome d'hydrogène ou un groupe méthyle ou éthyle ;
$R^4$ représente un groupe trifluoromethyle, cyano, allyle, méthoxycarbonyle ou triméthylsilyle ; et
A et B, identiques ou différents, représentent chacun un groupe pnényle non substitué ou bien substitué et comportant un ou deux restes identiques ou différents qui sont chacun un reste chloro, fluoro, nitro, méthyle et éthyle ; ou bien un groupe thiényle non substitué.

**8.** Utilisation selon la revendication 7, dans laquelle, dans le composé :
X et X' représentent O ;
$R^1$ représente un atome d'hydrogène ; et
$R^2$ représente un atome d'hydrogène, $R^3$ représente un groupe méthyle, $R^4$ représente un groupe trifluorométhyle, et A et B, identiques ou différents, représentent chacun un groupe phényle, non substitué ou substitué, dans lequel les substituants peuvent être un ou deux restes, identiques ou différents, qui sont chacun un reste chloro, méthyle ou nitro ; ou bien
$R^2$ et $R^3$, identiques ou différents, représentent chacun un groupe méthyle ou éthyle, $R^4$ représente un groupe cyano, et A et B, identiques ou différents, représentent chacun un groupe phényle non substitué ou bien substitué et dans lequel les substituants peuvent être formés par un ou deux restes, identiques ou différents, chaque reste étant un reste chloro, fluoro, méthyle, éthyle ou nitro ; ou bien
$R^2$ et $R^3$ représentent chacun un groupe méthyle, $R^4$ repréprésente un groupe allyle, et A et B, identiques ou différents, représentent chacun un groupe phényle non substitué ou un groupe phényle substitué par un reste méthyle ; ou bien
$R^2$ et $R^3$ représentent chacun un atome d'hydrogène, $R^4$ représente un groupe triméthylsilyle, et A

et B, identiques ou différents, représentent chacun un groupe phényle substitué dans lequel les substituants peuvent être formés par un ou deux restes, identiques ou différents, chaque reste étant un reste méthyle, éthyle ou nitro.

**9.** Utilisation selon la revendication 8, dans laquelle, dans le composé :
X et X' représentent chacun O ;
$R^1$ représente un atome d'hydrogène ; et
$R^2$ représente un atome d'hydrogène, $R^3$ représente un groupe méthyle, $R^4$ représente un groupe trifluorométhyle, et A et B représentent chacun un groupe phényle non substitué, ou bien A représente un groupe 2,3-diméthylphényle et B représente un groupe 2,4-dichlorophényle, 3,5-diméthylphényle ou 2-nitro-5-méthylphényle ; ou
$R^2$ et $R^3$ représentent chacun un groupe méthyle, $R^4$ représente un groupe allyle, et A et B représentent chacun un groupe phényle non substitué ou bien chacun un groupe 3-méthyl-phényle ; ou bien
$R^2$ et $R^3$ représentent chacun un atome d'hydrogène, $R^4$ représente un groupe triméthylsilyle, et A représente un groupe 4-éthylphényle et B représente un groupe 2-nitro-5-méthylphényle, ou bien A et B représentent chacun un groupe 2-méthylphényle ou bien chacun un groupe 2-nitrophényle.

**10.** Utilisation selon la revendication 8, dans laquelle, dans le composé :
X et X' représentent chacun O ;
$R^1$ représente un atome d'hydrogène ;
$R^2$ et $R^3$ représentent chacun un groupe méthyle ;
$R^4$ représente un groupe cyano ; et
A et B, identiques ou différents, représentent chacun un groupe phényle non substitué ou bien un groupe phényle substitué dans lequel les substituants peuvent être constitués par un ou deux restes, identiques ou différents, dont chacun est un reste chloro, fluoro, méthyle, éthyle ou nitro.

**11.** Utilisation selon l'une quelconque des revendications précédentes du composé pour préparer une composition contenant ledit composé insecticide présent en une quantité de 0,0001 à 99 % en poids de la composition.

**12.** Utilisation selon la revendication 11 du composé pour préparer une composition dans laquelle la quantité dudit composé insecticide va de 0,01 à 99 % du poids de la composition.

**13.** Utilisation d'un composé tel que défini dans l'une quelconque des revendications 1 à 10, éventuellement dans une composition avec un diluant, véhicule ou excipient agronomiquement acceptables pour maîtriser des insectes, par mise en contact desdits insectes avec une quantité, efficace du point de vue insecticide, du composé ou de la composition.

**14.** Procédé mécanique pour améliorer la valeur commerciale et/ou le rendement commercial ou "profit" de récoltesvendables ou marchandes provenant de plantes dont la croissance est affectée ou risque d'être affectée par des insectes, ce procédé comprenant (1) le chargement d'introduction dans un récipient, un dispositif pour fumigation ou un dispositif de dissémination mécanique, d'un composé insecticide ou d'un sel de ce composé, tel que défini dans l'une quelconque des revendications 1 à 10, éventuellement en mélange avec un diluant, véhicule ou excipient agronomiquement acceptable ; (2) l'utilisation du récipient, du dispositif de fumigation ou du dispositif de dissémination mécanique pour appliquer le composé insecticide ou son sel, sous forme de granules, de poudre fine, de fumée, de vapeur ou de préparation liquide contenant du surfactif, à des plantes en cours de croissance ou à un milieu de croissance et de culture dans lequel on cultive les plantes ou dans lequel les plantes doivent être cultivées, ou sur les insectes eux-mêmes ; (3) le réglage de la dose de l'ingrédient actif, pendant cette étape d'application, de sorte que le taux d'application du composé à activité insecticide soit suffisant pour combattre les insectes mais ne suffise pas pour provoquer un effet adverse inacceptable sur les plantes de récolte, cultivées ou devant être cultivées dans la zone ainsi traitée.

**15.** Utilisation ou procédé selon la revendication 13 ou 14, dans lequel ou dans laquelle on applique aux insectes ou aux plantes en cours de croissance ou à une zone dans laquelle les plantes doivent être cultivées, ledit composé ou ladite composition en une quantité allant de 10 g à 10 kg du composé par hectare.

**16.** Utilisation ou procédé selon la revendication 13, 14 ou 15, dans lequel ou dans laquelle le taux d'application va de 100 g à 5 kg du composé par hectare.

**17.** Utilisation ou procédé selon l'une quelconque des revendications 13 à 16, dans lequel ou dans laquelle les insectes appartiennent à l'ordre des Lepidoptères ou à celui des Coléoptères.

## Ansprüche

**1.** Insektizid wirkende, substituierte Hydrazin-Verbindung mit der Formel:

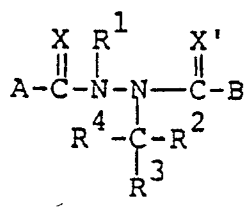

(I),

wobei

X und X' gleich oder unterschiedlich die Bedeutung O, S oder NR haben;

$R^1$ bedeutet:

Wasserstoff; $(C_1\text{-}C_6)$-Alkyl; $(C^1\text{-}C^6)$-Alkoxy-$(C_1\text{-}C_6)$ -alkyl mit unabhängig der angegebenen Anzahl an Kohlenstoffatomen in jeder Alkylgruppe; $(C_1\text{-}C_6)$-Alkythio-$(C_1\text{-}C_6)$ alkyl mit unabhängig der angegebenen Anzahl von Kohlenstoffatomen in jeder Alkylgruppe; $(C_2\text{-}C_6)$-Alkenyl; $(C_2\text{-}C_6)$-Alkynyl; oder Phen-$(C_1\text{-}C_4)$-Alkyl, wobei der Phenylring unsubstituiert ist oder gleich oder unterschiedlich substituiert ist mit eins bis drei aus Halo, Cyano, Nitro, Hydroxy, $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, Carboxy, $(C_1\text{-}C_4)$ -Alkoxycarbonyl, und $(C_1\text{-}C_4)$ -Alkanoyloxy oder NZZ';

$R^2$ und $R^3$ gleich oder unterschiedlich Wasserstoff oder $(C_1\text{-}C_4)$ -Alkyl bedeuten;

$R_4$ bedeutet:

$(C_1\text{-}C_4)$-Alkyl substituiert mit 1 bis 4 Fluor; geradkettiges $(C_2\text{-}C_4)$-Alkenyl; Carboxyl; $(C_1\text{-}C_3)$-Alkoxycarbonyl; Cyano; cyano-substituiertes $(C_1\text{-}C_4)$-Alkyl; Tri$(C_1\text{-}C_4)$-alkylsilyl mit unabhängig der angegebenen Anzahl an Kohlenstoffatomen in jeder Alkylgruppe; oder Tri$(C_1\text{-}C_2)$-alkylsilylmethyl mit unabhängig der angegebenen Anzahl an Kohlenstoffatomen in jeder Alkylgruppe; mit der Maßgabe, daß sowohl $R^2$ als auch $R^3$ Alkyl sind, wenn $R^4$ Carboxyl oder Alkoxycarbonyl ist; und

A und B bedeuten:

gleich oder unterschiedlich unsubstituiertes oder substituiertes Naphthyl, wobei die Substituenten gleich oder unterschiedlich eins bis drei aus Halo; Nitro; $(C_1\text{-}C_4)$-Alkoxy; $(C_1\text{-}C_4)$-Alkyl oder NZZ' sein können;

unsubstituiertes oder substituiertes Phenyl, wobei die Substituenten sein können gleich oder unterschiedlich von eins bis fünf aus Halo; Nitroso; Nitro; Cyano; Hydroxy; $(C_1\text{-}C_6)$-Alkyl; Halo$(C_1\text{-}C_6)$-alkyl; Cyano-$(C_1\text{-}C_6)$-alkyl; Hydroxy$(C_1\text{-}C_6)$-alkyl; $(C_1\text{-}C_6)$-Alkoxy; Halo-$(C_1\text{-}C_6)$-alkoxy; $(C_1\text{-}C_6)$-Alkoxy-$(C_1\text{-}C_6)$-alkyl mit unabhängig der angegebenen Anzahl von Kohlenstoffatomen in jeder Alkylgruppe; $(C_1\text{-}C_6)$ -Alkoxy$(C_1\text{-}C_6)$ -alkoxy mit unabhängig der angegebenen Anzahl von Kohlenstoffatomen in jeder Alkylgruppe; - ORSR'-Gruppe; $-OCO_2R$-Gruppe; $-OCO_2H$-Gruppe; $(C_1\text{-}C_6)$ -Alkanoyloxy-$(C_1\text{-}C_6)$-alkyl mit unabhängig der angegebenen Anzahl von Kohlenstoffatomen in jeder Alkylgruppe; $(C_2\text{-}C_6)$-Alkenyl, wahlweise substituiert mit Halo, Cyano, $(C_1\text{-}C_4)$-Alkyl, Halo$(C_1\text{-}C_4)$-alkoxy, $(C_1\text{-}C_4)$-Alkylthio oder $(C_1\text{-}C_4)$-Alkoxy; $(C_3\text{-}C_5)$-Alkadienyl; $(C_2\text{-}C_6)$-Alkenyloxy; $(C_2\text{-}C_6)$-Alkenylcarbonyl; $(C_2\text{-}C_6)$-Alkenyloxycarbonyloxy; $(C_2\text{-}C_6)$-Alkvnyl, wahlweise substituiert mit Halo, Cyano, Nitro, Hydroxy, $(C_1\text{-}C_6)$-Alkoxy, Halo-$(C_1\text{-}C_4)$-alkyl, $(C_1\text{-}C_3)$-Alkylthio oder $(C_1\text{-}C_4)$-Alkyl; Carboxy; $-RCO_2R'$-Gruppe; -COR; COH; Halo- $(C_1\text{-}C_6)$-Alkylcarbonyl; $-CO_2R$-Gruppe; $(C_1\text{-}C_6)$-Haloalkoxycarbonyl; -OCOR-Gruppe; $-0RC0_2R^1$-Gruppe; Amino (-NZZ'); Amino substituiert mit Hydroxy, $(C_1\text{-}C_4)$-Alkoxy oder $(C_1\text{-}C_4)$-Alkylthio; -CONZZ'Gruppe; $(C_2\text{-}C_6)$-Alkenylcarbonylamino; Hydroxy$(C_1\text{-}C_6)$-alkylaminocarbonyl; -0C0NZZ'-Gruppe; -NZC0Z'-Gruppe; $-NZC0_2Z'$-Gruppe, Thiocyanato; Isothiocyanato; $(C_1\text{-}C_6)$-Tiocyanatoalkyl; $(C_1\text{-}C_6)$-Al-kylthio; Halo-$(C_1\text{-}C_6)$-alkylthio; -S(0)Z-Gruppe; $-SO_2Z$-Gruppe; $-OSO_2R$-Gruppe; $-0S0_2H$-Gruppe; $-SO_2NZZ'$-Gruppe, -CSR-Gruppe; -CSH-Gruppe; -SCOR-Gruppe; -SCOH-Gruppe; -NSCSZ'-Gruppe; unsubstituiertes oder substituiertes Phenyl mit gleich oder unterschiedlich ein bis drei aus Halo, Cyano, Nitro, Hydroxy, ($C_1$-$C_4$)-Alkyl, Halo-($C_1$-$C_4$)-alkyl, ($C_1$-$C_4$) -Alkoxy, Carboxy, ($C_1$-$C_4$)-Alkoxycarbonyl, ($C_1$-$C_4$)-Alkanoyloxy, Amino, ($C_1$-$C_4$) 3-Alkylamino oder Di ($C_1$-$C_4$)-alkylamino mit unabhängig der angegebenen Anzahl von Kohlenstoffatomen in jeder Alkylgruppe; Phenoxy, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$)-Alkoxycarbonyl, ($C_1$-$C_4$)-Alkanoyloxy, Amino, ($C_1$-$C_4$)-Alkylamino oder Di ($C_1$-$C_4$)-Alkylamino mit unabhängig der angegebenen Anzahl von Kohlenstoffatomen in jeder Alkylgruppe; Benzoyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$) -Alkoxycarbonyl, ($C_1$-$C_4$)-Alkanoyloxy, Amino, ($C_1$-$C_4$ )-Alkylamino oder Di ($C_1$-$C_4$)-Alkylamino mit unabhängig der angegebenen Anzahl von Kohlenstoffatomen in jeder Alkylgruppe; Benzoyloxy($C_1$-$C_6$)-alkyl; Phenylthio-($C_1$-$C_6$)-alkyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$) -Alkoxycarbonyl, ($C_1$-$C_4$)-Alkanoyloxy, Amino, ($C_1$-$C_4$)-Alkylamino oder Di ($C_1$-$C_4$)-Alkylamino mit unabhängig der angegebenen Anzahl von Kohlenstoffatomen in jeder Alkylgruppe; -CR=N-R'"-Gruppe, wobei R'" die Bedeutung Hydroxy, ($C_1$-$C_4$)-Alkyl, ($C_1$-$C_4$)-Alkoxy, Amino (-NZZ'), Phenylamino, -COR, -COH oder Benzoyl hat; ($C_2$-$C_6$)-Oxiranyl; Acetylthiosemicarbazon; Pyrrolyl; Oxazolyl unsubstituiert oder substituiert mit einer oder zwei Methylgruppen; oder, wenn zwei benachbarte Positionen am Phenylring mit Alkoxygruppen substituiert sind, diese Gruppen verbunden sind, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes ($C_1$-$C_{10}$) -Alkyl mit gleich oder unterschiedlich eins bis vier aus Halo, Cyano, Nitro, Hydroxy, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, Phenyl, oder -NZZ';

unsubstituiertes oder substituiertes ($C_3$-$C_8$)-Cycloalkyl oder unsubstituiertes oder substituiertes ($C_3$-$C_8$ )-Cyclo($C_1$-$C_4$ )-alkyl mit gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkanoyl, ($C_1$-$C_4$ )-Alkoxycarbonyl, ($C_1$-$C_4$)-Alkanoyloxy, Phenyl oder -NZZ';

unsubstituiertes oder substituiertes ($C_2$-$C_8$)-Alkenyl oder unsubstituiertes oder substituiertes ($C_3$-$C_5$ )-Alkadienyl, welche als Substituenten ein Furyl-, Thienyl- oder Pyridyl oder gleich oder unterschiedlich eins bis vier aus Halo, Cyano, Nitro, Hydroxy, ($C_1$-$C_4$)-Alkyl, ($C_3$-$C_6$)-Cycloalkyl, Halo-($C_1$-$C_4$)-alkyl, ($C_1$-$C_4$)-Alkoxy, Halo-($C_1$-$C_4$)alkoxy, Carboxy, ($C_1$-$C_4$ )-Alkoxycarbonyl, ($C_1$-$C_4$ )-Alkanoyloxy oder -NZZ' haben;

unsubstituiertes oder substituiertes ($C_3$-$C_8$)-Cycloalkenyl oder unsubstituiertes oder substituiertes (Ca )-Cycloalkadienyl, welche als Substituenten gleich oder unterschiedlich eins bis vier aus Halo, Cyano, Nitro, Hydroxy, ($C_1$-$C_4$)-Alkyl, Halo($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$) -Alkanoyloxy oder -NZZ' haben;

unsubstituiertes oder substituiertes ($C_2$-$C_8$)-Alkynyl, welches als Substituenten gleich oder unterschiedlich eins bis vier aus Halo, Cyano, Nitro, Hydroxy, ($C_1$-$C_4$) -Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, Phenyl oder -NZZ' haben;

Phenylalkyl mit eins bis vier Kohlenstoffatomen in der Alkylgruppe, wobei die Alkylgruppe unsubstituiert ist oder gleich oder unterschiedlich substituiert ist mit eins bis drei aus Halo, Cyano, Hydroxy, ($C_1$-$C_4$)-Alkoxy, ($C_1$-$C_4$)-Alkoxycarbonyl oder -NZZ', und wobei der Phenylring unsubstituiert ist oder gleich oder unterschiedlich substituiert ist mit eins bis drei aus Halo, Cyano, Nitro, Hydroxy ($C_1$-$C_4$)-Alkyl, Halo-($C_1$-$C_4$)-alkyl, Cyano-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, ($C_2$-$C_6$)-Alkenyl, Halo-($C_2$-$C_6$)-alkenyl, ($C_2$-$C_6$)-Al-kenyl, oder -NZZ';

Phenalkenyl mit zwei bis sechs Kohlenstoffatomen in der Alkenylgruppe, wobei die Alkenylgruppe

unsubstituiert ist oder gleich oder unterschiedlich substituiert ist mit eins bis drei aus Halo, Cyano, Hydroxy, $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, $(C_1\text{-}C_4)$-Alk-oxycarbonyl oder -NZZ', und wobei der Phenylring unsubstituiert ist oder gleich oder unterschiedlich substituiert ist mit eins bis drei aus Halo, Cyano, Nitro, Hydroxy $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, Carboxy, $(C_1\text{-}C_4)$-Alkoxycarbonyl, $(C_1\text{-}C_4)$-Alkanoyloxy oder -NZZ';

unsubstituierter oder substituierter fünfgliedriger Heterocyclus ausgewählt aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl, wobei die Substituenten gleich oder unterschiedlich eins bis drei sein können aus Halo; Nitro; Hydroxy; $(C_1\text{-}C_6)$ -Alkyl; $(C_1\text{-}C_6)$-Alk-oxy; Carboxy; $(C_1\text{-}C_6)$-Alkoxycarbonyl; $-RCO_2H$-Gruppe; $RCO_2R'$-Gruppe; -CONZZ`-Gruppe; Amino (-NZZ'); -NZCOZ'-Gruppe; $(C_1\text{-}C_6)$-Alkylthio; oder unsubstituiertes oder substituiertes Phenyl mit gleich oder unterschiedlich eins bis drei aus Ha10, Nitro, $(C_1\text{-}C_6)$-Alkyl, Halo-$(C_1\text{-}C_6)$-alkyl, $(C_1\text{-}C_6)$-Alkoxy, Halo-$(C_1\text{-}C_6)$-alkoxy, Carboxy, $(C_1\text{-}C_4)$-Alkoxycarbonyl oder Amino (-NZZ'); oder

unsubstituierter oder substituierter sechsgliedriger Heterocyclus mit eins, zwei, drei oder vier Stickstoffatomen und zwei bis fünf Kohlenstoffatomen im Kern, wobei die Substituenten gleich oder unterschiedlich eins bis drei sein können aus Hal0; Nitro; Hydroxy; $(C_1\text{-}C_6)$-Alkyl; $(C_1\text{-}C_6)$-Alk-oxy; Carboxy; $(C_1\text{-}C_6)$-Alkoxycarbonyl; $-RC0_2H$-Gruppe; $RCO_2R'$-Gruppe; -CONZZ`-Gruppe; Amino (-NZZ'); -NZCOZ'-Gruppe; $(C_1\text{-}C_6)$-Alkylthio; oder unsubstituiertes oder substituiertes Phenyl mit gleich oder unterschiedlich eins bis drei aus Halo, Nitro, $(C_1\text{-}C_6)$-Alkyl, Halo-$(C_1\text{-}C_6)$-alkyl, $(C_1\text{-}C_6)$-Alkoxy, Halo-$(C_1\text{-}C_6)$-alkoxy, Carboxy, $(C_1\text{-}C_4)$-Alkoxycarbonyl oder Amino (-NZZ');

wobei R und R' die Bedeutung $(C_1\text{-}C_6)$-Alkyl haben; Z und Z' die Bedeutung Wasserstoff oder $(C_1\text{-}C_4)$-Alkyl haben; "Amino" die Bedeutung NZZ' hat; oder ein landwirtschaftlich vertretbares Salz davon.

2. Verbindung nach Anspruch 1, wobei
X und X' die Bedeutung O oder S haben;
$R^1$ bedeutet:
Wasserstoff; $(C_1\text{-}C_4)$-Alkyl; $(C_1\text{-}C_4)$-Alkoxy$(C_1\text{-}C_4)$-alkyl mit unabhängig der angegebenen Anzahl Kohlenstoffatome in jeder Alkylgruppe; Methylthiomethyl, $(C_2\text{-}C_5)$-Alkenyl; $(C_2\text{-}C_5)$-Alkynyl oder Phen-$(C_1\text{-}C_2)$-Alkyl, wobei der Phenylring unsubstituiert ist oder substituiert ist mit eins oder zwei aus Halo, Nitro, $(C_1\text{-}C_4)$-Alkyl oder $(C_1\text{-}C_4)$-Alkoxy;
$R^2$ und $R^3$ gleich oder unterschiedlich Wasserstoff oder $(C_1\text{-}C_3)$-Alkyl bedeuten;
$R^4$ bedeutet: $(C_1\text{-}C_3)$-Alkyl, substituiert mit eins bis vier Fluor; geradkettiges $(C_2\text{-}C_4)$-Alkenyl; Carboxyl; $(C_1\text{-}C_2)$-Alkoxycarbonyl; Cyano; cyanosubstituiertes $(C_1\text{-}C_3)$-Alkyl; Tri$(C_1\text{-}C_2)$-Alkylsilyl mit unabhängig der angegebenen Anzahl an Kohlenstoffatomen in jeder Alkylgruppe; oder Tri$(C_1\text{-}C_2)$-alkylsilylmethyl mit unabhängig der angegebenen Anzahl an Kohlenstoffatomen in jeder Alkylgruppe; und
A und B gleich oder unterschiedlich bedeuten:
unsubstituiertes Naphthyl;

unsubstituiertes oder substituiertes Phenyl, wobei die Substituenten gleich oder unterschiedlich eins bis drei sein können aus Halo; Nitro; Cyano; $(C_1\text{-}C_4)$-Alkyl; Halo-$(C_1\text{-}C_4)$-alkyl; Cyano-$(C_1\text{-}C_4)$-alkyl; $(C_1\text{-}C_4)$-Alkoxy; $(C_1\text{-}C_4)$ -Alkoxy-$(C_1\text{-}C_4)$ -alkyl mit unabhängig der angegebenen Anzahl an Kohlenstoffatomen in jeder Alkylgruppe, -COZ; Carboxy; $(C_1\text{-}C_4)$-Alkoxycarbonyl; $(C_1\text{-}C_4)$ -Alkanoyloxy; $(C_2\text{-}C_6)$-Alkenyl; $(C_2\text{-}C_6)$-Alkynyl; Amino (-NZZ'); Thiocyanato; $(C_1\text{-}C_4)$ -Alkylthio; -CSR; -CSH; unsubstituiertes oder substituiertes Phenyl mit gleich oder unterschiedlich eins bis zwei aus Halo, Nitro, $(C_1\text{-}C_4)$-Alkyl, $(C_1\text{-}C_4)$-Alkoxy, Carboxy, -NZZ'; Phenoxy, wobei der Phenylring unsubstituiert ist oder gleich oder unterschiedlich substituiert ist mit eins oder zwei aus Halo, Nitro, $(C_1\text{-}C_4)$-Alkyl, $(C_1\text{-}C_4)$-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5-oder 6-gliedrigen heterocyclischen Dioxolano- oder Dioxanoring zu bilden;

unsubstituiertes oder substituiertes $(C_1\text{-}C_8)$-Alkyl mit gleich oder unterschiedlich eins bis drei aus

Halo, Cyano, ($C_1$-$C_4$)-Alkoxy, PhenYl, ($C_1$-$C_4$)-Alkoxycarbonyl oder Halo-($C_1$-$C_4$)-alkoxy;

unsubstituiertes oder substituiertes ($C_3$-$C_6$)-Cycloalkyl oder unsubstituiertes oder substituiertes ($C_3$-$C_6$)-Cycloalkyl-($C_1$-$C_4$) -alkyl mit gleich oder unterschiedlich eins oder zwei aus Halo, ($C_1$-$C_4$) -Alkyl, Halo-($C_1$-$C_4$)alkyl, ($C_1$-$C_4$)-Alkoxy, ($C_1$-$C_4$)-Alkanoyl oder Halo-($C_1$-$C_4$) -Alkoxy;

unsubstituiertes oder substituiertes ($C_2$-$C_6$)-Alkenyl mit einem Furyl als Substituenten oder gleich oder unterschiedlich eins bis drei aus ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder substituiertes ($C_3$-$C_6$)-Cycloalkenyl, welches als Substituenten gleich oder unterschiedlich hat eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder phenylsubstituiertes Alkynyl;

Phenalkyl mit einem oder zwei Kohlenstoffatomen in der Alkylgruppe, wobei die Alkylgruppe unsubstituiert ist oder substituiert ist mit gleich oder unterschiedlich eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)-Alkoxy oder Halo-($C_1$-$C_4$)-alkoxy;

Phenalkenyl mit zwei oder drei Kohlenstoffatomen in der Alkenylgruppe, wobei die Alkenylgruppe unsubstituiert ist oder substituiert ist mit Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)– Alkoxy oder Halo-($C_1$-$C_4$)-alkoxy;

unsubstituierter oder substituierter fünfgliedriger Heterocyclus, ausgewählt aus Furyl, Thienyl, Triazolyl, Pyrrolyl und Oxazolyl, wobei die Substituenten gleich oder unterschiedlich sein können eins oder zwei aus Halo; Nitro; ($C_1$-$C_4$)-Alkyl; ($C_1$-$C_4$)-Alkoxy; -NZZ'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NZZ'; oder

unsubstituierter oder substituierter sechsgliedriger Heterocyclus mit einem oder zwei Stickstoffatomen und vier bis fünf Kohlenstoffatome im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy; -NZZ'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NZZ';

wobei R und R' die Bedeutung ($C_{1-4}$)-Alkyl haben; Z und Z' die Bedeutung Wasserstoff oder ($C_1$-$C_4$)-Alkyl haben.

3. Verbindung nach Anspruch 2, wobei
X und X' die Bedeutung O oder S haben;
$R^1$ bedeutet:
Wasserstoff; ($C_1$-$C_4$)-Alkyl; ($C_1$-$C_4$)-Alkoxy-($C_1$-$C_4$)-alkyl mit unabhängig der angegebenen Anzahl von Kohlenstoffatome in jeder Alkylgruppe; ($C_2$-$C_5$)-Alkenyl; ($C_2$-$C_5$)-Alkynyl oder Benzyl, wobei der Phenylring unsubstituiert ist oder substituiert ist mit einem Halo, Nitro, ($C_1$-$C_4$)-Alkyl oder ($C_1$-$C_4$)-Alkoxy;
$R^2$ und $R^3$ gleich oder unterschiedlich Wasserstoff oder ($C_1$-$C_2$)-Alkyl bedeuten;
$R^4$ bedeutet:
($C_1$-$C_3$)-Alkyl, substituiert mit eins bis vier Fluor; geradkettiges ($C_2$-$C_4$)-Alkenyl; Methoxycarbonyl; Cyano; cyanosubstituiertes ($C_1C_2$)-Alkyl; Tri($C_1$-$C_2$)-alkylsilyl mit unabhängig der angegebenen Anzahl von Kohlenstoffatomen in jeder Alkylgruppe; oder Tri($C_1$-$C_2$)-alkylsilylmethyl mit unabhängig der angegebenen Anzahl von Kohlenstoffatomen in jeder Alkylgruppe; mit der Maßgabe, daß sowohl $R^2$ als auch $R^3$ Alkyl sind, wenn $R^4$ Methoxycarbonyl ist; und

A und B gleich oder unterschiedlich bedeuten:
unsubstituiertes Naphthyl;

unsubstituiertes oder substituiertes Phenyl mit gleich oder unterschiedlich eins bis drei aus Halo; Nitro; Cyano; $(C_1-C_4)$-Alkyl; Halo-$(C_1-C_4)$-alkyl; Cyano-$(C_1-C_4)$alkyl; $(C_1-C_4)$-Alkoxy; $(C_1-C_4)$-Alkoxy-$(C_1-C_4)$-alkyl mit unabhängig der angegebenen Anzahl von Kohlenstoffatomen in jeder Alkylgruppe, -COZ; $(C_1-C_4)$-Alkoxycarbonyl; $(C_1-C_4)$-Alkanoyloxy; Thiocyanat; -NZZ'; $(C_1-C_4)$-Alkylthio; -CSZ; $-CS_2Z$; unsubstituiertes oder substituiertes Phenyl mit gleich oder unterschiedlich eins oder zwei aus Halo, Nitro, $(C_1-C_4)$-Alkyl, $(C_1-C_4)$-Alkoxy, Carboxy, -NZZ'; oder Phenoxy, wobei der Phenylring unsubstituiert ist oder gleich oder unterschiedlich substituiert ist mit eins oder zwei aus Halo, Nitro, $(C_1-C_4)$-Alkyl, $(C_1-C_4)$-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes $(C_1-C_6)$-Alkyl mit gleich oder unterschiedlich eins bis drei aus Halo, Cyano, Nitro, Carboxy, $(C_1-C_4)$-Alkoxy, Phenyl, $(C_1-C_4)$-Alkoxycarbonyl oder Halo-$(C_1-C_4)$-alkoxy;

unsubstituiertes oder substituiertes $(C_3-C_6)$-Cycloalkyl oder unsubstituiertes oder substituiertes $(C_3-C_6)$-Cycloalkyl-$(C_1-C_4)$-alkyl, wobei der Substituent Halo, $(C_1-C_4)$-Alkyl, $(C_1-C_4)$-Alkanoyl, $(C_1-C_4)$-Alkoxy oder Halo-$(C_1-C_4)$-alkoxy ist;

unsubstituiertes oder substituiertes $(C_2-C_6)$-Alkenyl mit gleich oder unterschiedlich eins oder zwei aus Halo-$(C_1-C_4)$-alkyl, $(C_1-C_4)$-Alkoxy oder Halo-$(C_1-C_4)$-alkoxy;

unsubstituiertes oder substituiertes $(C_4-C_6)$-Cycloalkenyl, wobei der Substituent Halo, $(C_1-C_4)$-Alkyl, $(C_1-C_4)$-Alkoxy oder Halo-$(C_1-C_4)$-alkoxy ist;

unsubstituiertes oder phenylsubstituiertes Alkynyl;

Benzyl, wobei der Phenylring unsubstituiert ist oder gleich oder unterschiedlich substituiert ist mit eins oder zwei aus Halo, Methyl oder Ethyl;

unsubstituierter oder substituierter fünfgliedriger Heterocyclus ausgewählt aus Furyl, Thienyl, Pyrrolyl und Oxazolyl, wobei die Substituenten gleich oder unterschiedlich sein können eins oder zwei aus Halo, Nitro, $(C_1-C_4)$-Alkyl oder $(C_1-C_4)$-Alkoxy; oder

unsubstituierter oder substituierter sechsgliedriger Heterocyclus mit eins oder zwei Stickstoffatomen und vier oder fünf Kohlenstoffatome im Kern, wobei die Substituenten gleich oder unterschiedlich sein können eins oder zwei aus Halo; Nitro; $(C_1-C_4)$-Alkyl; $(C_1-C_4)$-Alkoxy oder Thio-$(C_1-C_4)$-alkoxy;

wobei Z und Z' die Bedeutung Wasserstoff oder $(C_1-C_4)$-Alkyl haben.

**4.** Verbindung nach Anspruch 3, wobei
X und X' die Bedeutung O haben;
$R^1$ bedeutet:
Wasserstoff; Methyl; Ethyl; $(C_1-C_2)$-Alkoxy-$(C_1-C_2)$-alkyl mit unabhängig der angegebenen Anzahl von Kohlenstoffatomen in jeder Alkylgruppe; $(C_2-C_5)$-Alkenyl; $(C_2-C_5)$-Alkynyl; oder Benzyl, wobei der Phenylring unsubstituiert ist oder mit Halo substituiert ist;
$R^2$ und $R^3$ gleich oder unterschiedlich Wasserstoff oder $(C_1-C_2)$-Alkyl bedeuten;
$R^4$ bedeutet:
$(C_1-C_2)$-Alkyl, substituiert mit eins bis vier Fluor; geradkettiges $(C_2-C_4)$-Alkenyl; Cyano; cyanosubstituiertes $(C_1-C_2)$-Alkyl; Tri$(C_1-C_2)$-Alkylsilyl mit unabhängig der angegebenen Anzahl an Kohlenstoffatomen in jeder Alkylgruppe; oder Tri$(C_1-C_2)$-alkylsilylmethyl mit unabhängig der angegebenen Anzahl an Kohlenstoffatomen in jeder Alkylgruppe; und
A und B bedeuten:
gleich oder unterschiedlich Phenyl oder substituiertes Phenyl, wobei die Substituenten gleich oder

unterschiedlich eins bis drei sein können aus Halo; Nitro; $(C_1-C_4)$-Alkyl; $(C_1-C_4)$-Alkoxy oder Halo-$(C_1-C_4)$-alkyl;

unsubstituiertes oder substituiertes $(C_1-C_6)$-Alkyl mit gleich oder unterschiedlich eins bis zwei aus Halo, Phenyl oder Cyano als Substituenten;

unsubstituiertes $(C_4-C_6)$-Cycloalkyl;

unsubstituiertes oder substituiertes $(C_2-C_6)$-Alkenyl mit gleich oder unterschiedlich eins bis drei aus Halo oder $(C_1-C_4)$-Alkyl als Substituenten;

unsubstituiertes $(C_4-C_6)$-Cycloalkenyl;

Phenalkyl mit eins oder zwei Kohlenstoffatomen in der Alkylgruppe, wobei der Phenylring unsubstituiert ist oder gleich oder unterschiedlich substituiert ist mit eins oder zwei aus Halo, $(C_1-C_4)$-Alkyl oder $(C_1-C_4)$-Alkoxy;

unsubstituiertes substituiertes Pyridyl oder 2,5-Pyrazinyl, wobei die Substituenten Halo, Nitro, $(C_1-C_4)$-Alkyl, $C_1-C_4)$-Alkoxy oder $C_1-C_4)$-Thioalkoxy sein können; oder

unsubstituiertes Furyl oder Thienyl oder ein unsubstituiertes oder substituiertes Pyrrolyl, wobei der Substituent $(C_1-C_4)$-Alkyl sein kann.

**5.** Verbindung nach Anspruch 4, wobei
X und X' die Bedeutung O haben;
$R^1$ bedeutet: Wasserstoff; Methyl; Methoxymethyl; $(C_2-C_4)$-Alkenyl; $(C_2-C_5)$-Alkynyl; Benzyl oder 4-Halobenzyl;
$R^2$ und $R^3$ gleich oder unterschiedlich Wasserstoff, Methyl oder Ethyl bedeuten;
$R^4$ bedeutet:
Trifluormethyl; 2,2,2-Trifluoroethyl; geradkettiges $(C_2-C_4)$-Alkenyl; Cyano; Cyanomethyl; Tri$(C_1-C_2)$-alkylsilyl mit unabhängig der angegebenen Anzahl an Kohlenstoffatomen in jeder Alkylgruppe; oder Trimethylsilylmethyl; und
A und B bedeuten:
gleich oder unterschiedlich Phenyl oder substituiertes Phenyl, wobei die Substituenten gleich oder unterschiedlich eins, zwei oder drei aus Chlor, Fluor, Brom, Iod, Nitro, Methyl, Ethyl, Methoxy oder Trifluormethyl sein können;

$(C_2-C_3)$ substituiertes Alkenyl mit gleich oder unterschiedlich eins bis drei aus Chlor, Brom, Methyl oder Ethyl;

Cyclohexenyl;

Benzyl;

unsubstituiertes oder substituiertes Pyridyl oder 2,5-Pyrazinyl, wobei der Substituent Halo, Methyl, Ethyl oder Methoxy sein kann; oder

unsubstituiertes Furyl oder Thienyl oder ein unsubstituiertes oder substituiertes Pyrrolyl, wobei der Substituent Ethyl oder Methyl sein kann.

**6.** Verbindung nach Anspruch 2, wobei
X und X' die Bedeutung O haben;
$R^1$ die Bedeutung Wasserstoff oder Methyl hat;
$R^2$ und $R^3$ die Bedeutung Wasserstoff oder $(C_1-C_3)$-Alkyl haben;
$R^4$ bedeutet:
Trifluormethyl, 2,2,2-Trifluorethyl, Cyano, Cyanomethyl, geradkettiges $(C_2-C_4)$-Alkenyl, Carboxyl, Methoxycarbonyl, Tri$(C_1-C_2)$-Alkylsilyl mit unabhängig der angegebenen Anzahl an Kohlenstoffatomen in jeder Alkylgruppe oder Tri$(C_1-C_2)$-Alkylsilylmethyl mit unabhängig der angegebenen Anzahl

an Kohlenstoffatomen in jeder Alkylgruppe; und
A und B gleich oder unterschiedlich Thienyl oder unsubstituiertes Phenyl bedeuten, wobei die Substituenten gleich oder unterschiedlich eins bis drei aus Chlor, Fluor, Nitro, Methyl, Ethyl oder Propyl sein können.

**7.** Verbindung nach Anspruch 6, wobei
X und X' die Bedeutung O haben;
$R^1$ die Bedeutung Wasserstoff hat;
$R^2$ und $R^3$ die Bedeutung Wasserstoff, Methyl oder Ethyl haben;
$R^4$ die Bedeutung Trifluormethyl, Cyano, Allyl, Methoxycarbonyl oder Trimethylsilyl hat; und
A und B bedeuten:
gleich oder unterschiedlich unsubstituiertes oder substituiertes Phenyl mit gleich oder unterschiedlich eins oder zwei aus Chlor, Fluor, Nitro, Methyl und Ethyl; oder unsubstituiertes Thienyl.

**8.** Verbindung nach Anspruch 7, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B gleich oder unterschiedlich unsubstituiertes oder substituiertes Phenyl bedeuten, wobei die Substituenten gleich oder unterschiedlich eins oder zwei aus Chlor, Methyl oder Nitro sein können; oder
$R^2$ und $R^3$ gleich oder unterschiedlich Methyl oder Ethyl bedeuten, $R^4$ die Bedeutung Cyano hat, und
A und B gleich oder unterschiedlich unsubstituiertes oder substituiertes Phenyl bedeuten, wobei die Substituenten gleich oder unterschiedlich eins oder zwei aus Chlor, Fluor, Methyl, Ethyl oder Nitro sein können; oder
$R^2$ und $R^3$ die Bedeutung Methyl haben, $R^4$ die Bedeutung Allyl hat, und A und B gleich oder unterschiedlich unsubstituiert ist oder methylsubstituiertes Phenyl bedeuten; oder
$R^2$ und $R^3$ die Bedeutung Wasserstoff haben, $R^4$ die Bedeutung Trimethylsilyl hat, und A und B gleich oder unterschiedlich substituiertes Phenyl bedeutet, wobei die Substituenten gleich oder unterschiedlich eins oder zwei aus Methyl, Ethyl oder Nitro sein können.

**9.** Verbindung nach Anspruch 8, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B die Bedeutung unsubstituiertes Phenyl haben, oder A die Bedeutung 2,3-Dimethylphenyl hat und B die Bedeutung 2,4-Dichlorphenyl, 3,5-Dimethylphenyl oder 2-Nitro-5-methylphenyl hat; oder
$R^2$ und $R^3$ die Bedeutung Methyl haben, $R^4$ die Bedeutung Allyl hat, und sowohl A als auch B die Bedeutung unsubstituiertes Phenyl oder 3-Methylphenyl haben; oder
$R^2$ und $R^3$ die Bedeutung Wasserstoff haben, $R^4$ die Bedeutung Trimethylsilyl hat, und A die Bedeutung 4-Ethylphenyl hat und B die Bedeutung 2-Nitro-5-methylphenyl hat, oder sowohl A als auch B die Bedeutung 2-Methylphenyl oder 2-Nitrophenyl haben.

**10.** Verbindung nach Anspruch 8, wobei
X und X' die Bedeutung O haben;
$R^1$ die Bedeutung Wasserstoff hat; und
$R^2$ und $R^3$ die Bedeutung Methyl haben;
$R^4$ die Bedeutung Cyano hat; und
A und B gleich oder unterschiedlich unsubstituiertes oder substituiertes Phenyl bedeutet, wobei die Substituenten gleich oder unterschiedlich eins oder zwei aus Chlor, Fluor, Methyl, Ethyl oder Nitro sein können.

**11.** Insektizide Zusammensetzung, enthaltend landwirtschaftlich vertretbaren Verdünnungs- oder Trägerstoff und eine insektizide Verbindung nach einem der vorhergehenden Ansprüche.

**12.** Zusammensetzung nach Anspruch 11, mit einem Gehalt der insektiziden Verbindung in einer Menge

von etwa 0,0001 bis 99 Gew.-% der Zusammensetzung.

13. Zusammensetzung nach Anspruch 12, wobei die Menge der insektiziden Verbindung 0,01 bis 99 Gew.-% der Zusammensetzung beträgt.

14. Verfahren zum Kontrollieren von Insekten, bei der man die Insekten mit einer insektizid wirksamen Menge einer Verbindung nach einem der Ansprüche 1 bis 10 in Kontakt bringt, wahlweise in einer Zusammensetzung nach einem der Ansprüche 11 bis 13.

15. Verfahren nach Anspruch 14, wobei man die Verbindung oder Zusammensetzung Insekten oder wachsenden Pflanzen oder einem Gebiet, in dem Pflanzen zu ziehen sind, in einer Menge von 10 g bis 10 kg pro Hektar der Verbindung zuführt.

16. Verfahren nach Anspruch 15, wobei die Applikationsrate 100 g bis 5 kg pro Hektar der Verbindung beträgt.

17. Verfahren nach einem der Ansprüche 14 bis 16, wobei die Insekten von der Ordnung Lepidoptera oder Coleoptera sind.

Patentansprüche für folgenden Vertragsstaat : AT

1. Insektizid wirkende Zusammensetzung, enthaltend eine Verbindung der Formel:

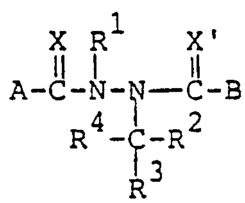

(I),

wobei

X und X' gleich oder unterschiedlich die Bedeutung O, S oder NR haben;
$R^1$ bedeutet:
Wasserstoff; $(C_1\text{-}C_6)$-Alkyl; $(C_1\text{-}C_6)$-Alkoxy-$(C_1\text{-}C_6)$-alkyl mit unabhängig der angegebenen Anzahl an Kohlenstoffatomen in jeder Alkylgruppe; $(C_1\text{-}C_6)$-Alkythio-$(C_1\text{-}C_6)$alkyl mit unabhängig der angegebenen Anzahl von Kohlenstoffatomen in jeder Alkylgruppe; $(C_2\text{-}C_6)$-Alkenyl; $(C_2\text{-}C_6)$-Alkynyl; oder Phen-$(C_1\text{-}C_4)$-Alkyl, wobei der Phenylring unsubstituiert ist oder gleich oder unterschiedlich substituiert ist mit eins bis drei aus Halo, Cyano, Nitro, Hydroxy, $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, Carboxy, $(C_1\text{-}C_4)$-Alkoxycarbonyl, und $(C_1\text{-}C_4)$-Alkanoyloxy oder NZZ';
$R^2$ und $R^3$ gleich oder unterschiedlich Wasserstoff oder $(C_1\text{-}C_4)$-Alkyl bedeuten;
$R^4$ bedeutet:
$(C_1\text{-}C_4)$-Alkyl substituiert mit 1 bis 4 Fluor; geradkettiges $(C_2\text{-}C_4)$-Alkenyl; Carboxyl; $(C_1\text{-}C_3)$-Alkoxycarbonyl; Cyano; cyano-substituiertes $(C_1\text{-}C_4)$-Alkyl; Tri$(C_1\text{-}C_4)$-alkylsilyl mit unabhängig der angegebenen Anzahl an Kohlenstoffatomen in jeder Alkylgruppe; oder Tri$(C_1\text{-}C_2)$-alkylsilylmethyl mit unabhängig der angegebenen Anzahl an Kohlenstoffatomen in jeder Alkylgruppe; mit der Maßgabe, daß sowohl $R^2$ als auch $R^3$ Alkyl sind, wenn $R^4$ Carboxyl oder Alkoxycarbonyl ist; und
A und B bedeuten:
gleich oder unterschiedlich unsubstituiertes oder substituiertes Naphthyl, wobei die Substituenten gleich oder unterschiedlich eins bis drei aus Halo; Nitro; $(C_1\text{-}C_4)$-Alkoxy; $(C_1\text{-}C_4)$-Alkyl oder NZZ' sein können;

unsubstituiertes oder substituiertes Phenyl, wobei die Substituenten sein können gleich oder unterschiedlich von eins bis fünf aus Halo; Nitroso; Nitro; Cyano; Hydroxy; $(C_1\text{-}C_6)$-Alkyl; Halo-$(C_1\text{-}C_6)$-alkyl; Cyano-$(C_1\text{-}C_6)$-alkyl; Hydroxy-$(C_1\text{-}C_6)$-alkyl; $(C_1\text{-}C_6)$-Alkoxy; Halo-$(C_1\text{-}C_6)$-alkoxy; $(C_1\text{-}C_6)$-Alkoxy-$(C_1\text{-}C_6)$-alkyl mit unabhängig der angegebenen Anzahl von Kohlenstoffatomen in jeder Alkylgruppe; $(C_1\text{-}C_6)$-Alkoxy-$(C_1\text{-}C_6)$-alkoxy mit unabhängig der angegebenen Anzahl von Kohlenstoffatomen in jeder Alkylgruppe; -ORSR'-Gruppe; $-OCO_2R$-Gruppe; $-OCO_2H$-Gruppe; $(C_1\text{-}C_6)$-Alkanoyloxy-$(C_1\text{-}C_6)$-alkyl mit unabhängig der angegebenen Anzahl von Kohlenstoffatomen in jeder

Alkylgruppe; ($C_2$-$C_6$)-Alkenyl, wahlweise substituiert mit Halo, Cyano, ($C_1$-$C_4$)-Alkyl, Halo-($C_1$-$C_4$)-alkoxy, ($C_1$-$C_4$)-Alkylthio oder ($C_1$-$C_4$)-Alkoxy; ($C_3$-$C_5$)-Alkadienyl; ($C_2$-$C_6$)-Alkenyloxy; ($C_2$-$C_6$)-Alkenylcarbonyl; ($C_2$-$C_6$)-Alkenyloxycarbonyloxy; ($C_2$-$C_6$)-Alkynyl, wahlweise substituiert mit Halo, Cyano, Nitro, Hydroxy, ($C_1$-$C_4$)-Alkoxy, Halo-($C_1$-$C_4$)-alkyl, ($C_1$-$C_4$)-Alkylthio oder ($C_1$-$C_4$)-Alkyl; Carboxy; -$RCO_2R'$-Gruppe; -COR; COH; Halo-($C_1$-$C_6$)-Alkylcarbonyl; -$CO_2R$-Gruppe; ($C_1$-$C_6$)-Haloalkoxycarbonyl; -OCOR-Gruppe; -$ORCO_2R'$-Gruppe; Amino (-NZZ'); Amino substituiert mit Hydroxy, ($C_1$-$C_4$)-Alkoxy oder ($C_1$-$C_4$)-Alkylthio; -CONZZ'-Gruppe; ($C_2$-$C_6$)-Alkenylcarbonylamino; Hydroxy-($C_1$-$C_6$)-alkylaminocarbonyl; -OCONZZ'-Gruppe; -NZCOZ'-Gruppe; -$NZCO_2Z'$-Gruppe, Thiocyanato; Isothiocyanato; ($C_1$-$C_6$)-Tiocyanatoalkyl; ($C_1$-$C_6$)-Alkylthio; Halo-($C_1$-$C_6$)-alkylthio; -S(O)-Z-Gruppe; -$SO_2Z$-Gruppe; -$OSO_2R$-Gruppe; -$OSO_2H$-Gruppe; -$SO_2NZZ'$-Gruppe, -CSR-Gruppe; -CSH-Gruppe; -SCOR-Gruppe; -SCOH-Gruppe; -NSCSZ'-Gruppe; unsubstituiertes oder substituiertes Phenyl mit gleich oder unterschiedlich ein bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$)-Alkoxycarbonyl, ($C_1$-$C_4$)-Alkanoyloxy, Amino, ($C_1$-$C_4$)-Alkylamino oder Di($C_1$-$C_4$)-alkylamino mit unabhängig der angegebenen Anzahl von Kohlenstoffatomen in jeder Alkylgruppe; Phenoxy, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$)-Alkoxycarbonyl, ($C_1$-$C_4$)-Alkanoyloxy, Amino, ($C_1$-$C_4$)-Alkylamino oder Di($C_1$-$C_4$)-Alkylamino mit unabhängig der angegebenen Anzahl von Kohlenstoffatomen in jeder Alkylgruppe; Benzoyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$)-Alkoxycarbonyl, ($C_1$-$C_4$)-Alkanoyloxy, Amino, ($C_1$-$C_4$)-Alkylamino oder Di($C_1$-$C_4$)-Alkylamino mit unabhängig der angegebenen Anzahl von Kohlenstoffatomen in jeder Alkylgruppe; Benzoyloxy-($C_1$-$C_6$)-alkyl; Phenylthio-($C_1$-$C_6$)-alkyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, ($C_1$-$C_4$)-Alkoxy, Carboxy, ($C_1$-$C_4$)-Alkoxycarbonyl, ($C_1$-$C_4$)-Alkanoyloxy, Amino, ($C_1$-$C_4$)-Alkylamino oder Di($C_1$-$C_4$)-Alkylamino mit unabhängig der angegebenen Anzahl von Kohlenstoffatomen in jeder Alkylgruppe; -CR=N-R'"-Gruppe, wobei R'" die Bedeutung Hydroxy, ($C_1$-$C_4$)-Alkyl, ($C_1$-$C_4$)-Alkoxy, Amino (-NZZ'), Phenylamino, -COR, -COH oder Benzoyl hat; ($C_2$-$C_6$)-Oxiranyl; Acetylthiosemicarbazon; Pyrrolyl; Oxazolyl unsubstituiert oder substituiert mit einer oder zwei Methylgruppen; oder, wenn zwei benachbarte Positionen am Phenylring mit Alkoxygruppen substituiert sind, diese Gruppen verbunden sind, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes ($C_1$-$C_{10}$)-Alkyl mit gleich oder unterschiedlich eins bis vier aus Halo, Cyano, Nitro, Hvdroxy, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, Phenyl, oder -NZZ';

unsubstituiertes oder substituiertes ($C_3$-$C_8$)-Cycloalkyl oder unsubstituiertes oder substituiertes ($C_3$-$C_8$)-Cyclo($C_1$-$C_4$)-alkyl mit gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkanoyl, ($C_1$-$C_4$)-Alkoxycarbonyl, ($C_1$-$C_4$)-Alkanoyloxy, Phenyl oder -NZZ';

unsubstituiertes oder substituiertes ($C_2$-$C_8$)-Alkenyl oder unsubstituiertes oder substituiertes ($C_3$-$C_5$)-Alkadienyl, welche als Substituenten ein Furyl-, Thienyl- oder Pyridyl oder gleich oder unterschiedlich eins bis vier aus Halo, Cyano, Nitro, Hydroxy, ($C_1$-$C_4$)-Alkyl, ($C_3$-$C_6$)-Cycloalkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy oder -NZZ' haben;

unsubstituiertes oder substituiertes ($C_3$-$C_8$)-Cycloalkenyl oder unsubstituiertes oder substituiertes ($C_8$)-Cycloalkadienyl, welche als Substituenten gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy oder -NZZ' haben;

unsubstituiertes oder substituiertes ($C_2$-$C_8$)-Alkynyl, welches als Substituenten gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, Phenyl oder -NZZ' haben;

Phenylalkyl mit eins bis vier Kohlenstoffatomen in der Alkylgruppe, wobei die Alkylgruppe unsubstituiert ist oder gleich oder unterschiedlich substituiert ist mit eins bis drei aus Halo, Cyano, Hydroxy, ($C_1$-$C_4$)-Alkoxy, ($C_1$-$C_4$)-Alkoxycarbonyl oder -NZZ', und wobei der Phenylring unsubstituiert ist oder gleich oder unterschiedlich substituiert ist mit eins bis drei aus Halo, Cyano, Nitro, Hydroxy ($C_1$-$C_4$)-Alkyl, Halo-($C_1$-$C_4$)-alkyl, Cyano-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, ($C_2$-$C_6$)-Alkenyl, Halo-($C_2$-$C_6$)-alkenyl, ($C_2$-$C_6$)-Alkenyl, oder -NZZ';

Phenalkenyl mit zwei bis sechs Kohlenstoffatomen in der Alkenylgruppe, wobei die Alkenylgruppe unsubstituiert ist oder gleich oder unterschiedlich substituiert ist mit eins bis drei aus Halo, Cyano, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, ($C_1$-$C_4$)-Alkoxycarbonyl oder -NZZ', und wobei der Phenylring unsubstituiert ist oder gleich oder unterschiedlich substituiert ist mit eins bis drei aus Halo, Cyano, Nitro, Hydroxy ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy oder -NZZ';

unsubstituierter oder substituierter fünfgliedriger Heterocyclus ausgewählt aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl, wobei die Substituenten gleich oder unterschiedlich eins bis drei sein können aus Halo; Nitro; Hydroxy; ($C_1$-$C_6$)-Alkyl; ($C_1$-$C_6$)-Alkoxy; Carboxy; ($C_1$-$C_6$)-Alkoxycarbonyl; -$RCO_2H$-Gruppe; $RCO_2R'$-Gruppe; -CONZZ'-Gruppe; Amino (-NZZ'); -NZCOZ'-Gruppe; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl oder Amino (-NZZ'); oder

unsubstituierter oder substituierter sechsgliedriger Heterocyclus mit eins, zwei, drei oder vier Stickstoffatomen und zwei bis fünf Kohlenstoffatomen im Kern, wobei die Substituenten gleich oder unterschiedlich eins bis drei sein können aus Halo; Nitro; Hydroxy; ($C_1$-$C_6$)-Alkyl; ($C_1$-$C_6$)-Alkoxy; Carboxy; ($C_1$-$C_6$)-Alkoxycarbonyl; -$RCO_2H$-Gruppe; $RCO_2R'$-Gruppe; -CONZZ'-Gruppe; Amino (-NZZ'); -NZCOZ'-Gruppe; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl oder Amino (-NZZ');

wobei R und R' die Bedeutung ($C_{1-6}$)-Alkyl haben; Z und Z' die Bedeutung Wasserstoff oder ($C_1$-$C_4$)-Alkyl haben; "Amino" die Bedeutung NZZ' hat; oder ein landwirtschaftlich vertretbares Salz davon; zusammen mit landwirtschaftlich vertretbarem Verdünnungs- oder Trägerstoff.

**2.** Zusammensetzung nach Anspruch 1, wobei
X und X' die Bedeutung O oder S haben;
$R^1$ bedeutet:
Wasserstoff; ($C_1$-$C_4$)-Alkyl; ($C_1$-$C_4$)-Alkoxy-($C_1$-$C_4$)-alkyl mit unabhängig der angegebenen Anzahl Kohlenstoffatome in jeder Alkylgruppe; Methylthiomethyl, ($C_2$-$C_5$)-Alkenyl; ($C_2$-$C_5$)-Alkynyl oder Phen-($C_1$-$C_2$)-Alkyl, wobei der Phenylring unsubstituiert ist oder substituiert ist mit eins oder zwei aus Halo, Nitro, ($C_1$-$C_4$)-Alkyl oder ($C_1$-$C_4$)-Alkoxy;
$R^2$ und $R^3$ gleich oder unterschiedlich Wasserstoff oder ($C_1$-$C_3$)-Alkyl bedeuten;
$R^4$ bedeutet:
($C_1$-$C_3$)-Alkyl, substituiert mit eins bis vier Fluor; geradkettiges ($C_2$-$C_4$)-Alkenyl; Carboxyl; ($C_1$-$C_2$)-Alkoxycarbonyl; Cyano; cyanosubstituiertes ($C_1$-$C_3$)-Alkyl; Tri($C_1$-$C_2$)-Alkylsilyl mit unabhängig der angegebenen Anzahl an Kohlenstoffatomen in jeder Alkylgruppe; oder Tri($C_1$-$C_2$)-alkylsilylmethyl mit unabhängig der angegebenen Anzahl an Kohlenstoffatomen in jeder Alkylgruppe; und
A und B gleich oder unterschiedlich bedeuten:
unsubstituiertes Naphthyl;

unsubstituiertes oder substituiertes Phenyl, wobei die Substituenten gleich oder unterschiedlich eins bis drei sein können aus Halo; Nitro; Cyano; ($C_1$-$C_4$)-Alkyl; Halo-($C_1$-$C_4$)-alkyl; Cyano-($C_1$-$C_4$)-alkyl; ($C_1$-$C_4$)-Alkoxy; ($C_1$-$C_4$)-Alkoxy-($C_1$-$C_4$)-alkyl mit unabhängig der angegebenen Anzahl an Kohlenstoffatomen in jeder Alkylgruppe, -COZ; Carboxy; ($C_1$-$C_4$)-Alkoxycarbonyl; ($C_1$-$C_4$)-Alkanoyloxy; ($C_2$-

$C_6$)-Alkenyl; ($C_2$-$C_6$)-Alkynyl; Amino (-NZZ'); Thiocyanato; ($C_1$-$C_4$)-Alkylthio; -CSR; -CSH; unsubstituiertes oder substituiertes Phenyl mit gleich oder unterschiedlich eins bis zwei aus Halo, Nitro, ($C_1$-$C_4$)-Alkyl, ($C_1$-$C_4$)-Alkoxy, Carboxy, -NZZ'; Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes ($C_1$-$C_8$)-Alkyl mit gleich oder unterschiedlich eins bis drei aus Halo, Cyano, ($C_1$-$C_4$)-Alkoxy, Phenyl, ($C_1$-$C_4$)-Alkoxycarbonyl oder Halo-($C_1$-$C_4$)-alkoxy;

unsubstituiertes oder substituiertes ($C_3$-$C_6$)-Cycloalkyl oder unsubstituiertes oder substituiertes ($C_3$-$C_6$)-Cycloalkyl-($C_1$-$C_4$)-alkyl mit gleich oder unterschiedlich eins oder zwei aus Halo, ($C_1$-$C_4$)-Alkyl, Halo-($C_1$-$C_4$)alkyl, ($C_1$-$C_4$)-Alkoxy, ($C_1$-$C_4$)-Alkanoyl oder Halo-($C_1$-$C_4$)-Alkoxy;

unsubstituiertes oder substituiertes ($C_2$-$C_6$)-Alkenyl mit einem Furyl als Substituenten oder gleich oder unterschiedlich eins bis drei aus ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder substituiertes ($C_3$-$C_6$)-Cycloalkenyl, welches als Substituenten gleich oder unterschiedlich hat eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder phenylsubstituiertes Alkynyl;

Phenalkyl mit einem oder zwei Kohlenstoffatomen in der Alkylgruppe, wobei die Alkylgruppe unsubstituiert ist oder substituiert ist mit gleich oder unterschiedlich eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)-Alkoxy oder Halo-($C_1$-$C_4$)-alkoxy;

Phenalkenyl mit zwei oder drei Kohlenstoffatomen in der Alkenylgruppe, wobei die Alkenylgruppe unsubstituiert ist oder substituiert ist mit Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituierter oder substituierter fünfgliedriger Heterocyclus, ausgewählt aus Furyl, Thienyl, Triazolyl, Pyrrolyl und Oxazolyl, wobei die Substituenten gleich oder unterschiedlich sein können eins oder zwei aus Halo; Nitro; ($C_1$-$C_4$)-Alkyl; ($C_1$-$C_4$)-Alkoxy; -NZZ'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NZZ'; oder

unsubstituierter oder substituierter sechsgliedriger Heterocyclus mit einem oder zwei Stickstoffatomen und vier bis fünf Kohlenstoffatome im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy; -NZZ'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NZZ';

wobei R und R' die Bedeutung ($C_{1-4}$)-Alkyl haben; Z und Z' die Bedeutung Wasserstoff oder ($C_1$-$C_4$)-Alkyl haben.

**3.** Zusammensetzung nach Anspruch 2, wobei
X und X' die Bedeutung O oder S haben;
$R^1$ bedeutet:
Wasserstoff; ($C_1$-$C_4$)-Alkyl; ($C_1$-$C_4$)-Alkoxy-($C_1$-$C_4$)-alkyl mit unabhängig der angegebenen Anzahl

von Kohlenstoffatome in jeder Alkylgruppe; $(C_2-C_5)$-Alkenyl; $(C_2-C_5)$-Alkynyl oder Benzyl, wobei der Phenylring unsubstituiert ist oder substituiert ist mit einem Halo, Nitro, $(C_1-C_4)$-Alkyl oder $(C_1-C_4)$-Alkoxy;
$R^2$ und $R^3$ gleich oder unterschiedlich Wasserstoff oder $(C_1-C_2)$-Alkyl bedeuten;
$R^4$ bedeutet:
$(C_1-C_3)$-Alkyl, substituiert mit eins bis vier Fluor; geradkettiges $(C_2-C_4)$-Alkenyl; Methoxycarbonyl; Cyano; cyanosubstituiertes $(C_1C_2)$-Alkyl; Tri$(C_1-C_2)$-alkylsilyl mit unabhängig der angegebenen Anzahl von Kohlenstoffatomen in jeder Alkylgruppe; oder Tri$(C_1-C_2)$-alkylsilylmethyl mit unabhängig der angegebenen Anzahl von Kohlenstoffatomen in jeder Alkylgruppe; mit der Maßgabe, daß sowohl $R^2$ als auch $R^3$ Alkyl sind, wenn $R^4$ Methoxycarbonyl ist; und
A und B gleich oder unterschiedlich bedeuten:
unsubstituiertes Naphthyl;

unsubstituiertes oder substituiertes Phenyl mit gleich oder unterschiedlich eins bis drei aus Halo; Nitro; Cyano; $(C_1-C_4)$-Alkyl; Halo-$(C_1-C_4)$-alkyl; Cyano-$(C_1-C_4)$-alkyl; $(C_1-C_4)$-Alkoxy; $(C_1-C_4)$-Alkoxy-$(C_1-C_4)$-alkyl mit unabhängig der angegebenen Anzahl von Kohlenstoffatomen in jeder Alkylgruppe, -COZ; $(C_1-C_4)$-Alkoxycarbonyl; $(C_1-C_4)$-Alkanoyloxy; Thiocyanat; -NZZ'; $(C_1-C_4)$-Alkylthio; -CSZ; $-CS_2Z$; unsubstituiertes oder substituiertes Phenyl mit gleich oder unterschiedlich eins oder zwei aus Halo, Nitro, $(C_1-C_4)$-Alkyl, $(C_1-C_4)$-Alkoxy, Carboxy, -NZZ'; oder Phenoxy, wobei der Phenylring unsubstituiert ist oder gleich oder unterschiedlich substituiert ist mit eins oder zwei aus Halo, Nitro, $(C_1-C_4)$-Alkyl, $(C_1-C_4)$-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes $(C_1-C_6)$-Alkyl mit gleich oder unterschiedlich eins bis drei aus Halo, Cyano, Nitro, Carboxy, $(C_1-C_4)$-Alkoxy, Phenyl, $(C_1-C_4)$-Alkoxycarbonyl oder Halo-$(C_1-C_4)$-alkoxy;

unsubstituiertes oder substituiertes $(C_3-C_6)$-Cycloalkyl oder unsubstituiertes oder substituiertes $(C_3-C_6)$-Cycloalkyl-$(C_1-C_4)$-alkyl, wobei der Substituent Halo, $(C_1-C_4)$-Alkyl, $(C_1-C_4)$-Alkanoyl, $(C_1-C_4)$-Alkoxy oder Halo-$(C_1-C_4)$-alkoxy ist;

unsubstituiertes oder substituiertes $(C_2-C_6)$-Alkenyl mit gleich oder terschiedlich eins oder zwei aus Halo-$(C_1-C_4)$-alkyl, $(C_1-C_4)$-Alkoxy oder Halo-$(C_1-C_4)$-alkoxy;

unsubstituiertes oder substituiertes $(C_4-C_6)$-Cycloalkenyl, wobei der Substituent Halo, $(C_1-C_4)$-Alkyl, $(C_1-C_4)$-Alkoxy oder Halo-$(C_1-C_4)$-alkoxy ist;

unsubstituiertes oder phenylsubstituiertes Alkynyl;

Benzyl, wobei der Phenylring substituiert ist oder gleich oder unterschiedlich substituiert ist mit eins oder zwei aus Halo, Methyl oder Ethyl;

unsubstituierter oder substituierter fünfgliedriger Heterocyclus ausgewählt aus Furyl, Thienyl, Pyrrolyl und Oxazolyl, wobei die Substituenten gleich oder unterschiedlich sein können eins oder zwei aus Halo, Nitro, $(C_1-C_4)$-Alkyl oder $(C_1-C_4)$-Alkoxy; oder

unsubstituierter oder substituierter sechsgliedriger Heterocyclus mit eins oder zwei Stickstoffatomen und vier oder fünf Kohlenstoffatome im Kern, wobei die Substituenten gleich oder unterschiedlich sein können eins oder zwei aus Halo; Nitro; $(C_1-C_4)$-Alkyl; $(C_1-C_4)$-Alkoxy oder Thio-$(C_1-C_4)$-alkoxy;

wobei Z und Z' die Bedeutung Wasserstoff oder $(C_1-C_4)$-Alkyl haben.

**4.** Zusammensetzung nach Anspruch 3, wobei
X und X' die Bedeutung O haben;
$R^1$ bedeutet:
Wasserstoff; Methyl; Ethyl; $(C_1-C_2)$-Alkoxy-$(C_1-C_2)$-alkyl mit unabhängig der angegebenen Anzahl

von Kohlenstoffatomen in jeder Alkylgruppe; ($C_2$-$C_5$)-Alkenyl; ($C_2$-$C_5$)-Alkynyl; oder Benzyl, wobei der Phenylring unsubstituiert ist oder mit Halo substituiert ist;
$R^2$ und $R^3$ gleich oder unterschiedlich Wasserstoff oder ($C_1$-$C_2$)-Alkyl bedeuten;
$R^4$ bedeutet:
($C_1$-$C_2$)-Alkyl, substituiert mit eins bis vier Fluor; geradkettiges ($C_2$-$C_4$)-Alkenyl; Cyano; cyanosubstituiertes ($C_1$-$C_2$)-Alkyl; Tri($C_1$-$C_2$)-Alkylsilyl mit unabhängig der angegebenen Anzahl an Kohlenstoffatomen in jeder Alkylgruppe; oder Tri($C_1$-$C_2$)-alkylsilylmethyl mit unabhängig der angegebenen Anzahl an Kohlenstoffatomen in jeder Alkylgruppe; und
A und B bedeuten:
gleich oder unterschiedlich Phenyl oder substituiertes Phenyl, wobei die Substituenten gleich oder unterschiedlich eins bis drei sein können aus Halo; Nitro; ($C_1$-$C_4$)-Alkyl; ($C_1$-$C_4$)-Alkoxy oder Halo-($C_1$-$C_4$)-alkyl;

unsubstituiertes oder substituiertes ($C_1$-$C_6$)-Alkyl mit gleich oder unterschiedlich eins bis zwei aus Halo, Phenyl oder Cyano als Substituenten;

unsubstituiertes ($C_4$-$C_6$)-Cycloalkyl;

unsubstituiertes oder substituiertes ($C_2$-$C_6$)-Alkenyl mit gleich oder unterschiedlich eins bis drei aus Halo oder ($C_1$-$C_4$)-Alkyl als Substituenten;

unsubstituiertes ($C_4$-$C_6$)-Cycloalkenyl;

Phenalkyl mit eins oder zwei Kohlenstoffatomen in der Alkylgruppe, wobei der Phenylring unsubstituiert ist oder gleich oder unterschiedlich substituiert ist mit eins oder zwei aus Halo, ($C_1$-$C_4$)-Alkyl oder ($C_1$-$C_4$)-Alkoxy;

unsubstituiertes substituiertes Pyridyl oder 2,5-Pyrazinyl, wobei die Substituenten Halo, Nitro, ($C_1$-$C_4$)-Alkyl, $C_1$-$C_4$)-Alkoxy oder $C_1$-$C_4$)-Thioalkoxy sein können; oder

unsubstituiertes Furyl oder Thienyl oder ein unsubstituiertes oder substituiertes Pyrrolyl, wobei der Substituent ($C_1$-$C_4$)-Alkyl sein kann.

**5.** Zusammensetzung nach Anspruch 4, wobei
X und X' die Bedeutung O haben;
$R^1$ bedeutet: Wasserstoff; Methyl; Methoxymethyl; ($C_2$-$C_4$)-Alkenyl; ($C_2$-$C_5$)-Alkynyl; Benzyl oder 4-Halobenzyl;
$R^2$ und $R^3$ gleich oder unterschiedlich Wasserstoff, Methyl oder Ethyl bedeuten;
$R^4$ bedeutet:
Trifluormethyl; 2,2,2-Trifluoroethyl; geradkettiges ($C_2$-$C_4$)-Alkenyl; Cyano; Cyanomethyl; Tri($C_1$-$C_2$)-alkylsilyl mit unabhängig der angegebenen Anzahl an Kohlenstoffatomen in jeder Alkylgruppe; oder Trimethylsilylmethyl; und
A und B bedeuten:
gleich oder unterschiedlich Phenyl oder substituiertes Phenyl, wobei die Substituenten gleich oder unterschiedlich eins, zwei oder drei aus Chlor, Fluor, Brom, Iod, Nitro, Methyl, Ethyl, Methoxy oder Trifluormethyl sein können;

($C_2$-$C_3$) substituiertes Alkenyl mit gleich oder unterschiedlich eins bis drei aus Chlor, Brom, Methyl oder Ethyl;

Cyclohexenyl;

Benzyl;

unsubstituiertes oder substituiertes Pyridyl oder 2,5-Pyrazinyl, wobei der Substituent Halo, Methyl, Ethyl oder Methoxy sein kann; oder

unsubstituiertes Furyl oder Thienyl oder ein unsubstituiertes oder substituiertes Pyrrolyl, wobei der

Substituent Ethyl oder Methyl sein kann.

6. Zusammensetzung nach Anspruch 2, wobei
X und X' die Bedeutung O haben;
$R^1$ die Bedeutung Wasserstoff oder Methyl hat;
$R^2$ und $R^3$ die Bedeutung Wasserstoff oder $(C_1-C_3)$-Alkyl haben;
$R^4$ bedeutet:
Trifluormethyl, 2,2,2-Trifluorethyl, Cyano, Cyanomethyl, geradkettiges $(C_2-C_4)$-Alkenyl, Carboxyl, Methoxycarbonyl, Tri$(C_1-C_2)$-Alkylsilyl mit unabhängig der angegebenen Anzahl an Kohlenstoffatomen in jeder Alkylgruppe oder Tri$(C_1-C_2)$-Alkylsilylmethyl mit unabhängig der angegebenen Anzahl an Kohlenstoffatomen in jeder Alkylgruppe; und
A und B gleich oder unterschiedlich Thienyl oder unsubstituiertes Phenyl bedeuten, wobei die Substituenten gleich oder unterschiedlich eins bis drei aus Chlor, Fluor, Nitro, Methyl, Ethyl oder Propyl sein können.

7. Zusammensetzung nach Anspruch 6, wobei
X und X' die Bedeutung O haben;
$R^1$ die Bedeutung Wasserstoff hat;
$R^2$ und $R^3$ die Bedeutung Wasserstoff, Methyl oder Ethyl haben;
$R^4$ die Bedeutung Trifluormethyl, Cyano, Allyl, Methoxycarbonyl oder Trimethylsilyl hat; und
A und B bedeuten: gleich oder unterschiedlich unsubstituiertes oder
substituiertes Phenyl mit gleich oder unterschiedlich eins oder zwei aus Chlor, Fluor, Nitro, Methyl und Ethyl; oder unsubstituiertes Thienyl.

8. Zusammensetzung nach Anspruch 7, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B gleich oder unterschiedlich unsubstituiertes oder substituiertes Phenyl bedeuten, wobei die Substituenten gleich oder unterschiedlich eins oder zwei aus Chlor, Methyl oder Nitro sein können; oder
$R^2$ und $R^3$ gleich oder unterschiedlich Methyl oder Ethyl bedeuten, $R^4$ die Bedeutung Cyano hat, und
A und B gleich oder unterschiedlich unsubstituiertes oder substituiertes Phenyl bedeuten, wobei die Substituenten gleich oder unterschiedlich eins oder zwei aus Chlor, Fluor, Methyl, Ethyl oder Nitro sein können; oder
$R^2$ und $R^3$ die Bedeutung Methyl haben, $R^4$ die Bedeutung Allyl hat, und A und B gleich oder unterschiedlich unsubstituiert ist oder methylsubstituiertes Phenyl bedeuten; oder
$R^2$ und $R^3$ die Bedeutung Wasserstoff haben, $R^4$ die Bedeutung Trimethylsilyl hat, und A und B gleich oder unterschiedlich substituiertes Phenyl bedeutet, wobei die Substituenten gleich oder unterschiedlich eins oder zwei aus Methyl, Ethyl oder Nitro sein können.

9. Zusammensetzung nach Anspruch 8, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B die Bedeutung unsubstituiertes Phenyl haben, oder A die Bedeutung 2,3-Dimethylphenyl hat und B die Bedeutung 2,4-Dichlorphenyl, 3,5-Dimethylphenyl oder 2-Nitro-5-methylphenyl hat; oder
$R^2$ und $R^3$ die Bedeutung Methyl haben, $R^4$ die Bedeutung Allyl hat, und sowohl A als auch B die Bedeutung unsubstituiertes Phenyl oder 3-Methylphenyl haben; oder
$R^2$ und $R^3$ die Bedeutung Wasserstoff haben, $R^4$ die Bedeutung Trimethylsilyl hat, und A die Bedeutung 4-Ethylphenyl hat und B die Bedeutung 2-Nitro-5-methylphenyl hat, oder sowohl A als auch B die Bedeutung 2-Methylphenyl oder 2-Nitrophenyl haben.

10. Zusammensetzung nach Anspruch 8, wobei
X und X' die Bedeutung O haben;
$R^1$ die Bedeutung Wasserstoff hat; und

$R^2$ und $R^3$ die Bedeutung Methyl haben;
$R^4$ die Bedeutung Cyano hat; und
A und B gleich oder unterschiedlich unsubstituiertes oder substituiertes Phenyl bedeutet, wobei die Substituenten gleich oder unterschiedlich eins oder zwei aus Chlor, Fluor, Methyl, Ethyl oder Nitro sein können.

11. Zusammensetzung nach einem der vorhergehenden Ansprüche mit einem Gehalt der insektiziden Verbindung in einer Menge von etwa 0,0001 bis 99 Gew.-% der Zusammensetzung.

12. Zusammensetzung nach Anspruch 11, wobei die Menge der insektiziden Verbindung 0,01 bis 99 Gew.-% der Zusammensetzung beträgt.

13. Verfahren zum Kontrollieren von Insekten, bei der man die Insekten mit einer insektizid wirksamen Menge einer Verbindung nach einem der Ansprüche 1 bis 10 in Kontakt bringt, wahlweise in einer Zusammensetzung nach einem der Ansprüche 1 bis 12.

14. Verfahren nach Anspruch 13, wobei man die Verbindung oder Zusammensetzung Insekten oder wachsenden Pflanzen oder einem Gebiet, in dem Pflanzen zu ziehen sind, in einer Menge von 10 g bis 10 kg pro Hektar der Verbindung zuführt.

15. Verfahren nach Anspruch 14, wobei die Applikationsrate 100 g bis 5 kg pro Hektar der Verbindung beträgt.

16. Verfahren nach einem der Ansprüche 13 bis 15, wobei die Insekten von der Ordnung Lepidoptera oder Coleoptera sind.

Patentansprüche für folgenden Vertragsstaat : ES

1. Verwendung einer insektizid wirksamen Hydrazin-Verbindung mit der Formel:

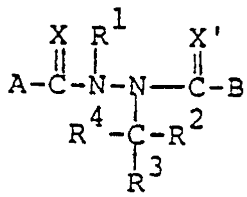

(I),

wobei
X und X' gleich oder unterschiedlich die Bedeutung O, S oder NR haben;
$R^1$ bedeutet:
Wasserstoff; $(C_1\text{-}C_6)$-Alkyl; $(C_1\text{-}C_6)$-Alkoxy-$(C_1\text{-}C_6)$-alkyl mit unabhängig der angegebenen Anzahl an Kohlenstoffatomen in jeder Alkylgruppe; $(C_1\text{-}C_6)$-Alkythio-$(C_1\text{-}C_6)$alkyl mit unabhängig der angegebenen Anzahl von Kohlenstoffatomen in jeder Alkylgruppe; $(C_2\text{-}C_6)$-Alkenyl; $(C_2\text{-}C_6)$-Alkynyl; oder Phen-$(C_1\text{-}C_4)$-Alkyl, wobei der Phenylring unsubstituiert ist oder gleich oder unterschiedlich substituiert ist mit eins bis drei aus Halo, Cyano, Nitro, Hydroxy, $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, Carboxy, $(C_1\text{-}C_4)$-Alkoxycarbonyl, und $(C_1\text{-}C_4)$-Alkanoyloxy oder NZZ';
$R^2$ und $R^3$ gleich oder unterschiedlich Wasserstoff oder $(C_1\text{-}C_4)$-Alkyl bedeuten;
$R^4$ bedeutet:
$(C_1\text{-}C_4)$-Alkyl substituiert mit 1 bis 4 Fluor; geradkettiges $(C_2\text{-}C_4)$-Alkenyl; Carboxyl; $(C_1\text{-}C_3)$-Alkoxycarbonyl; Cyano; cyano-substituiertes $(C_1\text{-}C_4)$-Alkyl; Tri$(C_1\text{-}C_4)$-alkylsilyl mit unabhängig der angegebenen Anzahl an Kohlenstoffatomen in jeder Alkylgruppe; oder Tri$(C_1\text{-}C_2)$-alkylsilylmethyl mit unabhängig der angegebenen Anzahl an Kohlenstoffatomen in jeder Alkylgruppe; mit der Maßgabe, daß sowohl $R^2$ als auch $R^3$ Alkyl sind, wenn $R^4$ Carboxyl oder Alkoxycarbonyl ist; und
A und B bedeuten:
gleich oder unterschiedlich unsubstituiertes oder substituiertes Naphthyl, wobei die Substituenten gleich oder unterschiedlich eins bis drei aus Halo; Nitro; $(C_1\text{-}C_4)$-Alkoxy; $(C_1\text{-}C_4)$-Alkyl oder NZZ'

sein können;

unsubstituiertes oder substituiertes Phenyl, wobei die Substituenten sein können gleich oder unterschiedlich von eins bis fünf aus Halo; Nitroso; Nitro; Cyano; Hydroxy; $(C_1-C_6)$-Alkyl; Halo-$(C_1-C_6)$-alkyl; Cyano-$(C_1-C_6)$-alkyl; Hydroxy-$(C_1-C_6)$-alkyl; $(C_1-C_6)$-Alkoxy; Halo-$(C_1-C_6)$-alkoxy; $(C_1-C_6)$-Alkoxy-$(C_1-C_6)$-alkyl mit unabhängig der angegebenen Anzahl von Kohlenstoffatomen in jeder Alkylgruppe; $(C_1-C_6)$-Alkoxy-$(C_1-C_6)$-alkoxy mit unabhängig der angegebenen Anzahl von Kohlenstoffatomen in jeder Alkylgruppe; -ORSR'-Gruppe; $-OCO_2R$-Gruppe; $-OCO_2H$-Gruppe; $(C_1-C_6)$-Alkanoyloxy-$(C_1-C_6)$-alkyl mit unabhängig der angegebenen Anzahl von Kohlenstoffatomen in jeder Alkylgruppe; $(C_2-C_6)$-Alkenyl, wahlweise substituiert mit Halo, Cyano, $(C_1-C_4)$-Alkyl, Halo-$(C_1-C_4)$-alkoxy, $(C_1-C_4)$-Alkylthio oder $(C_1-C_4)$-Alkoxy; $(C_3-C_5)$-Alkadienyl; $(C_2-C_6)$-Alkenyloxy; $(C_2-C_6)$-Alkenylcarbonyl; $(C_2-C_6)$-Alkenyloxycarbonyloxy; $(C_2-C_6)$-Alkynyl, wahlweise substituiert mit Halo, Cyano, Nitro, Hydroxy, $(C_1-C_4)$-Alkoxy, Halo-$(C_1-C_4)$-alkyl, $(C_1-C_4)$-Alkylthio oder $(C_1-C_4)$-Alkyl; Carboxy; $-RCO_2R'$-Gruppe; -COR; COH; Halo-$(C_1-C_6)$-Alkylcarbonyl; $-CO_2R$-Gruppe; $(C_1-C_6)$-Haloalkoxycarbonyl; -OCOR-Gruppe; $-ORCO_2R'$-Gruppe; Amino (-NZZ'); Amino substituiert mit Hydroxy, $(C_1-C_4)$-Alkoxy oder $(C_1-C_4)$-Alkylthio; -CONZZ'-Gruppe; $(C_2-C_6)$-Alkenylcarbonylamino; Hydroxy-$(C_1-C_6)$-alkylaminocarbonyl; -OCONZZ'-Gruppe; -NZCOZ'-Gruppe; $-NZCO_2Z'$-Gruppe, Thiocyanato; Isothiocyanato; $(C_1-C_6)$-Tiocyanatoalkyl; $(C_1-C_6)$-Alkylthio; Halo-$(C_1-C_6)$-alkylthio; -S(O)-Z-Gruppe; $-SO_2Z$-Gruppe; $-OSO_2R$-Gruppe; $-OSO_2H$-Gruppe; $-SO_2NZZ'$-Gruppe, -CSR-Gruppe; -CSH-Gruppe; -SCOR-Gruppe; -SCOH-Gruppe; -NSCSZ'-Gruppe; unsubstituiertes oder substituiertes Phenyl mit gleich oder unterschiedlich ein bis drei aus Halo, Cyano, Nitro, Hydroxy, $(C_1-C_4)$-Alkyl, Halo-$(C_1-C_4)$-alkyl, $(C_1-C_4)$-Alkoxy, Carboxy, $(C_1-C_4)$-Alkoxycarbonyl, $(C_1-C_4)$-Alkanoyloxy, Amino, $(C_1-C_4)$-Alkylamino oder Di$(C_1-C_4)$-alkylamino mit unabhängig der angegebenen Anzahl von Kohlenstoffatomen in jeder Alkylgruppe; Phenoxy, wobei der Phenylring unsubstituiert ist oder gleich oder unterschiedlich substituiert ist mit eins bis drei aus Halo, Cyano, Nitro, Hydroxy, $(C_1-C_4)$-Alkyl, Halo-$(C_1-C_4)$-alkyl, $(C_1-C_4)$-Alkoxy, Carboxy, $(C_1-C_4)$-Alkoxycarbonyl, $(C_1-C_4)$-Alkanoyloxy, Amino, $(C_1-C_4)$-Alkylamino oder Di$(C_1-C_4)$-Alkylamino mit unabhängig der angegebenen Anzahl von Kohlenstoffatomen in jeder Alkylgruppe; Benzoyl, wobei der Phenylring unsubstituiert ist oder gleich oder unterschiedlich substituiert ist mit eins bis drei aus Halo, Cyano, Nitro, Hydroxy, $(C_1-C_4)$-Alkyl, Halo-$(C_1-C_4)$-alkyl, $(C_1-C_4)$-Alkoxy, Carboxy, $(C_1-C_4)$-Alkoxycarbonyl, $(C_1-C_4)$-Alkanoyloxy, Amino, $(C_1-C_4)$-Alkylamino oder Di$(C_1-C_4)$-Alkylamino mit unabhängig der angegebenen Anzahl von Kohlenstoffatomen in jeder Alkylgruppe; Benzoyloxy-$(C_1-C_6)$-alkyl; Phenylthio-$(C_1-C_6)$-alkyl, wobei der Phenylring unsubstituiert ist oder gleich oder unterschiedlich substituiert ist mit eins bis drei aus Halo, Cyano, Nitro, Hydroxy, $(C_1-C_4)$-Alkyl, Halo-$(C_1-C_4)$-alkyl, $(C_1-C_4)$-Alkoxy, Carboxy, $(C_1-C_4)$-Alkoxycarbonyl, $(C_1-C_4)$-Alkanoyloxy, Amino, $(C_1-C_4)$-Alkylamino oder Di$(C_1-C_4)$-Alkylamino mit unabhängig der angegebenen Anzahl von Kohlenstoffatomen in jeder Alkylgruppe; -CR=N-R'"-Gruppe, wobei R'" die Bedeutung Hydroxy, $(C_1-C_4)$-Alkyl, $(C_1-C_4)$-Alkoxy, Amino (-NZZ'), Phenylamino, -COR, -COH oder Benzoyl hat; $(C_2-C_6)$-Oxiranyl; Acetylthiosemicarbazon; Pyrrolyl; Oxazolyl unsubstituiert oder substituiert mit einer oder zwei Methylgruppen; oder, wenn zwei benachbarte Positionen am Phenylring mit Alkoxygruppen substituiert sind, diese Gruppen verbunden sind, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes $(C_1-C_{10})$-Alkyl mit gleich oder unterschiedlich eins bis vier aus Halo, Cyano, Nitro, Hydroxy, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, Phenyl, oder -NZZ';

unsubstituiertes oder substituiertes $(C_3-C_8)$-Cycloalkyl oder unsubstituiertes oder substituiertes $(C_3-C_8)$-Cyclo$(C_1-C_4)$-alkyl mit gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, Phenyl oder -NZZ';

unsubstituiertes oder substituiertes $(C_2-C_8)$-Alkenyl oder unsubstituiertes oder substituiertes $(C_3-C_5)$-Alkadienyl, welche als Substituenten ein Furyl-, Thienyl- oder Pyridyl oder gleich oder unterschiedlich eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy oder -NZZ' haben;

unsubstituiertes oder substituiertes ($C_3$-$C_8$)-Cycloalkenyl oder unsubstituiertes oder substituiertes ($C_8$)-Cycloalkadienyl, welche als Substituenten gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy oder -NZZ' haben;

unsubstituiertes cder substituiertes ($C_2$-$C_8$)-Alkynyl, welches als Substituenten gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, Phenyl oder -NZZ' haben;

Phenylalkyl mit eins bis vier Kohlenstoffatomen in der Alkylgruppe, wobei die Alkylgruppe unsubstituiert ist oder gleich oder unterschiedlich substituiert ist mit eins bis drei aus Halo, Cyano, Hydroxy, ($C_1$-$C_4$)-Alkoxy, ($C_1$-$C_4$)-Alkoxycarbonyl oder -NZZ', und wobei der Phenylring unsubstituiert ist oder gleich oder unterschiedlich substituiert ist mit eins bis drei aus Halo, Cyano, Nitro, Hydroxy ($C_1$-$C_4$)-Alkyl, Halo-($C_1$-$C_4$)-alkyl, Cyano-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, ($C_2$-$C_6$)-Alkenyl, Halo-($C_2$-$C_6$)-alkenyl, ($C_2$-$C_6$)-Alkenyl, oder -NZZ';

Phenalkenyl mit zwei bis sechs Kohlenstoffatomen in der Alkenylgruppe, wobei die Alkenylgruppe unsubstituiert ist oder gleich oder unterschiedlich substituiert ist mit eins bis drei aus Halo, Cyano, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, ($C_1$-$C_4$)-Alkoxycarbonyl oder -NZZ', und wobei der Phenylring unsubstituiert ist oder gleich oder unterschiedlich substituiert ist mit eins bis drei aus Halo, Cyano, Nitro, Hydroxy ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy oder -NZZ';

unsubstituierter oder substituierter fünfgliedriger Heterocyclus ausgewählt aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl, wobei die Substituenten gleich oder unterschiedlich eins bis drei sein können aus Halo; Nitro; Hydroxy; ($C_1$-$C_6$)-Alkyl; ($C_1$-$C_6$)-Alkoxy; Carboxy; ($C_1$-$C_6$)-Alkoxycarbonyl; -$RCO_2H$-Gruppe; $RCO_2R'$-Gruppe; -CONZZ'-Gruppe; Amino (-NZZ'); -NZCOZ'-Gruppe; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl oder Amino (-NZZ'); oder

unsubstituierter oder substituierter sechsgliedriger Heterocyclus mit eins, zwei, drei oder vier Stickstoffatomen und zwei bis fünf Kohlenstoffatomen im Kern, wobei die Substituenten gleich oder unterschiedlich eins bis drei sein können aus Halo; Nitro; Hydroxy; ($C_1$-$C_6$)-Alkyl; ($C_1$-$C_6$)-Alkoxy; Carboxy; ($C_1$-$C_6$)-Alkoxycarbonyl; -$RCO_2H$-Gruppe; $RCO_2R'$-Gruppe; -CONZZ'-Gruppe; Amino (-NZZ'); -NZCOZ'-Gruppe; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl oder Amino (-NZZ');

wobei R und R' die Bedeutung ($C_{1-6}$)-Alkyl haben; Z und Z' die Bedeutung Wasserstoff oder ($C_1$-$C_4$)-Alkyl haben; "Amino" die Bedeutung NZZ' hat; oder ein landwirtschaftlich vertretbares Salz davon; zusammen mit landwirtschaftlich vertretbarem Verdünnungs-oder Trägerstoff zur Herstellung einer insektiziden Zusammensetzung.

2. Verwendung nach Anspruch 1, wobei
X und X' die Bedeutung O oder S haben;
$R^1$ bedeutet:
Wasserstoff; ($C_1$-$C_4$)-Alkyl; ($C_1$-$C_4$)-Alkoxy-($C_1$-$C_4$)-alkyl mit unabhängig der angegebenen Anzahl Kohlenstoffatome in jeder Alkylgruppe; Methylthiomethyl, ($C_2$-$C_5$)-Alkenyl; ($C_2$-$C_5$)-Alkynyl oder Phen-($C_1$-$C_2$)-Alkyl, wobei der Phenylring unsubstituiert ist oder substituiert ist mit eins oder zwei aus Halo, Nitro, ($C_1$-$C_4$)-Alkyl oder ($C_1$-$C_4$)-Alkoxy;
$R^2$ und $R^3$ gleich oder unterschiedlich Wasserstoff oder ($C_1$-$C_3$)-Alkyl bedeuten;
$R^4$ bedeutet:
($C_1$-$C_3$)-Alkyl, substituiert mit eins bis vier Fluor; geradkettiges ($C_2$-$C_4$)-Alkenyl; Carboxyl; ($C_1$-$C_2$)-

Alkoxycarbonyl; Cyano; cyanosubstituiertes ($C_1$-$C_3$)-Alkyl; Tri($C_1$-$C_2$)-Alkylsilyl mit unabhängig der angegebenen Anzahl an Kohlenstoffatomen in jeder Alkylgruppe; oder Tri($C_1$-$C_2$)-alkylsilylmethyl mit unabhängig der angegebenen Anzahl an Kohlenstoffatomen in jeder Alkylgruppe; und
A und B gleich oder unterschiedlich bedeuten:
unsubstituiertes Naphthyl;

unsubstituiertes oder substituiertes Phenyl, wobei die Substituenten gleich oder unterschiedlich eins bis drei sein können aus Halo; Nitro; Cyano; ($C_1$-$C_4$)-Alkyl; Halo-($C_1$-$C_4$)-alkyl; Cyano-($C_1$-$C_4$)-alkyl; ($C_1$-$C_4$)-Alkoxy; ($C_1$-$C_4$)-Alkoxy-($C_1$-$C_4$)-alkyl mit unabhängig der angegebenen Anzahl an Kohlenstoffatomen in jeder Alkylgruppe, -COZ; Carboxy; ($C_1$-$C_4$)-Alkoxycarbonyl; ($C_1$-$C_4$)-Alkanoyloxy; ($C_2$-$C_6$)-Alkenyl; ($C_2$-$C_6$)-Alkynyl; Amino (-NZZ'); Thiocyanato; ($C_1$-$C_4$)-Alkylthio; -CSR; -CSH; unsubstituiertes oder substituiertes Phenyl mit gleich oder unterschiedlich eins bis zwei aus Halo, Nitro, ($C_1$-$C_4$)-Alkyl, ($C_1$-$C_4$)-Alkoxy, Carboxy, -NZZ'; Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes ($C_1$-$C_8$)-Alkyl mit gleich oder unterschiedlich eins bis drei aus Halo, Cyano, ($C_1$-$C_4$)-Alkoxy, Phenyl, ($C_1$-$C_4$)-Alkoxycarbonyl oder Halo-($C_1$-$C_4$)-alkoxy;

unsubstituiertes oder substituiertes ($C_3$-$C_6$)-Cycloalkyl oder unsubstituiertes oder substituiertes ($C_3$-$C_6$)-Cycloalkyl-($C_1$-$C_4$)-alkyl mit gleich oder unterschiedlich eins oder zwei aus Halo, ($C_1$-$C_4$)-Alkyl, Halo-($C_1$-$C_4$)alkyl, ($C_1$-$C_4$)-Alkoxy, ($C_1$-$C_4$)-Alkanoyl oder Halo-($C_1$-$C_4$)-Alkoxy;

unsubstituiertes oder substituiertes ($C_2$-$C_6$)-Alkenyl mit einem Furyl als Substituenten oder gleich oder unterschiedlich eins bis drei aus ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder substituiertes ($C_3$-$C_6$)-Cycloalkenyl, welches als Substituenten gleich oder unterschiedlich hat eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituiertes oder phenylsubstituiertes Alkynyl;

Phenalkyl mit einem oder zwei Kohlenstoffatomen in der Alkylgruppe, wobei die Alkylgruppe unsubstituiert ist oder substituiert ist mit gleich oder unterschiedlich eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)-Alkoxy oder Halo-($C_1$-$C_4$)-alkoxy;

Phenalkenyl mit zwei oder drei Kohlenstoffatomen in der Alkenylgruppe, wobei die Alkenylgruppe unsubstituiert ist oder substituiert ist mit Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy und wobei der Phenylring unsubstituiert ist oder gleich oder unterschiedlich substituiert ist mit eins oder zwei aus Halo, Cyano, ($C_1$-$C_4$)-Alkyl, Halo-($C_1$-$C_4$)-alkyl, ($C_1$-$C_4$)-Alkoxy oder Halo-($C_1$-$C_4$)-alkoxy;

unsubstituierter oder substituierter fünfgliedriger Heterocyclus, ausgewählt aus Furyl, Thienyl, Triazolyl, Pyrrolyl und Oxazolyl, wobei die Substituenten gleich oder unterschiedlich sein können eins oder zwei aus Halo; Nitro; ($C_1$-$C_4$)-Alkyl; ($C_1$-$C_4$)-Alkoxy; -NZZ'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NZZ'; oder

unsubstituierter oder substituierter sechsgliedriger Heterocyclus mit einem oder zwei Stickstoffatomen und vier bis fünf Kohlenstoffatome im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy; -NZZ'; oder unsubstituiertes oder substituiertes Phenyl mit gleich oder unterschiedlich eins oder

zwei aus Halo, Nitro, $(C_1\text{-}C_4)$-Alkyl, Halo-$(C_1\text{-}C_4)$-alkyl, $(C_1\text{-}C_4)$-Alkoxy, Halo-$(C_1\text{-}C_4)$-alkoxy, Carboxy oder -NZZ';

wobei R und R' die Bedeutung $(C_{1-4})$-Alkyl haben; Z und Z' die Bedeutung Wasserstoff oder $(C_1\text{-}C_4)$-Alkyl haben.

**3.** Verwendung nach Anspruch 2, wobei
X und X' die Bedeutung O oder S haben;
$R^1$ bedeutet:
Wasserstoff; $(C_1\text{-}C_4)$-Alkyl; $(C_1\text{-}C_4)$-Alkoxy-$(C_1\text{-}C_4)$-alkyl mit unabhängig der angegebenen Anzahl von Kohlenstoffatome in jeder Alkylgruppe; $(C_2\text{-}C_5)$-Alkenyl; $(C_2\text{-}C_5)$-Alkynyl oder Benzyl, wobei der Phenylring unsubstituiert ist oder substituiert ist mit einem Halo, Nitro, $(C_1\text{-}C_4)$-Alkyl oder $(C_1\text{-}C_4)$-Alkoxy;
$R^2$ und $R^3$ gleich oder unterschiedlich Wasserstoff oder $(C_1\text{-}C_2)$-Alkyl bedeuten;

$R^4$ bedeutet:
$(C_1\text{-}C_3)$-Alkyl, substituiert mit eins bis vier Fluor; geradkettiges $(C_2\text{-}C_4)$-Alkenyl; Methoxycarbonyl; Cyano; cyanosubstituiertes $(C_1C_2)$-Alkyl; Tri$(C_1\text{-}C_2)$-alkylsilyl mit unabhängig der angegebenen Anzahl von Kohlenstoffatomen in jeder Alkylgruppe; oder Tri$(C_1\text{-}C_2)$-alkylsilylmethyl mit unabhängig der angegebenen Anzahl von Kohlenstoffatomen in jeder Alkylgruppe; mit der Maßgabe, daß sowohl $R^2$ als auch $R^3$ Alkyl sind, wenn $R^4$ Methoxycarbonyl ist; und
A und B gleich oder unterschiedlich bedeuten:
unsubstituiertes Naphthyl;

unsubstituiertes oder substituiertes Phenyl mit gleich oder unterschiedlich eins bis drei aus Halo; Nitro; Cyano; $(C_1\text{-}C_4)$-Alkyl; Halo-$(C_1\text{-}C_4)$-alkyl; Cyano-$(C_1\text{-}C_4)$alkyl; $(C_1\text{-}C_4)$-Alkoxy; $(C_1\text{-}C_4)$-Alkoxy-$(C_1\text{-}C_4)$-alkyl mit unabhängig der angegebenen Anzahl von Kohlenstoffatomen in jeder Alkylgruppe, -COZ; $(C_1\text{-}C_4)$-Alkoxycarbonyl; $(C_1\text{-}C_4)$-Alkanoyloxy; Thiocyanat; -NZZ'; $(C_1\text{-}C_4)$-Alkylthio; -CSZ; $-CS_2Z$; unsubstituiertes oder substituiertes Phenyl mit gleich oder unterschiedlich eins oder zwei aus Halo, Nitro, $(C_1\text{-}C_4)$-Alkyl, $(C_1\text{-}C_4)$-Alkoxy, Carboxy, -NZZ'; oder Phenoxy, wobei der Phenylring unsubstituiert ist oder gleich oder unterschiedlich substituiert ist mit eins oder zwei aus Halo, Nitro, $(C_1\text{-}C_4)$-Alkyl, $(C_1\text{-}C_4)$-Alkoxy, Carboxy, -NZZ'; oder wenn zwei benachbarte Positionen am Phenylring mit Alkoxy-Gruppen substituiert sind, diese Gruppen verbunden sein können, um zusammen mit den Kohlenstoffatomen, an denen sie hängen, einen 5- oder 6-gliedrigen heterocyclischen Dioxolano- oder Dioxanoring zu bilden;

unsubstituiertes oder substituiertes $(C_1\text{-}C_6)$-Alkyl mit gleich oder unterschiedlich eins bis drei aus Halo, Cyano, Nitro, Carboxy, $(C_1\text{-}C_4)$-Alkoxy, Phenyl, $(C_1\text{-}C_4)$-Alkoxycarbonyl oder Halo-$(C_1\text{-}C_4)$-alkoxy;

unsubstituiertes oder substituiertes $(C_3\text{-}C_6)$-Cycloalkyl oder unsubstituiertes oder substituiertes $(C_3\text{-}C_6)$-Cycloalkyl-$(C_1\text{-}C_4)$-alkyl, wobei der Substituent Halo, $(C_1\text{-}C_4)$-Alkyl, $(C_1\text{-}C_4)$-Alkanoyl, $(C_1\text{-}C_4)$-Alkoxy oder Halo-$(C_1\text{-}C_4)$-alkoxy ist;

unsubstituiertes oder substituiertes $(C_2\text{-}C_6)$-Alkenyl mit gleich oder unterschiedlich eins oder zwei aus Halo-$(C_1\text{-}C_4)$-alkyl, $(C_1\text{-}C_4)$-Alkoxy oder Halo-$(C_1\text{-}C_4)$-alkoxy;

unsubstituiertes oder substituiertes $(C_4\text{-}C_6)$-Cycloalkenyl, wobei der Substituent Halo, $(C_1\text{-}C_4)$-Alkyl, $(C_1\text{-}C_4)$-Alkoxy oder Halo-$(C_1\text{-}C_4)$-alkoxy ist;

unsubstituiertes oder phenylsubstituiertes Alkynyl;

Benzyl, wobei der Phenylring unsubstituiert ist oder gleich oder unterschiedlich substituiert ist mit eins oder zwei aus Halo, Methyl oder Ethyl;

unsubstituierter oder substituierter fünfgliedriger Heterocyclus ausgewählt aus Furyl, Thienyl, Pyrrolyl und Oxazolyl, wobei die Substituenten gleich oder unterschiedlich sein können eins oder zwei aus Halo, Nitro, $(C_1\text{-}C_4)$-Alkyl oder $(C_1\text{-}C_4)$-Alkoxy; oder

unsubstituierter oder substituierter sechsgliedriger Heterocyclus mit eins oder zwei Stickstoffatomen und vier oder fünf Kohlenstoffatome im Kern, wobei die Substituenten gleich oder unterschiedlich sein können eins oder zwei aus Halo; Nitro; ($C_1$-$C_4$)-Alkyl; ($C_1$-$C_4$)-Alkoxy oder Thio-($C_1$-$C_4$)-alkoxy; wobei Z und Z' die Bedeutung Wasserstoff oder ($C_1$-$C_4$)-Alkyl haben.

**4.** Verwendung nach Anspruch 3, wobei
X und X' die Bedeutung O haben;
$R^1$ bedeutet:
Wasserstoff; Methyl; Ethyl; ($C_1$-$C_2$)-Alkoxy-($C_1$-$C_2$)-alkyl mit unabhängig der angegebenen Anzahl von Kohlenstoffatomen in jeder Alkylgruppe; ($C_2$-$C_5$)-Alkenyl; ($C_2$-$C_5$)-Alkynyl; oder Benzyl, wobei der Phenylring unsubstituiert ist oder mit Halo substituiert ist;
$R^2$ und $R^3$ gleich oder unterschiedlich Wasserstoff oder ($C_1$-$C_2$)-Alkyl bedeuten;
$R^4$ bedeutet:
($C_1$-$C_2$)-Alkyl, substituiert mit eins bis vier Fluor; geradkettiges ($C_2$-$C_4$)-Alkenyl; Cyano; cyanosubstituiertes ($C_1$-$C_2$)-Alkyl; Tri($C_1$-$C_2$)-Alkylsilyl mit unabhängig der angegebenen Anzahl an Kohlenstoffatomen in jeder Alkylgruppe; oder Tri($C_1$-$C_2$)-alkylsilylmethyl mit unabhängig der angegebenen Anzahl an Kohlenstoffatomen in jeder Alkylgruppe; und
A und B bedeuten:
gleich oder unterschiedlich Phenyl oder substituiertes Phenyl, wobei die Substituenten gleich oder unterschiedlich eins bis drei sein können aus Halo; Nitro; ($C_1$-$C_4$)-Alkyl; ($C_1$-$C_4$)-Alkoxy oder Halo-($C_1$-$C_4$)-alkyl;

unsubstituiertes oder substituiertes ($C_1$-$C_6$)-Alkyl mit gleich oder unterschiedlich eins bis zwei aus Halo, Phenyl oder Cyano als Substituenten;

unsubstituiertes ($C_4$-$C_6$)-Cycloalkyl;

unsubstituiertes oder substituiertes ($C_2$-$C_6$)-Alkenyl mit gleich oder unterschiedlich eins bis drei aus Halo oder ($C_1$-$C_4$)-Alkyl als Substituenten;

unsubstituiertes ($C_4$-$C_6$)-Cycloalkenyl;

Phenalkyl mit eins oder zwei Kohlenstoffatomen in der Alkylgruppe, wobei der Phenylring unsubstituiert ist oder gleich oder unterschiedlich substituiert ist mit eins oder zwei aus Halo, ($C_1$-$C_4$)-Alkyl oder ($C_1$-$C_4$)-Alkoxy;

unsubstituiertes substituiertes Pyridyl oder 2,5-Pyrazinyl, wobei die Substituenten Halo, Nitro, ($C_1$-$C_4$)-Alkyl, $C_1$-$C_4$)-Alkoxy oder $C_1$-$C_4$)-Thioalkoxy sein können; oder

unsubstituiertes Furyl oder Thienyl oder ein unsubstituiertes oder substituiertes Pyrrolyl, wobei der Substituent ($C_1$-$C_4$)-Alkyl sein kann.

**5.** Verwendung nach Anspruch 4, wobei
X und X' die Bedeutung O haben;
$R^1$ bedeutet: Wasserstoff; Methyl; Methoxymethyl; ($C_2$-$C_4$)-Alkenyl; ($C_2$-$C_5$)-Alkynyl; Benzyl oder 4-Halobenzyl;
$R^2$ und $R^3$ gleich oder unterschiedlich Wasserstoff, Methyl oder Ethyl bedeuten;
$R^4$ bedeutet:
Trifluormethyl; 2,2,2-Trifluoroethyl; geradkettiges ($C_2$-$C_4$)-Alkenyl; Cyano; Cyanomethyl; Tri($C_1$-$C_2$)-alkylsilyl mit unabhängig der angegebenen Anzahl an Kohlenstoffatomen in jeder Alkylgruppe; oder Trimethylsilylmethyl; und
A und B bedeuten:
gleich oder unterschiedlich Phenyl oder substituiertes Phenyl, wobei die Substituenten gleich oder unterschiedlich eins, zwei oder drei aus Chlor, Fluor, Brom, Iod, Nitro, Methyl, Ethyl, Methoxy oder Trifluormethyl sein können;

($C_2$-$C_3$) substituiertes Alkenyl mit gleich oder unterschiedlich eins bis drei aus Chlor, Brom, Methyl

oder Ethyl;

Cyclohexenyl;

Benzyl;

unsubstituiertes oder substituiertes Pyridyl oder 2,5-Pyrazinyl, wobei der Substituent Halo, Methyl, Ethyl oder Methoxy sein kann; oder

unsubstituiertes Furyl oder Thienyl oder ein unsubstituiertes oder substituiertes Pyrrolyl, wobei der Substituent Ethyl oder Methyl sein kann.

6. Verwendung nach Anspruch 2, wobei
X und X' die Bedeutung O haben;
$R^1$ die Bedeutung Wasserstoff oder Methyl hat;
$R^2$ und $R^3$ die Bedeutung Wasserstoff oder ($C_1$-$C_3$)-Alkyl haben;
$R^4$ bedeutet:
Trifluormethyl, 2,2,2-Trifluorethyl, Cyano, Cyanomethyl, geradkettiges ($C_2$-$C_4$)-Alkenyl, Carboxyl, Methoxycarbonyl, Tri($C_1$-$C_2$)-Alkylsilyl mit unabhängig der angegebenen Anzahl an Kohlenstoffatomen in jeder Alkylgruppe oder Tri($C_1$-$C_2$)-Alkylsilylmethyl mit unabhängig der angegebenen Anzahl an Kohlenstoffatomen in jeder Alkylgruppe; und
A und B gleich oder unterschiedlich Thienyl oder unsubstituiertes Phenyl bedeuten, wobei die Substituenten gleich oder unterschiedlich eins bis drei aus Chlor, Fluor, Nitro, Methyl, Ethyl oder Propyl sein können.

7. Verwendung nach Anspruch 6, wobei
X und X' die Bedeutung O haben;
$R^1$ die Bedeutung Wasserstoff hat;
$R^2$ und $R^3$ die Bedeutung Wasserstoff, Methyl oder Ethyl haben;
$R^4$ die Bedeutung Trifluormethyl, Cyano, Allyl, Methoxycarbonyl oder Trimethylsilyl hat; und
A und B bedeuten:
gleich oder unterschiedlich unsubstituiertes oder substituiertes Phenyl mit gleich oder unterschiedlich eins oder zwei aus Chlor, Fluor, Nitro, Methyl und Ethyl; oder unsubstituiertes Thienyl.

8. Verwendung nach Anspruch 7, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B gleich oder unterschiedlich unsubstituiertes oder substituiertes Phenyl bedeuten, wobei die Substituenten gleich oder unterschiedlich eins oder zwei aus Chlor, Methyl oder Nitro sein können; oder
$R^2$ und $R^3$ gleich oder unterschiedlich Methyl oder Ethyl bedeuten, $R^4$ die Bedeutung Cyano hat, und
A und B gleich oder unterschiedlich unsubstituiertes oder substituiertes Phenyl bedeuten, wobei die Substituenten gleich oder unterschiedlich eins oder zwei aus Chlor, Fluor, Methyl, Ethyl oder Nitro sein können; oder
$R^2$ und $R^3$ die Bedeutung Methyl haben, $R^4$ die Bedeutung Allylhat, und A und B gleich oder unterschiedlich unsubstituiert ist oder methylsubstituiertes Phenyl bedeuten; oder
$R^2$ und $R^3$ die Bedeutung Wasserstoff haben, $R^4$ die Bedeutung Trimethylsilyl hat, und A und B gleich oder unterschiedlich substituiertes Phenyl bedeutet, wobei die Substituenten gleich oder unterschiedlich eins oder zwei aus Methyl, Ethyl oder Nitro sein können.

9. Verwendung nach Anspruch 8, wobei
X und X' die Bedeutung O haben;
$R^1$ die Bedeutung Wasserstoff hat; und
$R^2$ die Bedeutung Wasserstoff hat, $R^3$ die Bedeutung Methyl hat, $R^4$ die Bedeutung Trifluormethyl hat, und A und B die Bedeutung unsubstituiertes Phenyl haben, oder A die Bedeutung 2,3-Dimethylphenyl hat und B die Bedeutung 2,4-Dichlorphenyl, 3,5-Dimethylphenyl oder 2-Nitro-5-

methylphenyl hat; oder
$R^2$ und $R^3$ die Bedeutung Methyl haben, $R^4$ die Bedeutung Allyl hat, und sowohl A als auch B die Bedeutung unsubstituiertes Phenyl oder 3-Methylphenyl haben; oder
$R^2$ und $R^3$ die Bedeutung Wasserstoff haben, $R^4$ die Bedeutung Trimethylsilyl hat, und A die Bedeutung 4-Ethylphenyl hat und B die Bedeutung 2-Nitro-5-methylphenyl hat, oder sowohl A als auch B die Bedeutung 2-Methylphenyl oder 2-Nitrophenyl haben.

**10.** Verwendung nach Anspruch 8, wobei
X und X' die Bedeutung O haben;
$R^1$ die Bedeutung Wasserstoff hat; und
$R^2$ und $R^3$ die Bedeutung Methyl haben;
$R^4$ die Bedeutung Cyano hat; und
A und B gleich oder unterschiedlich unsubstituiertes oder substituiertes Phenyl bedeutet, wobei die Substituenten gleich oder unterschiedlich eins oder zwei aus Chlor, Fluor, Methyl, Ethyl oder Nitro sein können.

**11.** Verwendung nach einem der vorhergehenden Ansprüche der Verbindung zur Herstellung einer Zusammensetzung, mit einem Gehalt der insektiziden Verbindung in einer Menge von 0,0001 bis 99 Gew.-% der Zusammensetzung.

**12.** Verwendung nach Anspruch 11 der Verbindung zur Herstellung einer Zusammensetzung, wobei die Menge der insektiziden Zusammensetzung 0,01 bis 99 Gew.-% beträgt.

**13.** Verwendung einer Verbindung wie in einem der Ansprüche 1 bis 10 definiert, wahlweise in einer Zusammensetzung mit landwirtschaftlich vertretbarem Verdünnungs- oder Trägerstoff zum Kontrollieren von Insekten, bei dem man die Insekten mit einer insektizid wirksamen Menge der Verbindung oder Zusammensetzung in Kontakt bringt.

**14.** Mechanisches Verfahren zur Verbesserung des Handelswertes und/oder der Rentabilität verkaufbarer Ernten aus Pflanzen, deren Wachstum von Insekten beeinflußt wird oder möglicherweise beeinflußt wird, wobei man
(1) einem Behälter, einer Vernebelungsvorrichtung oder einem mechanischen Ansbreiter eine insektizide Verbindung oder ein Salz wie in einem der Ansprüche 1 bis 10 definiert zuführt, wahlweise in einer Mischung mit landwirtschaftlich vertretbarem Verdünnungs- oder Trägerstoff,
(2) den Behälter, Vernebeler oder mechanischen Ausbreiter dazu verwendet, die insektizide Verbindung oder das Salz in Form von Granulat, Staub, Rauch, Dampf oder tensidhaltiger Flüssigkeitszubereitung wachsenden Pflanzen oder einem Wachstumsmedium zuzuführen, wo die Pflanzen wachsen oder zu ziehen sind, oder den Insekten selbst;
(3) die Dosis des aktiven Bestandteiles während des Applizierungsschrittes so steuert, daß die Applizierungsrate der wirksamen Insektizidenverbindung zum Bekämpfen der Insekten ausreicht, jedoch nicht ausreicht zur Erzeugung einer nicht hinnehmbaren nachteiligen Auswirkung auf die in dem behandelten Gebiet wachsenden oder zu ziehenden Erntepflanzen.

**15.** Verwendung oder Verfahren nach Anspruch 13 oder 14, wobei man die Verbindung oder Zusammensetzung Insekten oder wachsenden Pflanzen oder einem Gebiet, in dem Pflanzen zu ziehen sind, in einer Menge von 10 g bis 10 kg pro Hektar der Verbindung zuführt.

**16.** Verwendung oder Verfahren nach Anspruch 13, 14 oder 15, wobei die Applikationsrate 100 g bis 5 kg pro Hektar beträgt.

**17.** Verwendung oder Verfahren nach einem der Ansprüche 13 bis 16, wobei die Insekten von der Ordnung Lepidoptera oder Coleoptera sind.